# EUROPEAN PATENT APPLICATION

(11) **EP 3 398 948 A2**
(43) Date of publication of application: **07.11.2018**
(21) Application number: 18177058.7
(22) Date of filing: 21.08.2015
(51) Int. Cl.: C07D 475/02, C07D 475/06, C07D 475/00, C07D 475/08, A61K 31/519, A61P 19/00, A61P 17/00, A61P 3/00

(54) **2,4,6,7-TETRASUBSTITUTED PTERIDINE COMPOUNDS AND METHODS OF SYNTHESIS AND USE THEREOF**

(30) Priority: 22.08.2014 US 201462040824 P; 15.09.2014 US 201462050321 P
(62) Divisional of application: 15833999.4
(71) Applicant: Janus Biotherapeutics, Inc., Cambridge, MA 02142 (US)
(72) Inventor: LIPFORD, Grayson B, Watertown, MA 02472 (US); ZEPP, Charles M, Hardwick, MA 01037 (US)
(74) Representative: HGF Limited

(57) **Abstract**

Compounds as immune system modulators bearing a pteridine core are described. A pharmaceutical composition comprising the same, methods of making the same, and a method for treating or preventing autoimmunity disease using the same are described.

SEQUENCE LISTING

<110> JANUS BIOTHERAPEUTICS, INC.

<120> NOVEL N2, N4, N7, 6-TETRASUBSTITUTED PTERIDINE-2,4,7-TRIAMINE
      AND 2, 4, 6, 7-TETRASUBSTITUTED PTERIDINE COMPOUNDS AND
      METHODS OF SYNTHESIS AND USE THEREOF

<130> 2205963.129WO1

<140> PCT/US2015/046218
<141> 2015-08-21

<150> 62/050,321
<151> 2014-09-15

<150> 62/040,824
<151> 2014-08-22

<160> 3

<170> PatentIn version 3.5

<210> 1
<211> 24
<212> DNA
<213> Artificial Sequence

<220>
<223> Description of Artificial Sequence: Synthetic
      oligonucleotide

<400> 1
tcgtcgtttt gtcgttttgt cgtt                                              24


<210> 2
<211> 20
<212> DNA
<213> Artificial Sequence

<220>
<223> Description of Artificial Sequence: Synthetic
      oligonucleotide


<220>
<221> misc_feature
<222> (1)..(3)
<223> Phosphorothioate linkage between positions

<220>
<221> misc_feature
<222> (15)..(20)
<223> Phosphorothioate linkage between positions

<400> 2
gggggacgat cgtcgggggg                                                   20


<210> 3
<211> 22
<212> DNA

<213> Artificial Sequence

<220>
<223> Description of Artificial Sequence: Synthetic
      oligonucleotide

<400> 3
tcgtcgtttt cggcgcgcgc cg                                                22

## Description

### RELATED APPLICATIONS

This application claims the benefit and priority to U.S. Provisional Patent Application No. 62/040,824, filed August 22, 2014, and to U.S. Provisional Patent Application No. 62/050,321, filed September 15, 2014, the contents of which are hereby incorporated by reference in its entirety.

### FIELD OF THE INVENTION

The invention relates generally to the field of pharmaceutical science. More particularly, the invention relates to compositions useful as pharmaceuticals for altering immune function. More specifically, the invention relates to 2, 4, 6, 7-tetrasubstituted pteridine compounds, including N², N⁴, N⁷, 6-tetrasubstituted pteridine-2,4,7-triamine compounds, and their use in methods for affecting immune stimulation mediated through Toll-like receptor (TLR) molecules.

### BACKGROUND

Stimulation of the immune system, which includes stimulation of either or both innate immunity and adaptive immunity, is a complex phenomenon that can result in either protective or adverse physiologic outcomes for the host. In recent years there has been increased interest in the mechanisms underlying innate immunity, which is believed to initiate and support adaptive immunity. This interest has been fueled in part by the recent discovery of a family of highly conserved pattern recognition receptor proteins known as Toll-like receptors (TLRs) that are believed to be involved in innate immunity as receptors for pathogen-associated molecular patterns (PAMPs) and danger associated molecular patterns (DAMPs). Compositions and methods useful for modulating innate immunity are therefore of great interest, as they may affect therapeutic approaches to conditions involving autoimmunity, inflammation, atherosclerosis, allergy, asthma, graft rejection, graft versus host disease (GvHD), infection, cancer, and immunodeficiency.

Toll-like receptors (TLRs) are a family of pattern recognition and signaling molecules involved in innate immunity. This family includes at least twelve members, designated TLR1 - TLR13, for which the function and specificity are known for most but not all members. Certain of these TLRs are known to signal in response to encounters with particular types of nucleic acid molecules. For example, TLR9 signals in response to CpG-containing DNA, TLR3 signals in response to double-stranded RNA, and TLR7 and TLR8 signal in response to certain single-stranded RNA. There have been a number of reports describing the immunostimulatory effect of certain types of nucleic acid molecules, including CpG nucleic acids and double-stranded RNA. Of note, it was reported that Toll-like receptor 9 (TLR9) recognizes bacterial DNA and CpG DNA while TLR7 and 8 recognize single stranded RNA (Hemmi et al. (2000), Nature 408:740-5; Bauer et al. (2001), Proc Natl Acad Sci USA 98:9237-42; Heil et al. (2004), Science, 303:1526). In addition to their natural ligands, certain synthetic or artificial ligands for these nucleic-acid responsive TLRs are also known. These include certain CpG oligodeoxyribonucleotides (CpG ODN), oligoribonucleotides (ORN) and certain ORN analogs, and certain small molecules including imiquimod (R-837) and resiquimod (R-848). Imiquimod and resiquimod are classified as imidazoaminoquinoline-4-amines; the former is currently marketed as Aldara™ by 3M Pharmaceuticals for topical treatment of anogenital warts associated with papillomavirus infection. In addition to their use in the treatment of certain viral infections such as papillomavirus, certain TLR agonists are also believed to be useful as adjuvants, antitumor agents, and anti-allergy agents. Because a number of diseases and conditions can be treated by enhancing innate immunity, there is a continued need for additional and improved TLR agonists.

It was also recently reported that immune complexes containing IgG and nucleic acid can stimulate TLR9 and participate in B-cell activation in certain autoimmune diseases (Leadbetter et al. (2002), Nature 416:595-8). Similar and additional documentation of these claims have been made for TLR7, 8 and 9 (reviewed in Sun et al. (2007), Inflammation and Allergy - Drug Targets 6:223-235).

### SUMMARY OF THE INVENTION

Compounds useful as immune system modulators comprising a 2, 4, 6, 7-tetrasubstituted pteridine core, including N², N⁴, N⁷, 6-tetrasubstituted pteridine-2,4,7-triamine compounds, are described. The molecules described herein can alter TLR-mediated immuno-stimulatory signaling by inhibiting TLR signaling and thus can be useful as inhibitors of immune stimulation. Methods for synthesizing these compounds are also described herein. Compositions and methods described herein are useful for inhibiting immune stimulation in vitro and in vivo. Such compositions and methods thus are useful in a number of clinical applications, including as pharmaceutical agents and methods for treating conditions involving unwanted immune activity, including inflammatory and autoimmune disorders. The compositions of the invention can also be used in methods for the preparation of medicaments for use in the treatment of conditions involving unwanted immune activity, including a variety of inflammatory and autoimmune disorders.

In one aspect, a compound of Formula I or a pharmaceutically acceptable salt thereof is described, 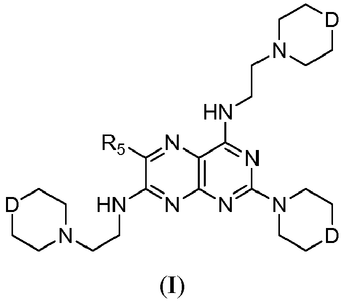 wherein
each occurrence of D is independently -O- or -N(Me)-; and
R₅ is H, F, or Cl.

In any one of the embodiments described herein, the compound has the structure selected from the group consisting of 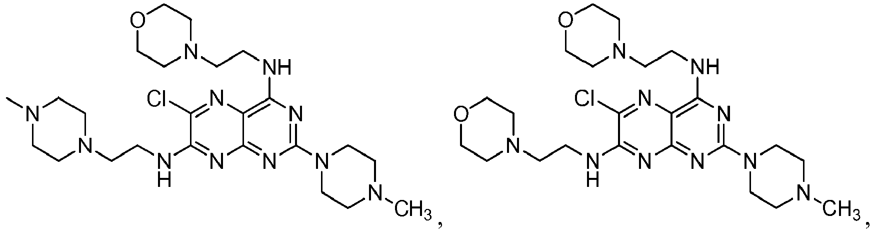 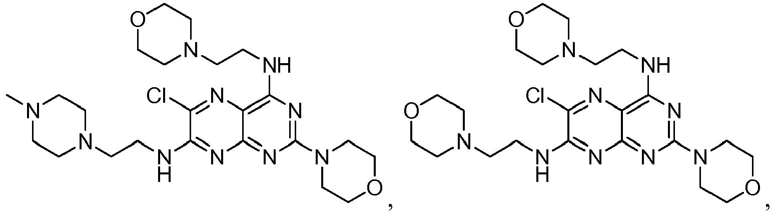 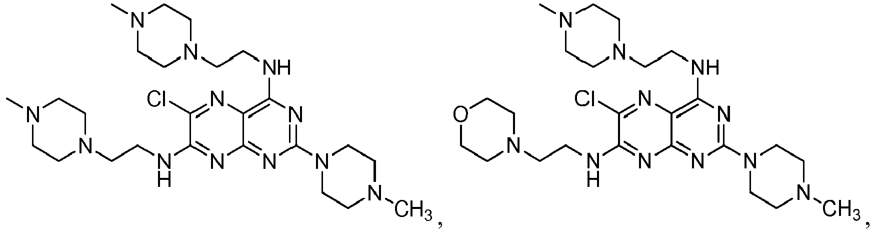 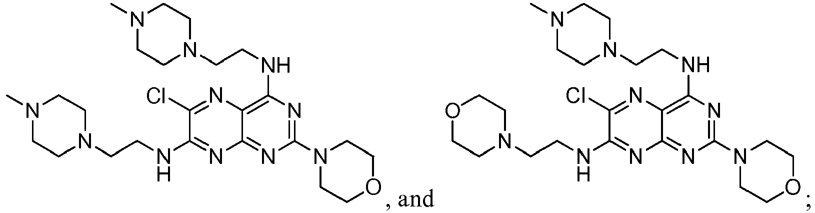 or a pharmaceutically acceptable salt thereof.

In any one of the embodiments described herein, the compound has the structure selected from the group consisting of 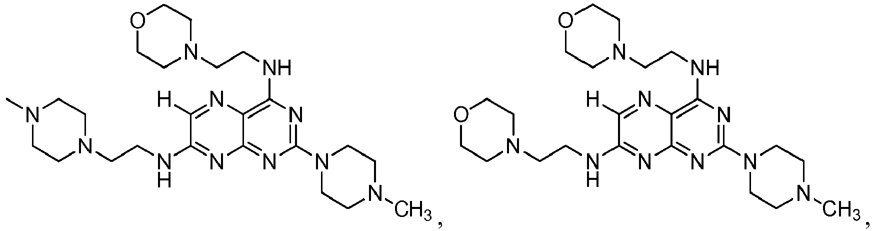 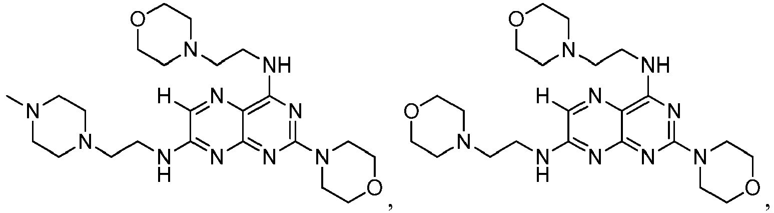 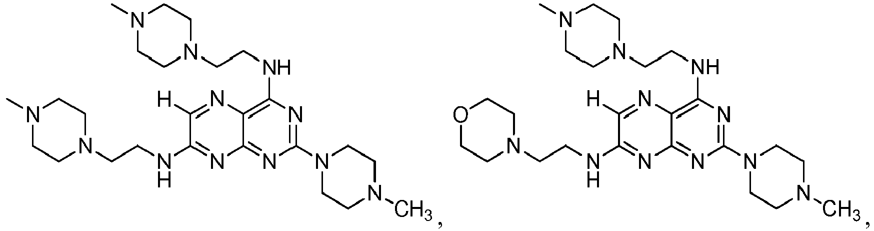 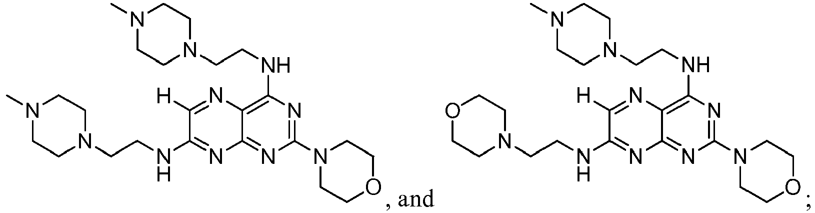 or a pharmaceutically acceptable salt thereof.

In any one of the embodiments described herein, the compound has the structure of 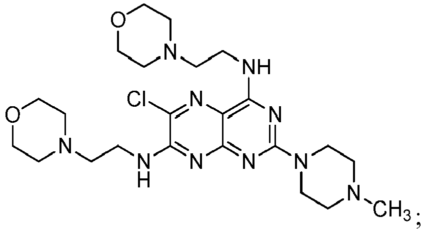 or a pharmaceutically acceptable salt thereof.

In any one of the embodiments described herein, the compound has the structure of 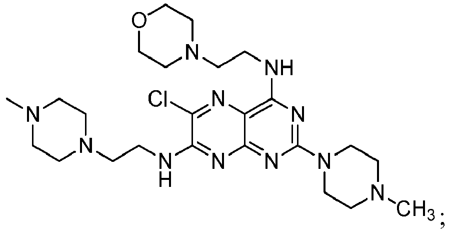 or a pharmaceutically acceptable salt thereof.

In any one of the embodiments described herein, the compound has an IC₅₀ of more than 10, 15, 20, 25, or 30 µM in a standard human ether-a-go-go related gene (hERG) patch clamp assay.

In any one of the embodiments described herein, the compound results in more than 75% hepatocyte viability in a hepatocyte viability assay after the hepatocyte has been exposed to 100 µM of the compound for 24 h.

In another aspect, a compound of Formula Ia or a pharmaceutically acceptable salt thereof is described, 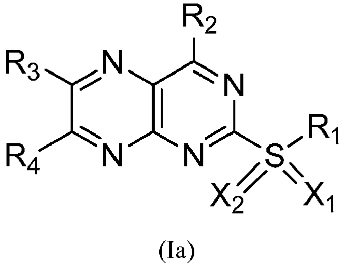 wherein
R₁ is hydrogen, alkyl, alkenyl, cycloalkyl, alkylcycloalkyl, aryl, alkylaryl, heterocycle, or alkylheterocycle;
X₁ and X₂ are each independently absent or O;
R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
provided that when R₂ is ORₐ, SRₐ, NR_{b}R_{c}, or NRₐ(CH₂)ₚNR_{b}R_{c}, at least one of X₁ and X₂ is O.

In any one of the embodiments described herein, R₁ is alkyl, optionally substituted aryl, or optionally substituted heteroaryl.

In any one of the embodiments described herein, X₁ and X₂ are both O.

In any one of the embodiments described herein, R₂ is Cl or Br.

In any one of the embodiments described herein, R₂ is OS(=O)₂Rₐ, or OC(=O)Rₐ.

In any one of the embodiments described herein, R₂ is NR_{b}R_{c} or NRₐ(CH₂)ₚNR_{b}R_{c}.

In any one of the embodiments described herein, R₄ is NR_{b}R_{c} or NRₐ(CH₂)ₚNR_{b}R_{c}.

In any one of the embodiments described herein, R₂ and R₄ are the same or different.

In any one of the embodiments described herein, R₂ and R₄ are each independently selected from the group consisting of: 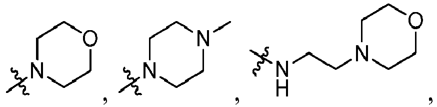 and

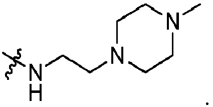

In yet another aspect, a method for the synthesis of a compound having the structure of Formula II is described, 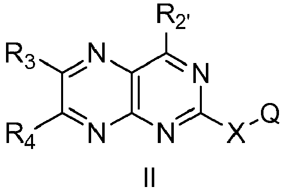 comprising:
(a) converting a compound having the structure of Formula III to a compound having the structure of Formula IV: 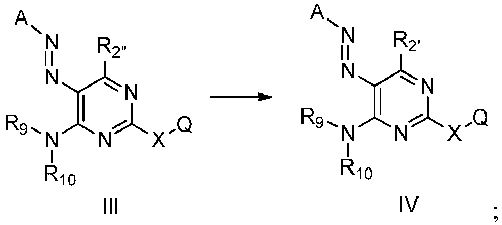 and
(b) converting the compound having the structure of Formula IV to the compound having the structure of Formula II: 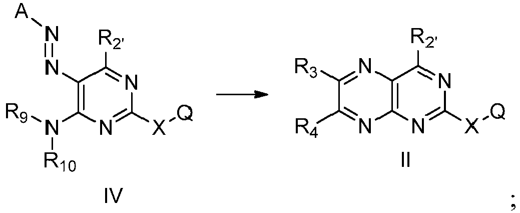 wherein
   each occurrence of X is independently absent or is an alkyl, cycloalkyl, aryl, or heterocycle;
   each occurrence of Q is independently H, (CH₂)_{q}NR_{b}R_{c}, NRₐ(CH₂)ₚNR_{b}R_{c}, OR₁, SR₁, 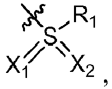 or CRₐR_{b}R_{c}, in which q is 0 or 1 and p is 2-4; and X₁ and X₂ are each independently absent or O;
   R₁ is hydrogen, alkyl, alkenyl, cycloalkyl, alkylcycloalkyl, aryl, alkylaryl, heterocycle, alkylheterocycle;
   R_{2"} is halogen, ORₐ, OS(=O)₂Rₐ, or OC(=O)Rₐ;
   R_{2'} is OH, NR_{b}R_{c}, or NRₐ(CH₂)ₚNR_{b}R_{c};
   A is aryl or heteroaryl;
   each occurrence of R₉ and R₁₀ is each independently hydrogen, OS(=O)₂Rₐ, CH₂C(=O)ORₐ, C(=O)C(=O)ORₐ, OC(=O)Rₐ, OC(=O)ORₐ, or R_{a'}, or alternatively R₉ and R₁₀ are taken together with the nitrogen atom to which that they are attached to form a mono- or bicyclic carbocycle or heterocycle, wherein the carbocycle or heterocycle is optionally substituted with oxo;
   R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}Rₑ, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
   each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   each occurrence of R_{b} and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
   wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl.

In any one of the embodiments described herein, X is absent and Q is (CH₂)_{q}NR_{b}R_{c}, NRₐ(CH₂)ₚNR_{b}R_{c}, OR₁, SR₁, or

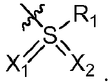

In any one of the embodiments described herein, R_{2'} and R₄ are the same.

In any one of the embodiments described herein, R_{2'} and R₄ are different.

In any one of the embodiments described herein, R₉ and R₁₀ are selected from the group consisting of Fmoc-, Cbz-, Boc-, Ac-, CF₃(C=O)-, Benzyl, triphenylmethyl, and p-Toluenesulfonyl; or R₉ and R₁₀ are taken together with the nitrogen atom to which they are bonded to form

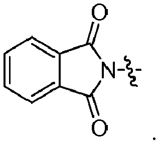

In any one of the embodiments described herein, A is phenyl.

In any one of the embodiments described herein, the method further includes a step of (a₁): 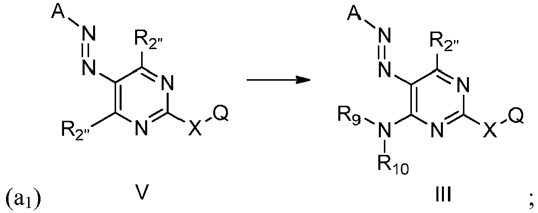 wherein each occurrence of R_{2"} is independent halogen, ORₐ, OS(=O)₂Rₐ, or OC(=O)Rₐ.

In any one of the embodiments described herein, the step (a₁) further comprises the steps of (a₂) and (a₃): 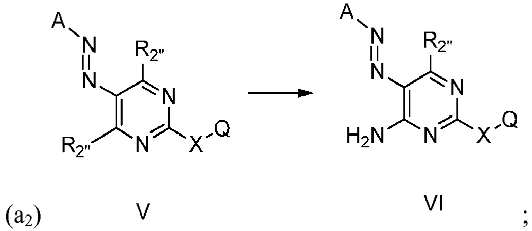 and 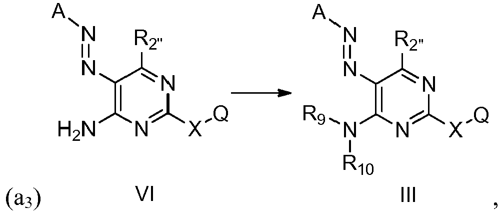 wherein at least one of R₉ and R₁₀ is not hydrogen.

In any one of the embodiments described herein, step (b) further comprises the steps of (b₁) and (b₂): 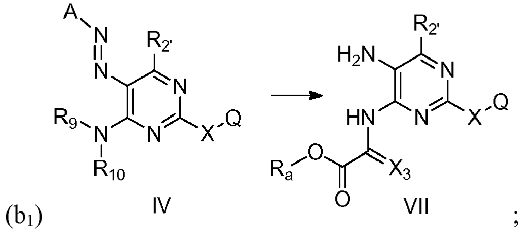 and 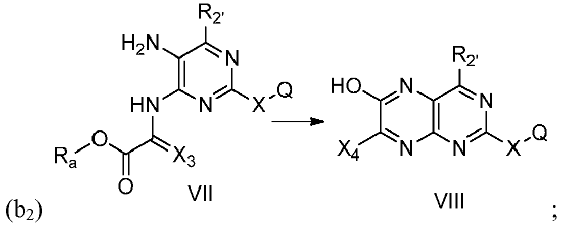 wherein X₃ is O or absent, X₄ is OH or absent, and Rₐ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl.

In any one of the embodiments described herein, R₉ is H and R₁₀ is -(C=O)ORₐ.

In any one of the embodiments described herein, the method further includes the steps of (b₃) and (b₄): 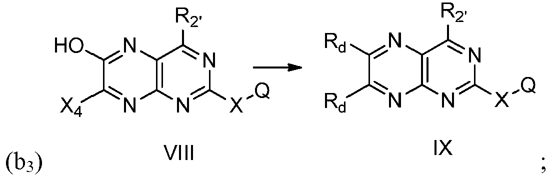 and 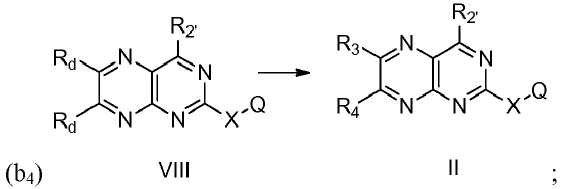 wherein each occurrence of R_{d} is independently H, halogen, OS(=O)₂Rₐ, or OC(=O)Rₐ.

In yet another aspect, a method for the synthesis of a compound having the structure of Formula II is described, 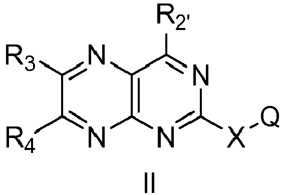 comprising:
(a) converting a compound having the structure of Formula X to a compound having the structure of Formula XI: 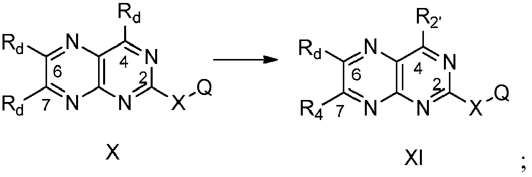 and
(b) converting the compound having the structure of Formula XI to the compound having the structure of Formula II: 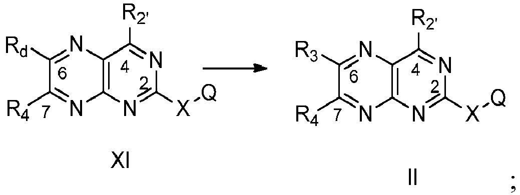 wherein
   each occurrence of X is independently absent or is an alkyl, cycloalkyl, aryl, or heterocycle;
   each occurrence of Q is independently H, R_{d}, (CH₂)_{q}NRₐR_{b}, NRₐ(CH₂)ₚNR_{b}R_{c}, OR₁, SR₁, 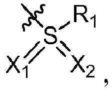 or CRₐR_{b}R_{c}, in which q is 0 or 1 and p is 2-4; and X₁ and X₂ are each independently absent or O;
   R₁ is hydrogen, alkyl, alkenyl, cycloalkyl, alkylcycloalkyl, aryl, alkylaryl, heterocycle, alkylheterocycle;
   each occurrence of R_{d} is independently halogen, OS(=O)₂Rₐ, or OC(=O)Rₐ;
   R_{2'} is OH, NR_{b}R_{c}, or NRₐ(CH₂)ₚNR_{b}R_{c};
   R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
   each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
   wherein the formed heterocycle is optionally substituted by (C₁-C₄)alkyl and one or more carbon atoms in the formed heterocycle are optionally replaced with O, NRg, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   with the proviso that step (b) can be omitted if R_{d} at the 6 position of the compound of Formula XI and R₃ are the same.

In any one of the embodiments described herein, R_{2'} and R₄ are the same.

In any one of the embodiments described herein, R_{2'} and R₄ are different.

In any one of the embodiments described herein, step (a) further comprises steps (a₁) and (a₂): 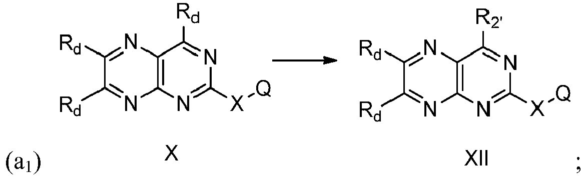 and 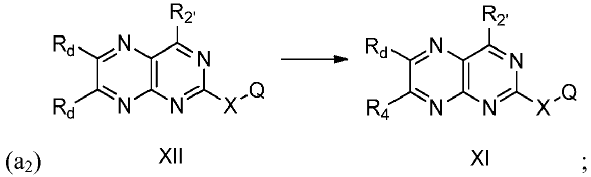 wherein step (a₁) further comprises purifying the compound having the structure of Formula XII.

In any one of the embodiments described herein, the purification is a column chromatography purification or HPLC purification.

In any one of the embodiments described herein, step (a) further comprises steps (a_{1'}) and (a_{2'}): 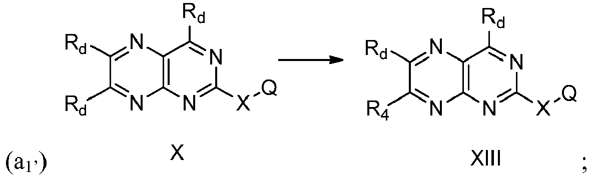 and 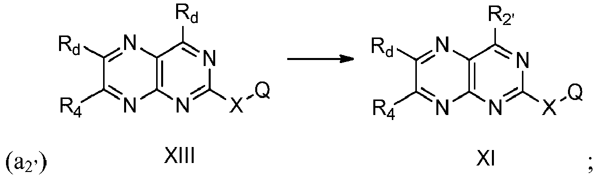 wherein step (a_{1'}) further compresses purifying the compound having the structure of Formula XIII.

In any one of the embodiments described herein, the purification is a column chromatography purification or HPLC purification.

In any one of the embodiments described herein, step (a) comprises a one-pot synthetic step (a₁ₓ): 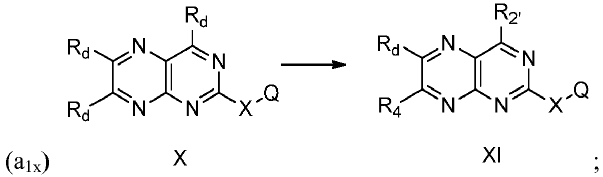 wherein step (a₁ₓ) further comprises purifying the compound having the structure of Formula XI.

In any one of the embodiments described herein, the purification is a column chromatography purification or HPLC purification.

In any one of the embodiments described herein, the substituent -X-Q in Formulae X and XI is R_{d}, and step (a) comprises converting a compound having the structure of Formula X' to a compound having the structure of Formula XI':

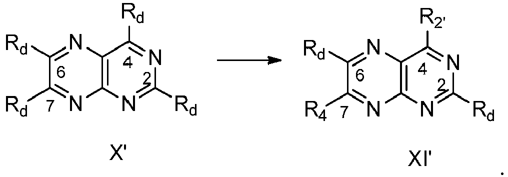

In any one of the embodiments described herein, the method further includes step (a'):
(a') converting a compound having the structure of Formula XI' to a compound having the structure of Formula XI": 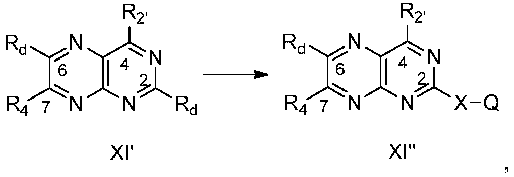 wherein -X-Q is not R_{d}.

In any one of the embodiments described herein, -X-Q is NRₐR_{b}, NRₐ(CH₂)ₚNR_{b}R_{c}, OR₁, or SR₁.

In any one of the embodiments described herein, -X-Q is

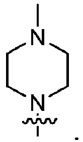

In any one of the embodiments described herein, R_{2'} and R₄ are the same.

In any one of the embodiments described herein, R_{2'} and R₄ are both

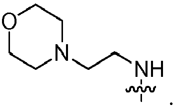

In any one of the embodiments described herein, R_{d} and R₃ are both Cl.

In any one of the embodiments described herein, the method includes the following two steps: 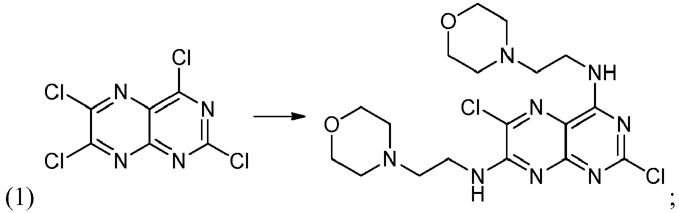 and

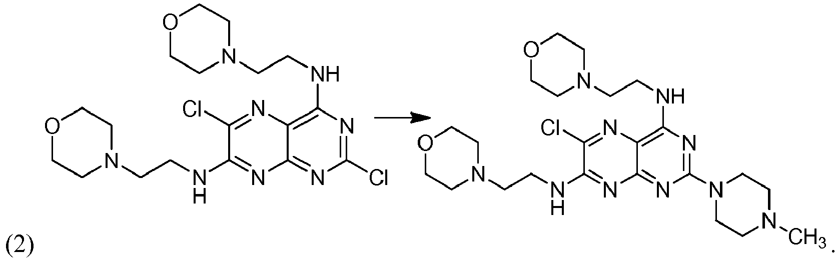

In any one of the embodiments described herein, the method further comprises preparing the compound having the structure of 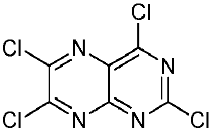 by the following steps:

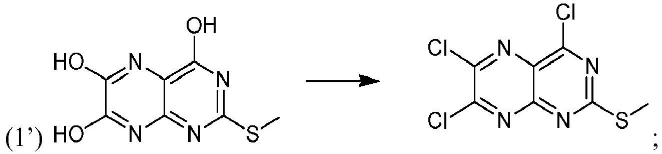

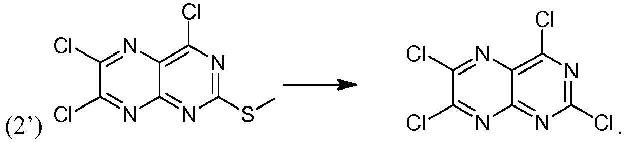

In any one of the embodiments described herein, the method further comprises preparing the compound having the structure of 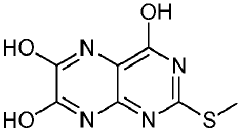 by the following steps: 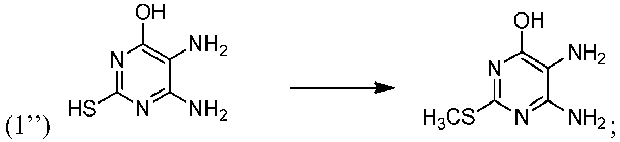 and

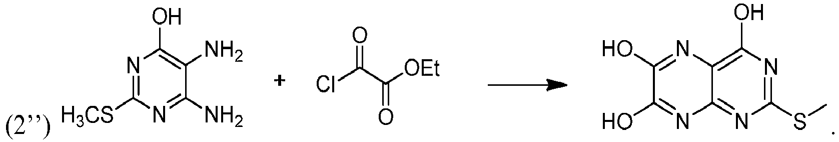

In yet another aspect, a compound is described, selected from the group consisting of: 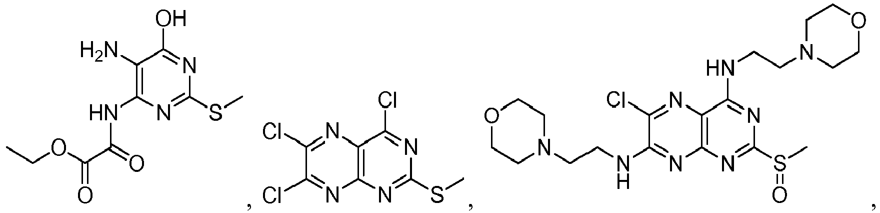 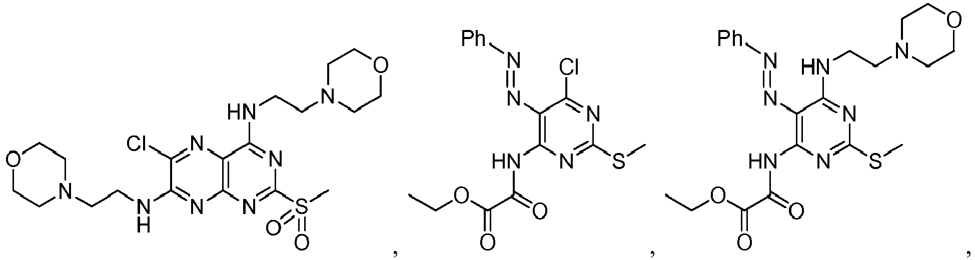 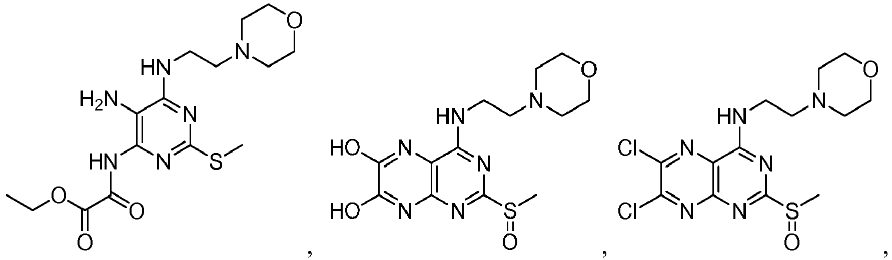 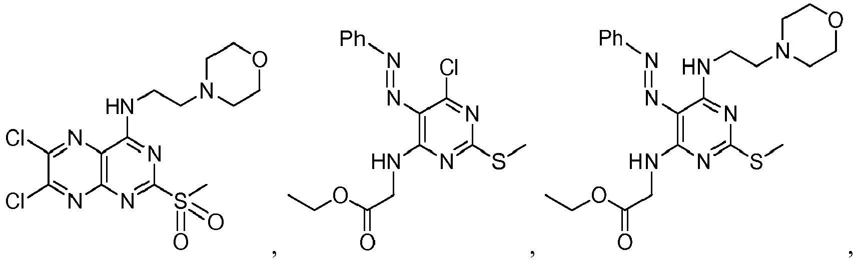 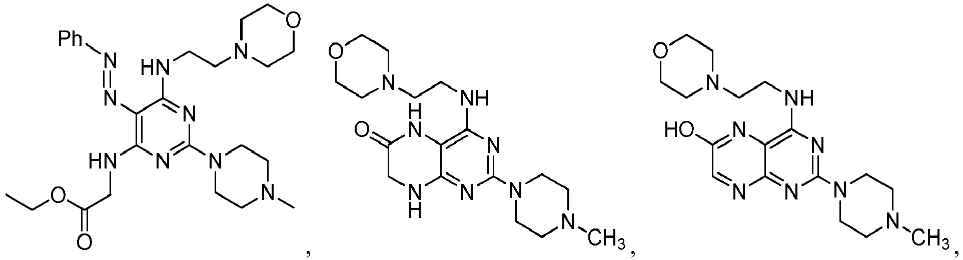 and

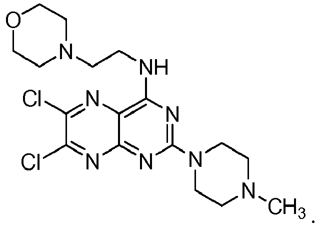

In any one of the embodiments described herein, a compound selected from Table 1 is described.

In yet another aspect, a pharmaceutical composition is described, including at least one compound according to any one of the embodiments described herein and a pharmaceutically acceptable carrier or diluent.

In yet another aspect, a method of treating an autoimmune disease in a mammalian species in need thereof is described, including administering to the mammalian species a therapeutically effective amount of at least one compound according to any one of the embodiments described herein.

In any one of the embodiments described herein, the mammalian species is human.

In any one of the embodiments described herein, the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.

In yet another aspect, a method of inhibiting TLR-mediated immunostimulation in a mammalian species in need thereof is described, including administering to the mammalian species a therapeutically effective amount of at least one compound according to any one of the embodiments described herein.

In any one of the embodiments described herein, the mammalian species is human.

In yet another aspect, a method of inhibiting TLR-mediated immunostimulatory signaling is described, including contacting a cell expressing a TLR with an effective amount of at least one compound according to any one of the embodiments described herein.

In yet another aspect, use of a therapeutically effective amount of at least one compound according any one of the embodiments described herein for the manufacture of a medicament for treating an autoimmune disease in a mammalian species in need thereof is described.

In any one of the embodiments described herein, the mammalian species is human.

In any one of the embodiments described herein, the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.

In yet another aspect, use of a therapeutically effective amount of at least one compound according to any one of the embodiments described herein for the manufacture of a medicament for inhibiting TLR-mediated immunostimulation in a mammalian species in need thereof is described.

In any one of the embodiments described herein, the mammalian species is human.

In yet another aspect, a therapeutically effective amount of at least one compound according to any one of the embodiments described herein for the treatment of an autoimmune disease in a mammalian species in need thereof is described.

In any one of the embodiments described herein, the mammalian species is human.

In any one of the embodiments described herein, the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.

In yet another aspect, a therapeutically effective amount of at least one compound according to any one of the embodiments described herein for the treatment of unwanted TLR-mediated immunostimulation in a mammalian species in need thereof.

In any one of the embodiments described herein, the mammalian species is human.

In yet another aspect, compound, method, or composition as described in the description is disclosed.

### BRIEF DESCRIPTION OF DRAWINGS

FIG. 1 shows hERG patch clamp studies of certain pteridine compounds, according to one or more embodiments.
FIG. 2A shows competitive inhibition studies of JB6121, according to one or more embodiments.
FIG. 2B shows antagonist IC₅₀ studies of JB6121 with escalating agonist challenge doses, according to one or more embodiments.
FIG. 3A shows in vivo TLP9 antagonism studies of JB6121, according to one or more embodiments.
FIG. 3B shows in vivo TLP7 antagonism studies of JB6121 with escalating agonist challenge doses, according to one or more embodiments.
FIG. 4 shows in vivo TLP9 activities of JB6121 in relation to its whole blood exposure, according to one or more embodiments.
FIG. 5 shows Cmax of JB6121 in relation to its oral dosing dosages, according to one or more embodiments.

### FURTHER DESCRIPTION OF THE INVENTION

### Definitions

The following are definitions of terms used in the present specification. The initial definition provided for a group or term herein applies to that group or term throughout the present specification individually or as part of another group, unless otherwise indicated. Unless otherwise defined, all technical and scientific terms used herein have the same meaning as commonly understood by one of ordinary skill in the art.

The terms "alkyl" and "alk" refer to a straight or branched chain alkane (hydrocarbon) radical containing from 1 to 12 carbon atoms, preferably 1 to 6 carbon atoms. Exemplary "alkyl" groups include methyl, ethyl, propyl, isopropyl, n-butyl, t-butyl, isobutyl pentyl, hexyl, isohexyl, heptyl, 4,4-dimethylpentyl, octyl, 2,2,4-trimethylpentyl, nonyl, decyl, undecyl, dodecyl, and the like. The term "(C₁-C₄) alkyl" refers to a straight or branched chain alkane (hydrocarbon) radical containing from 1 to 4 carbon atoms, such as methyl, ethyl, propyl, isopropyl, n-butyl, t-butyl, and isobutyl. "Substituted alkyl" refers to an alkyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. In the aforementioned exemplary substituents, groups such as alkyl, cycloalkyl, alkenyl, alkynyl, cycloalkenyl, heterocycle and aryl can themselves be optionally substituted.

The term "alkenyl" refers to a straight or branched chain hydrocarbon radical containing from 2 to 12 carbon atoms and at least one carbon-carbon double bond. Exemplary such groups include ethenyl or allyl. The term "C₂-C₆ alkenyl" refers to a straight or branched chain hydrocarbon radical containing from 2 to 6 carbon atoms and at least one carbon-carbon double bond, such as ethylenyl, propenyl, 2-propenyl, (*E*)-but-2-enyl, (*Z*)-but-2-enyl, 2-methy(*E*)-but-2-enyl, 2-methy(*Z*)-but-2-enyl, 2,3-dimethy-but-2-enyl, (*Z*)-pent-2-enyl, (*E*)-pent-1-enyl, (*Z*)-hex-1-enyl, (*E*)-pent-2-enyl, (*Z*)-hex-2-enyl, (*E*)-hex-2-enyl, (*Z*)-hex-1-enyl, (*E*)-hex-1-enyl,, (*Z*)-hex-3-enyl, (*E*)-hex-3-enyl, and (*E*)-hex-1,3-dienyl. "Substituted alkenyl" refers to an alkenyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted.

The term "alkynyl" refers to a straight or branched chain hydrocarbon radical containing from 2 to 12 carbon atoms and at least one carbon to carbon triple bond. Exemplary such groups include ethynyl. The term "C₂-C₆ alkynyl" refers to a straight or branched chain hydrocarbon radical containing from 2 to 6 carbon atoms and at least one carbon-carbon triple bond, such as ethynyl, prop-1-ynyl, prop-2-ynyl, but-1-ynyl, but-2-ynyl, pent-1-ynyl, pent-2-ynyl, hex-1-ynyl, hex-2-ynyl, hex-3-ynyl. "Substituted alkynyl" refers to an alkynyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted.

The term "cycloalkyl" refers to a fully saturated cyclic hydrocarbon group containing from 1 to 4 rings and 3 to 8 carbons per ring. "C₃-C₇ cycloalkyl" refers to cyclopropyl, cyclobutyl, cyclopentyl, cyclohexyl, or cycloheptyl. "Substituted cycloalkyl" refers to a cycloalkyl group substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cylic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "cycloalkenyl" refers to a partially unsaturated cyclic hydrocarbon group containing 1 to 4 rings and 3 to 8 carbons per ring. Exemplary such groups include cyclobutenyl, cyclopentenyl, cyclohexenyl, etc. "Substituted cycloalkenyl" refers to a cycloalkenyl group substituted with one more substituents, preferably 1 to 4 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cylic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "aryl" refers to cyclic, aromatic hydrocarbon groups that have 1 to 5 aromatic rings, especially monocyclic or bicyclic groups such as phenyl, biphenyl or naphthyl. Where containing two or more aromatic rings (bicyclic, *etc*.), the aromatic rings of the aryl group may be joined at a single point (*e.g.,* biphenyl), or fused (*e.g.,* naphthyl, phenanthrenyl and the like). "Substituted aryl" refers to an aryl group substituted by one or more substituents, preferably 1 to 3 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include fused cylic groups, especially fused cycloalkyl, fused cycloalkenyl, fused heterocycle, or fused aryl, where the aforementioned cycloalkyl, cycloalkenyl, heterocycle and aryl substituents can themselves be optionally substituted.

The term "carbocycle" refers to a fully saturated or partially saturated cyclic hydrocarbon group containing from 1 to 4 rings and 3 to 8 carbons per ring, or cyclic, aromatic hydrocarbon groups that have 1 to 5 aromatic rings, especially monocyclic or bicyclic groups such as phenyl, biphenyl or naphthyl. The term "carbocycle" encompasses cycloalkyl, cycloalkenyl, cycloalkynyl and aryl as defined hereinabove. The term "substituted carbocycle" refers to carbocycle or carbocyclic groups substituted with one or more substituents, preferably 1 to 4 substituents, at any available point of attachment. Exemplary substituents include, but are not limited to, those described above for substituted cycloalkyl, substituted cycloalkenyl, substituted cycloalkynyl and substituted aryl. Exemplary substituents also include spiro-attached or fused cyclic substituents at any available point or points of attachment, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The terms "heterocycle" and "heterocyclic" refer to fully saturated, or partially or fully unsaturated, including aromatic (*i.e.,* "heteroaryl") cyclic groups (for example, 4 to 7 membered monocyclic, 7 to 11 membered bicyclic, or 8 to 16 membered tricyclic ring systems) which have at least one heteroatom in at least one carbon atom-containing ring. Each ring of the heterocyclic group containing a heteroatom may have 1, 2, 3, or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. (The term "heteroarylium" refers to a heteroaryl group bearing a quaternary nitrogen atom and thus a positive charge.) The heterocyclic group may be attached to the remainder of the molecule at any heteroatom or carbon atom of the ring or ring system. Exemplary monocyclic heterocyclic groups include azetidinyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, hexahydrodiazepinyl, 4-piperidonyl, pyridyl, pyrazinyl, pyrimidinyl, pyridazinyl, triazinyl, triazolyl, tetrazolyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like. Exemplary bicyclic heterocyclic groups include indolyl, isoindolyl, benzothiazolyl, benzoxazolyl, benzoxadiazolyl, benzothienyl, benzo[d][1,3]dioxolyl, 2,3-dihydrobenzo[b][1,4]dioxinyl, quinuclidinyl, quinolinyl, tetrahydroisoquinolinyl, isoquinolinyl, benzimidazolyl, benzopyranyl, indolizinyl, benzofuryl, benzofurazanyl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl] or furo[2,3-b]pyridinyl), dihydroisoindolyl, dihydroquinazolinyl (such as 3,4-dihydro-4-oxo-quinazolinyl), triazinylazepinyl, tetrahydroquinolinyl and the like. Exemplary tricyclic heterocyclic groups include carbazolyl, benzidolyl, phenanthrolinyl, acridinyl, phenanthridinyl, xanthenyl and the like.

"Substituted heterocycle" and "substituted heterocyclic" (such as "substituted heteroaryl") refer to heterocycle or heterocyclic groups substituted with one or more substituents, preferably 1 to 4 substituents, at any available point of attachment. Exemplary substituents include but are not limited to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cyclic substituents at any available point or points of attachment, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "alkylamino" refers to a group having the structure -NHR', wherein R' is hydrogen, alkyl or substituted alkyl, cycloalkyl or substituted cyclolakyl, as defined herein. Examples of alkylamino groups include, but are not limited to, methylamino, ethylamino, n-propylamino, iso-propylamino, cyclopropylamino, n-butylamino, tert-butylamino, neopentylamino, n-pentylamino, hexylamino, cyclohexylamino, and the like.

The term "dialkylamino" refers to a group having the structure -NRR', wherein R and R' are each independently alkyl or substituted alkyl, cycloalkyl or substituted cycloalkyl, cycloalkenyl or substituted cyclolalkenyl, aryl or substituted aryl, heterocylyl or substituted heterocyclyl, as defined herein. R and R' may be the same or different in a dialkyamino moiety. Examples of dialkylamino groups include, but are not limited to, dimethylamino, methyl ethylamino, diethylamino, methylpropylamino, di(n-propyl)amino, di(iso-propyl)amino, di(cyclopropyl)amino, di(n-butyl)amino, di(tert-butyl)amino, di(neopentyl)amino, di(n-pentyl)amino, di(hexyl)amino, di(cyclohexyl)amino, and the like. In certain embodiments, R and R' are linked to form a cyclic structure. The resulting cyclic structure may be aromatic or non-aromatic. Examples of cyclic diaminoalkyl groups include, but are not limited to, aziridinyl, pyrrolidinyl, piperidinyl, morpholinyl, pyrrolyl, imidazolyl, 1,3,4-trianolyl, and tetrazolyl.

The terms "halogen" or "halo" refer to chlorine, bromine, fluorine or iodine.

Unless otherwise indicated, any heteroatom with unsatisfied valences is assumed to have hydrogen atoms sufficient to satisfy the valences.

The compounds of the present invention may form salts which are also within the scope of this invention. Reference to a compound of the present invention is understood to include reference to salts thereof, unless otherwise indicated. The term "salt(s)", as employed herein, denotes acidic and/or basic salts formed with inorganic and/or organic acids and bases. In addition, when a compound of the present invention contains both a basic moiety, such as but not limited to a pyridine or imidazole, and an acidic moiety such as but not limited to a carboxylic acid, zwitterions ("inner salts") may be formed and are included within the term "salt(s)" as used herein. Pharmaceutically acceptable (*i.e.,* non-toxic, physiologically acceptable) salts are preferred, although other salts are also useful, *e.g.,* in isolation or purification steps which may be employed during preparation. Salts of the compounds of the present invention may be formed, for example, by reacting a compound described herein with an amount of acid or base, such as an equivalent amount, in a medium such as one in which the salt precipitates or in an aqueous medium followed by lyophilization.

The compounds of the present invention which contain a basic moiety, such as but not limited to an amine or a pyridine or imidazole ring, may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (*e.g.,* 2-hydroxyethanesulfonates), lactates, maleates, methanesulfonates, naphthalenesulfonates (*e.g.,* 2-naphthalenesulfonates), nicotinates, nitrates, oxalates, pectinates, persulfates, phenylpropionates (*e.g*., 3-phenylpropionates), phosphates, picrates, pivalates, propionates, salicylates, succinates, sulfates (such as those formed with sulfuric acid), sulfonates, tartrates, thiocyanates, toluenesulfonates such as tosylates, undecanoates, and the like.

The compounds of the present invention which contain an acidic moiety, such but not limited to a carboxylic acid, may form salts with a variety of organic and inorganic bases. Exemplary basic salts include ammonium salts, alkali metal salts such as sodium, lithium and potassium salts, alkaline earth metal salts such as calcium and magnesium salts, salts with organic bases (for example, organic amines) such as benzathines, dicyclohexylamines, hydrabamines (formed with N,N-bis(dehydroabietyl) ethylenediamine), N-methyl-D-glucamines, N-methyl-D-glycamides, t-butyl amines, and salts with amino acids such as arginine, lysine and the like. Basic nitrogen-containing groups may be quaternized with agents such as lower alkyl halides (*e.g*., methyl, ethyl, propyl, and butyl chlorides, bromides and iodides), dialkyl sulfates (*e.g*., dimethyl, diethyl, dibutyl, and diamyl sulfates), long chain halides (*e.g.,* decyl, lauryl, myristyl and stearyl chlorides, bromides and iodides), aralkyl halides (*e.g.,* benzyl and phenethyl bromides), and others.

Prodrugs and solvates of the compounds of the invention are also contemplated herein. The term "prodrug" as employed herein denotes a compound that, upon administration to a subject, undergoes chemical conversion by metabolic or chemical processes to yield a compound of the present invention, or a salt and/or solvate thereof. Solvates of the compounds of the present invention include, for example, hydrates.

Compounds of the present invention, and salts or solvates thereof, may exist in their tautomeric form (for example, as an amide or imino ether). All such tautomeric forms are contemplated herein as part of the present invention.

All stereoisomers of the present compounds (for example, those which may exist due to asymmetric carbons on various substituents), including enantiomeric forms and diastereomeric forms, are contemplated within the scope of this invention. Individual stereoisomers of the compounds of the invention may, for example, be substantially free of other isomers (*e.g*., as a pure or substantially pure optical isomer having a specified activity), or may be admixed, for example, as racemates or with all other, or other selected, stereoisomers. The chiral centers of the present invention may have the S or R configuration as defined by the International Union of Pure and Applied Chemistry (IUPAC) 1974 Recommendations. The racemic forms can be resolved by physical methods, such as, for example, fractional crystallization, separation or crystallization of diastereomeric derivatives or separation by chiral column chromatography. The individual optical isomers can be obtained from the racemates by any suitable method, including without limitation, conventional methods, such as, for example, salt formation with an optically active acid followed by crystallization.

Compounds of the present invention are, subsequent to their preparation, preferably isolated and purified to obtain a composition containing an amount by weight equal to or greater than 90%, for example, equal to greater than 95%, equal to or greater than 99% of the compounds ("substantially pure" compounds), which is then used or formulated as described herein. Such "substantially pure" compounds of the present invention are also contemplated herein as part of the present invention.

All configurational isomers of the compounds of the present invention are contemplated, either in admixture or in pure or substantially pure form. The definition of compounds of the present invention embraces both *cis* (*Z*) and *trans* (*E*) alkene isomers, as well as *cis* and *trans* isomers of cyclic hydrocarbon or heterocyclic rings.

Throughout the specification, groups and substituents thereof may be chosen to provide stable moieties and compounds.

Definitions of specific functional groups and chemical terms are described in more detail below. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in "Organic Chemistry", Thomas Sorrell, University Science Books, Sausalito (1999), the entire contents of which are incorporated herein by reference.

Certain compounds of the present invention may exist in particular geometric or stereoisomeric forms. The present invention contemplates all such compounds, including *cis-* and *trans*-isomers, *R-* and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

Isomeric mixtures containing any of a variety of isomer ratios may be utilized in accordance with the present invention. For example, where only two isomers are combined, mixtures containing 50:50, 60:40, 70:30, 80:20, 90:10, 95:5, 96:4, 97:3, 98:2, 99:1, or 100:0 isomer ratios are all contemplated by the present invention. Those of ordinary skill in the art will readily appreciate that analogous ratios are contemplated for more complex isomer mixtures.

The present invention also includes isotopically labeled compounds, which are identical to the compounds disclosed herein, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, or an enantiomer, diastereomer, tautomer, or pharmaceutically acceptable salt or solvate thereof, which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, *i.e.,* ³H, and carbon-14, *i.e.,* ¹⁴C, isotopes are particularly preferred for their ease of preparation and detectability. Further, substitution with heavier isotopes such as deuterium, *i.e.,* ²H, can afford certain therapeutic advantages resulting from greater metabolic stability, for example increased in vivo half-life or reduced dosage requirements and, hence, may be preferred in some circumstances. Isotopically labeled compounds can generally be prepared by carrying out the procedures disclosed in the Schemes and/or in the Examples below, by substituting a readily available isotopically labeled reagent for a non-isotopically labeled reagent.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be appreciated that the compounds, as described herein, may be substituted with any number of substituents or functional moieties. In general, the term "substituted" whether preceded by the term "optionally" or not, and substituents contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. For purposes of this invention, heteroatoms such as nitrogen may have hydrogen substituents and/or any permissible substituents of organic compounds described herein which satisfy the valencies of the heteroatoms. Furthermore, this invention is not intended to be limited in any manner by the permissible substituents of organic compounds. Combinations of substituents and variables envisioned by this invention are preferably those that result in the formation of stable compounds useful in the treatment, for example, of infectious diseases or proliferative disorders. The term "stable", as used herein, preferably refers to compounds which possess stability sufficient to allow manufacture and which maintain the integrity of the compound for a sufficient period of time to be detected and preferably for a sufficient period of time to be useful for the purposes detailed herein.

As used herein, the term "adaptive immune response" refers to any type of antigen-specific immune response. Adaptive immune responses, which are also known in the art as specific immune responses, involve lymphocytes are also characterized by immunological memory, whereby response to a second or subsequent exposure to antigen is more vigorous than the response to a first exposure to the antigen. The term adaptive immune response encompasses both humoral (antibody) immunity and cell-mediated (cellular) immunity.

As used herein, "allergy" refers to acquired hypersensitivity to a substance (allergen). Allergic conditions include eczema, allergic rhinitis or coryza, hay fever, asthma, urticaria (hives) and food allergies, and other atopic conditions.

As used herein, the term "antigenic substance" refers to any substance that induces an adaptive (specific) immune response. An antigen typically is any substance that can be specifically bound by a T-cell antigen receptor, antibody, or B-cell antigen receptor. Antigenic substances include, without limitation, peptides, proteins, carbohydrates, lipids, phospholipids, nucleic acids, autacoids, and hormones. Antigenic substances further specifically include antigens that are classified as allergens, cancer antigens, and microbial antigens.

As used herein, "asthma" refers to a disorder of the respiratory system characterized by inflammation, narrowing of the airways and increased reactivity of the airways to inhaled agents. Asthma is frequently, although not exclusively associated with atopic or allergic symptoms. For example, asthma can be precipitated by exposure to an allergen, exposure to cold air, respiratory infection, and exertion.

As used herein, the terms "autoimmune disease" and, equivalently, "autoimmune disorder" and "autoimmunity", refer to immunologically mediated acute or chronic injury to a tissue or organ derived from the host. The terms encompass both cellular and antibody-mediated autoimmune phenomena, as well as organ-specific and organ-nonspecific autoimmunity. Autoimmune diseases include insulin-dependent diabetes mellitus, rheumatoid arthritis, systemic lupus erythematosus, multiple sclerosis, atherosclerosis, psoriasis and inflammatory bowel disease. Autoimmune diseases also include, without limitation, ankylosing spondylitis, autoimmune hemolytic anemia, Beget's syndrome, Goodpasture's syndrome, Graves' disease, Guillain-Barre syndrome, Hashimoto's thyroiditis, idiopathic thrombocytopenia, myasthenia gravis, pernicious anemia, polyarteritis nodosa, polymyositis/dermatomyositis, primary biliary sclerosis, sarcoidosis, sclerosing cholangitis, Sjögren's syndrome, systemic sclerosis (scleroderma and CREST syndrome), Takayasu's arteritis, temporal arteritis, and Wegener's granulomatosis. Autoimmune diseases also include certain immune complex-associated diseases.

As used herein, the terms "cancer" and, equivalently, "tumor" refer to a condition in which abnormally replicating cells of host origin are present in a detectable amount in a subject. The cancer can be a malignant or non-malignant cancer. Cancers or tumors include but are not limited to biliary tract cancer; brain cancer; breast cancer; cervical cancer; choriocarcinoma; colon cancer; endometrial cancer; esophageal cancer; gastric (stomach) cancer; intraepithelial neoplasms; leukemias; lymphomas; liver cancer; lung cancer (*e.g*., small cell and non-small cell); melanoma; neuroblastomas; oral cancer; ovarian cancer; pancreatic cancer; prostate cancer; rectal cancer; renal (kidney) cancer; sarcomas; skin cancer; testicular cancer; thyroid cancer; as well as other carcinomas and sarcomas. Cancers can be primary or metastatic.

As used herein, the term "CpG DNA" refers to an immunostimulatory nucleic acid which contains a cytosine-guanine (CG) dinucleotide, the C residue of which is unmethylated. The effects of CpG nucleic acids on immune modulation have been described extensively in U. S. patents such as U.S. Pat. No. 6,194,388; U.S. Pat. No. 6,207,646; U.S. Pat. No. 6,239,116; and U.S. Pat. No. 6,218,371, and published international patent applications, such as WO98/37919, WO98/40100, WO98/52581, and WO99/56755. The entire contents of each of these patents and published patent applications are hereby incorporated by reference. The entire immunostimulatory nucleic acid can be unmethylated or portions may be unmethylated but at least the C of the 5'-CG-3' must be unmethylated.

In one embodiment the CpG DNA is a CpG ODN that has a base sequence provided by 5'-TCGTCGTTTTGTCGTTTTGTCGTT-3' (ODN 2006; SEQ ID NO:1). CpG ODN have been further classified by structure and function into at least the following three classes or types, all of which are intended to be encompassed within the term CpG DNA as used herein: B-class CpG ODN such as ODN 2006 include the originally described immunostimulatory CpG ODN and characteristically activate B cells and NK cells but do not induce or only weakly induce expression of type I interferon (*e.g*., IFN-a). A-class CpG ODN, described in published PCT international application WO 01/22990, incorporate a CpG motif, include a chimeric phosphodiester/phosphorothioate backbone, and characteristically activate NK cells and induce plasmacytoid dendritic cells to express large amounts of IFN-α but do not activate or only weakly activate B cells. An example of an A-class CpG ODN is 5'-G*G*GGGACGATCGTCG*G*G*G*G*G-3' (ODN 2216, SEQ ID NO: 2), wherein "*" represents phosphorothioate and "" represents phosphodiester. C-class CpG ODN incorporate a CpG, include a wholly phosphorothioate backbone, include a GC-rich palindromic or nearly-palindromic region, and are capable of both activating B cells and inducing expression of IFN-α. C-class CpG ODN has been described, for example, in published U.S. patent application US2003/0148976. An example of a C-class CpG ODN is 5'-TCGTCGTTTTCGGCGCGCGCCG-3' (ODN 2395; SEQ ID NO : 3). For a review of the various classes of CpG ODN, see also Vollmer et al. (2004), Eur J Immunol 34 : 251-62.

As used herein, "cytokine" refers to any of a number of soluble proteins or glycoproteins that act on immune cells through specific receptors to affect the state of activation and function of the immune cells. Cytokines include interferons, interleukins, tumor necrosis factor, transforming growth factor beta, colony-stimulating factors (CSFs), chemokines, as well as others. Various cytokines affect innate immunity, acquired immunity, or both. Cytokines specifically include, without limitation, IFN-α, IFN-β, IFN-γ, IL-1, IL-2, IL-3, IL-4, IL-5, IL-6, IL-9, IL-10, IL-12, IL-13, IL-18, TNF-α, TGF-β, granulocyte colony-stimulating factor (G-CSF), and granulocyte-macrophage colony-stimulating factor (GM-CSF). Chemokines specifically include, without limitation, IL-8, IP-10, I-TAC, RANTES, MIP-1α, MIP-1β, Gro-α, Gro-β, Gro-γ, MCP-1, MCP-2, and MCP-3.

Most mature CD4+ T helper cells can be categorized into cytokine-associated, cross-regulatory subsets or phenotypes: Th1, Th2, Th17, or Treg. Th1 cells are associated with IL-2, IL-3, IFN, GM-CSF and high levels of TNF-α. Th2 cells are associated with IL-3, IL-4, IL-5, IL-6, IL-9, IL-10, IL-13, GM-CSF and low levels of TNF-α. The Th1 subset promotes both cell-mediated immunity and humoral immunity that is characterized by immunoglobulin class switching to IgG2a in mice. Th1 responses can also be associated with delayed-type hypersensitivity and autoimmune disease. The Th2 subset induces primarily humoral immunity and induces immunoglobulin class switching to IgE and IgG1 in mice. The antibody isotypes associated with Th1 responses generally have good neutralizing and opsonizing capabilities, whereas those associated with Th2 responses are associated more with allergic responses.

Several factors have been shown to influence commitment to Th1 or Th2 profiles. The best characterized regulators are cytokines. IL-12 and IFN-γ are positive Th1 and negative Th2 regulators. IL-12 promotes IFN-γ production, and IFN-γ provides positive feedback for IL-12. IL-4 and IL-10 appear to be required for the establishment of the Th2 cytokine profile and to down-regulate Th1 cytokine production; the effects of IL-4 are in some cases dominant over those of IL-12. IL-13 was shown to inhibit expression of inflammatory cytokines, including IL-12 and TNF-α by LPS-induced monocytes, in a way similar to IL-4.

As used herein, "effective amount" refers to any amount that is necessary or sufficient for achieving or promoting a desired outcome. In some instances an effective amount is a therapeutically effective amount. A therapeutically effective amount is any amount that is necessary or sufficient for promoting or achieving a desired biological response in a subject. The effective amount for any particular application can vary depending on such factors as the disease or condition being treated, the particular agent being administered, the size of the subject, or the severity of the disease or condition. One of ordinary skill in the art can empirically determine the effective amount of a particular agent without necessitating undue experimentation.

As used herein, "graft rejection" refers to immunologically mediated hyperacute, acute, or chronic injury to a tissue or organ derived from a source other than the host. The term thus encompasses both cellular and antibody-mediated rejection, as well as rejection of both allografts and xenografts.

As used herein, the term "immune cell" refers to a cell belonging to the immune system. Immune cells include T lymphocytes (T cells), B lymphocytes (B cells), natural killer (NK) cells, granulocytes, neutrophils, macrophages, monocytes, dendritic cells, and specialized forms of any of the foregoing, *e.g*., plasmacytoid dendritic cells, plasma cells, NKT, T helper, and cytotoxic T lymphocytes (CTL).

As used herein, the term "immune complex" refers to any conjugate including an antibody and an antigen specifically bound by the antibody. In one embodiment, the antigen is an autoantigen.

As used herein, the term "immune complex comprising a nucleic acid" refers to any conjugate including an antibody and a nucleic acid-containing antigen specifically bound by the antibody. The nucleic acid-containing antigen can include chromatin, ribosomes, small nuclear proteins, histones, nucleosomes, DNA, RNA, or any combination thereof. The antibody can but need not necessarily bind specifically to a nucleic acid component of the nucleic acid-containing antigen. In some embodiments, the term "immune complex comprising a nucleic acid" refers also to non-antibody complexes such as HMGB1, LL-37, and other nucleic acid binding proteins such as histones, transcription factors and enzymes complexed with nucleic acids.

As used herein, the term "immune complex-associated disease" refers to any disease characterized by the production and/or tissue deposition of immune complexes, including, but not limited to systemic lupus erythematosus (SLE) and related connective tissue diseases, rheumatoid arthritis, hepatitis C-and hepatitis B-related immune complex disease (*e.g.,* cryoglobulinemia), Beget's syndrome, autoimmune glomerulonephritides, and vasculopathy associated with the presence of LDL/anti-LDL immune complexes.

As used herein, "immunodeficiency" refers to a disease or disorder in which the subject's immune system is not functioning in normal capacity or in which it would be useful to boost a subject's immune response for example to eliminate a tumor or cancer (*e.g.,* tumors of the brain, lung (*e.g*., small cell and non-small cell), ovary, breast, prostate, colon, as well as other carcinomas and sarcomas) or an infection in a subject. The immunodeficiency can be acquired or it can be congenital.

As used herein, "immunostimulatory nucleic acid-associated response in a subject" refers to a measurable response in a subject associated with administration to the subject of an immunostimulatory nucleic acid. Such responses include, without limitation, elaboration of cytokines, chemokines, growth factors, or immunoglobulin; expression of immune cell surface activation markers; Thl/Th2 skewing; and clinical disease activity.

As used herein, the terms "infection" and, equivalently, "infectious disease" refer to a condition in which an infectious organism or agent is present in a detectable amount in the blood or in a normally sterile tissue or normally sterile compartment of a subject. Infectious organisms and agents include viruses, bacteria, fungi, and parasites. The terms encompass both acute and chronic infections, as well as sepsis.

As used herein, the term "innate immune response" refers to any type of immune response to certain pathogen-associated molecular patterns (PAMPs) or danger associated molecular patterns (DAMPs). Innate immunity, which is also known in the art as natural or native immunity, involves principally neutrophils, granulocytes, mononuclear phagocytes, dendritic cells, NKT cells, and NK cells. Innate immune responses can include, without limitation, type I interferon production (*e.g*. , IFN-α), neutrophil activation, macrophage activation, phagocytosis, opsonization, complement activation, and any combination thereof.

As used herein, the term "self-DNA" refers to any DNA derived from the genome of a host subject. In one embodiment, self-DNA includes complementary DNA (cDNA) derived from a host subject. Self-DNA includes intact and degraded DNA.

As used herein, the term "self-RNA" refers to any RNA derived from the genome of a host subject. In one embodiment self-RNA is a messenger RNA (mRNA) derived from a host subject. In another embodiment self-RNA is a regulatory RNA such as micro RNAs. In one embodiment self-RNA includes ribosomal RNA (rRNA) derived from a host subject. Self-RNA includes intact and degraded RNA.

As used herein, the term "subject" refers to a vertebrate animal. In one embodiment the subject is a mammal. In one embodiment the subject is a human. In other embodiments the subject is a non-human vertebrate animal, including, without limitation, non-human primates, laboratory animals, livestock, racehorses, domesticated animals, and non-domesticated animals.

As used herein, "subject having or at risk of developing TLR-mediated immunostimulation" refers to a subject exposed to or at risk of exposure to a PAMPs, DAMPs or other TLR ligand.

As used herein, the terms "Toll-like receptor" and, equivalently, "TLR" refer to any member of a family of at least thirteen highly conserved mammalian pattern recognition receptor proteins (TLR1-TLR13) which recognize PAMPs, DAMPs and act as key signaling elements in innate immunity. TLR polypeptides share a characteristic structure that includes an extracellular (extracytoplasmic) domain that has leucine-rich repeats, a transmembrane domain, and an intracellular (cytoplasmic) domain that is involved in TLR signaling. TLRs include but are not limited to human TLRs.

Nucleic acid and amino acid sequences for all ten currently known human TLRs are available from public databases such as GenBank. Similarly, nucleic acid and amino acid sequences for various TLRs from numerous non-human species are also available from public databases including GenBank. For example, nucleic acid and amino acid sequences for human TLR9 (hTLR9) can be found as GenBank accession numbers AF245704 (coding region spanning nucleotides 145-3243) and AAF78037, respectively. Nucleic acid and amino acid sequences for murine TLR9 (mTLR9) can be found as GenBank accession numbers AF348140 (coding region spanning nucleotides 40-3138) and AAK29625, respectively. The deduced human TLR9 protein contains 1,032 amino acids and shares an overall amino acid identity of 75.5% with mouse TLR9. Like other TLR proteins, human TLR9 contains extracellular leucine-rich repeats (LRRs) and a cytoplasmic Toll/interleukin-1R (TIR) domain. It also has a signal peptide (residues 1-25) and a transmembrane domain (residues 819-836).

Nucleic acid and amino acid sequences for human TLR8 (hTLR8) can be found as GenBank accession numbers AF245703 (coding region spanning nucleotides 49-3174) and AAF78036, respectively. Nucleic acid and amino acid sequences for murine TLR8 (mTLR8) can be found as GenBank accession numbers AY035890 (coding region spanning nucleotides 59-3157) and AAK62677, respectively.

Nucleic acid and amino acid sequences for human TLR7 (hTLR7) can be found as GenBank accession numbers AF240467 (coding region spanning nucleotides 135-3285) and AAF60188, respectively. Nucleic acid and amino acid sequences for murine TLR7 (mTLR7) can be found as GenBank accession numbers AY035889 (coding region spanning nucleotides 49-3201) and AAK62676, respectively.

Nucleic acid and amino acid sequences for human TLR3 (hTLR3) can be found as GenBank accession numbers NM003265 (coding region spanning nucleotides 102-2816) and NP003256, respectively. Nucleic acid and amino acid sequences for murine TLR3 (hTLR3) can be found as GenBank accession numbers AF355152 (coding region spanning nucleotides 44-2761) and AAK26117, respectively.

While hTLR1 is ubiquitously expressed, hTLR2, hTLR4 and hTLR5 are present in monocytes, polymorphonuclear phagocytes, and dendritic cells. Muzio et al. (2000), J Leukoc Biol 67: 450-6. Recent publications reported that hTLR1, hTLR6, hTLR7, hTLR9 and hTLR10 are present in human B cells. Human TLR7 and hTLR9 are present in plasmacytoid dendritic cells (pDCs), while myeloid dendritic cells express hTLR7 and hTLR8 but not hTLR9. Human TLR8, however, appears not to be expressed in pDCs.

As members of the pro-inflammatory interleukin-1 receptor (IL-1R) family, TLRs share homologies in their cytoplasmic domains called Toll/IL-1R homology (TIR) domains. *See* PCT published applications PCT/US98/08979 and PCT/US01/16766. Intracellular signaling mechanisms mediated by TLRs appear generally similar, with MyD88 and tumor necrosis factor receptor-associated factor 6 (TRAF6) believed to have critical roles. Wesche et al. (1997), Immunity 7: 837-47; Medzhitov et al. (1998), Mol Cell 2 : 253-8; Adachi et al. (1998), Immunity 9: 143-50; Kawai et al. (1999), Immunity 11:115-22); Cao et al. (1996), Nature 383: 443-6; Lomaga et al. (1999), Genes Dev 13: 1015-24. Signal transduction between MyD88 and TRAF6 is known to involve members of the serine-threonine kinase IL-1 receptor-associated kinase (IRAK) family, including at least IRAK-1 and IRAK-2. Muzio et al. (1997), Science 278: 1612-5.

Briefly, MyD88 is believed to act as an adapter molecule between the TIR domain of a TLR or IL-1R and IRAK (which includes at least any one of IRAK-1, IRAK-2, IRAK-4, and IRAK-M). MyD88 includes a C-terminal Toll homology domain and an N-terminal death domain. The Toll homology domain of MyD88 binds the TIR domain of TLR or IL-1R, and the death domain of MyD88 binds the death domain of the serine kinase IRAK IRAK interacts with TRAF6, which acts as an entryway into at least two pathways, one leading to activation of the transcription factor NF-KB and another leading to activation of Jun and Fos, members of the activator protein-1 (AP-1) transcription factor family. Activation of NF-KB involves the activation of TAK-1, a member of the MAP 3 kinase (MAPK) family, and IKB kinases. The IoB kinases phosphorylate IKB, leading to its degradation and the translocation of NF-KB to the nucleus. Activation of Jun and Fos is believed to involve MAP kinase kinases (MAPKKs) and MAP kinases ERK, p38, and JNK/SAPK. Both NF-KB and AP-1 are involved in controlling the transcription of a number of key immune response genes, including genes for various cytokines and costimulatory molecules. See Aderem et al. (2000), Nature 406: 782-7; Hacker et al. (1999), EMBO J 18:6973-82.

As used herein, the terms "TLR ligand" and, equivalently, "ligand for a TLR" and "TLR signaling agonist", refer to a molecule, other than a small molecule according to Formula I described herein that interacts, directly or indirectly, with a TLR through a TLR domain other than a TIR domain and induces TLR-mediated signaling. In one embodiment a TLR ligand is a natural ligand, *i.e.,* a TLR ligand that is found in nature. In one embodiment a TLR ligand refers to a molecule other than a natural ligand of a TLR, *e.g.,* a molecule prepared by human activity. In one embodiment the TLR is TLR9 and the TLR signal agonist is a CpG nucleic acid.

Ligands for many but not all of the TLRs have been described. For instance, it has been reported that TLR2 signals in response to peptidoglycan and lipopeptides. Yoshimura et al. (1999), JImmunol 163:1-5 ; Brightbill et al. (1999), Science 285: 732-6; Aliprantis et al. (1999), Science 285: 736-9; Takeuchi et al. (1999), Immunity 11: 443-51; Underhill et al. (1999), Nature 401: 811-5. TLR4 has been reported to signal in response to lipopolysaccharide (LPS). See Hoshino et al. (1999), Immunol. 162: 3749-52; Poltorak et al. (1998), Science 282: 2085-8; Medzhitov et al. (1997), Nature 388: 394-7. Bacterial flagellin has been reported to be a natural ligand for TLR5. See Hayashi et al. (2001), Nature 410:1099-1103. TLR6, in conjunction with TLR2, has been reported to signal in response to proteoglycan. See Ozinsky et al. (2000), Proc Natl Acad Sci USA 97 : 13766-71; Takeuchi et al. (2001), Int Immunol 13 : 933-40.

Recently it was reported that TLR9 is a receptor for CpG DNA. Hemmi H et al. (2000) Nature 408: 740-5; Bauer S et al. (2001) Proc Natl Acad Sci USA 98: 9237-42. CpG DNA, which includes bacterial DNA and synthetic DNA with CG dinucleotides in which cytosin is unmethylated, is described in greater detail elsewhere herein. Marshak-Rothstein and colleagues also recently reported their finding that TLR9 signaling can occur in certain autoimmune diseases in response to immune complexes containing IgG and chromatin. Leadbetter EA et al. (2002) Nature 416: 595-8. Thus, in a broader sense it appears that TLR9 can signal in response to self or non-self nucleic acid, either DNA or RNA, when the nucleic acid is presented in a suitable context, e. g., as part of an immune complex.

Recently it was reported that certain imidazoquinoline compounds having antiviral activity are ligands of TLR7 and TLR8. Hemmi H et al. (2002) Nat Immunol 3 : 196-200; Jurk M et al. (2002) Nat Immunol 3: 499. Imidazoquinolines are potent synthetic activators of immune cells with antiviral and antitumor properties. Using macrophages from wildtype and MyD88-deficient mice, Hemmi et al. recently reported that two imidazoquinolines, imiquimod and resiquimod (R848), induce tumor necrosis factor (TNF) and interleukin-12 (IL-12) and activate NF-KB only in wildtype cells, consistent with activation through a TLR. Hemmi H et al. (2002) Nat Immunol 3 : 196-200. Macrophages from mice deficient in TLR7 but not other TLRs produced no detectable cytokines in response to these imidazoquinolines. In addition, the imidazoquinolines induced dose-dependent proliferation of splenic B cells and the activation of intracellular signaling cascades in cells from wildtype but not TLR7-/- mice. Luciferase analysis established that expression of human TLR7, but not TLR2 or TLR4, in human embryonic kidney cells results in NF-KB activation in response to resiquimod. The findings of Hemmi et al. thus suggested that these imidazoquinoline compounds are non-natural ligands of TLR7 that can induce signaling through TLR7. Recently it was reported that R848 is also a ligand for human TLR8. See Jurk M et al. (2002) Nat Immunol 3:499. Nat Immunol 3 : 499. It has also been reported that ssRNA is natural ligand and that aberrant stimulation of TLR7 and or TLR8 by RNA:complexes is involved in autoimmunity.

It was recently reported that ligands of TLR3 include poly (I: C) and double-stranded RNA (dsRNA). For purposes of this invention, poly (I: C) and double-stranded RNA (dsRNA) are classified as oligonucleotide molecules. By stimulating kidney cells expressing one of a range of TLRs with poly (I: C), Alexopoulou et al. reported that only cells expressing TLR3 respond by activating NF-aB. See Alexopoulou L et al. (2001) Nature 413: 732-8.

Alexopoulou et al. also reported that wildtype cells stimulated with poly (I: C) activate NF-KB and produce inflammatory cytokines IL-6, IL-12, and TNF-a, whereas the corresponding responses of TLR3-/-cells were significantly impaired. In contrast, TLR3-/-cells responded equivalently to wildtype cells in response to lipopolysaccharide, peptidoglycan, and CpG dinucleotides. Analysis of MyD88-/-cells indicated that this adaptor protein is involved in dsRNA-induced production of cytokines and proliferative responses, although activation of NF-KB and MAP kinases are not affected, indicating distinct pathways for these cellular responses. Alexopoulou et al. proposed that TLR3 may have a role in host defense against viruses.

As used herein, a "cell expressing a TLR" refers to any cell which expresses, either naturally or artificially, a functional TLR. A functional TLR is a full-length TLR protein or a fragment thereof capable of inducing a signal in response to interaction with its ligand.

Generally, the functional TLR will include at least a TLR ligand-binding fragment of the extracellular domain of the full-length TLR and at least a fragment of a TIR domain capable of interacting with another Toll homology domain-containing polypeptide, e. g., MyD88. In various embodiments the functional TLR is a full-length TLR selected from TLR1, TLR2, TLR3, TLR4, TLR5, TLR6, TLR7, TLR8, TLR9, and TLR10.

### Compounds

Novel pteridine compounds are described. Applicants have surprisingly discovered novel N², N⁴, N⁷, 6-tetrasubstituted pteridine-2,4,7-triamines and 2, 4, 6, 7-tetrasubstituted pteridines as immune system modulators. It is unexpected that the pteridine compounds as disclosed herein are useful in methods for inhibiting an immune response, both in vitro and in vivo, including methods for treating immune complex associated diseases and autoimmune disorders. In another aspect, the invention provides novel pteridine compositions. As described further below, these compositions and other pteridine compositions have been discovered to be useful in methods for inhibiting an immune response, both in vitro and in vivo, including methods for treating immune complex associated diseases and autoimmune disorders. It is also believed that the novel pteridine compositions as described herein can be used for prevention and treatment of malaria, as well as for treatment of other diseases.

In one aspect, a compound of Formula I or a pharmaceutically acceptable salt thereof is described, 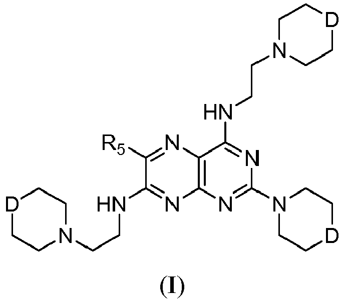 wherein
each occurrence of D is independently -O- or -N(Me)-; and
R₅ is H, F, or Cl.

In some embodiments, the compound has a structure selected from the group consisting of 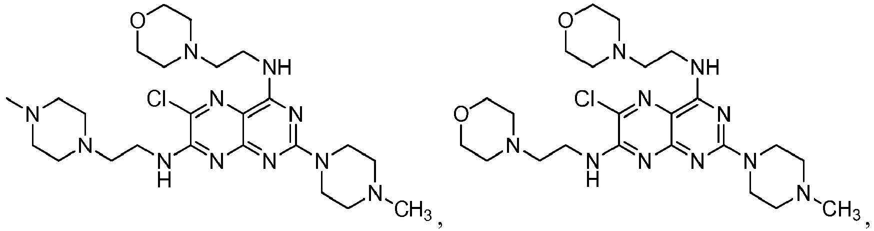 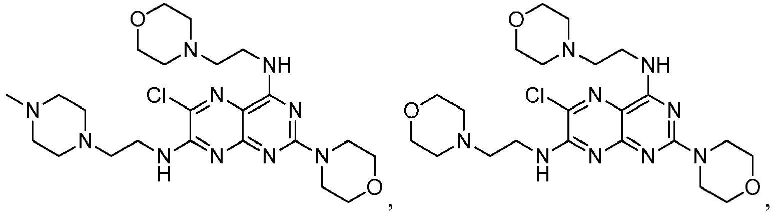 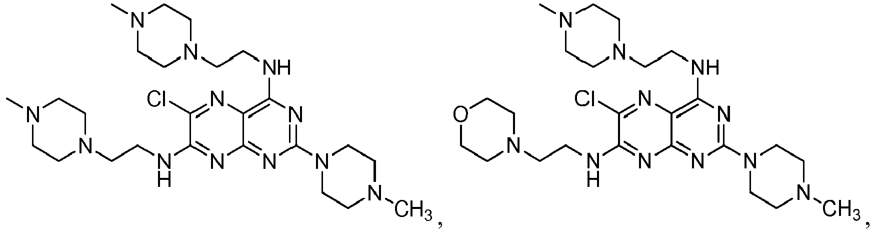 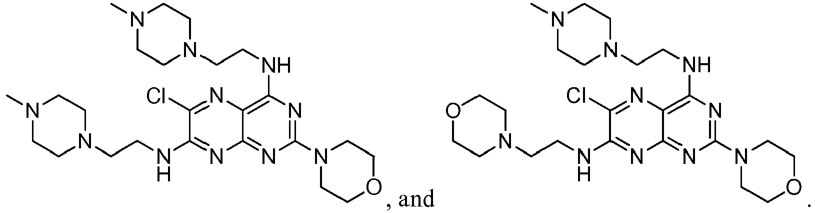 In other embodiments, the compound has the structure selected from the group consisting of

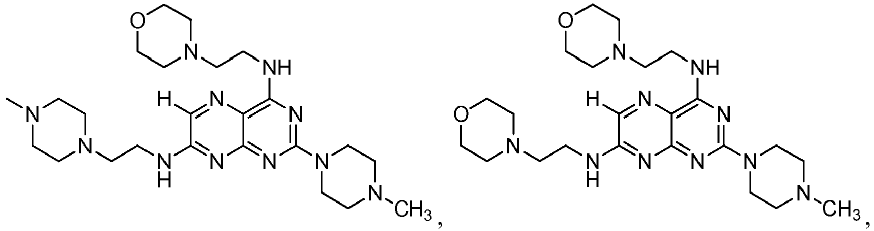

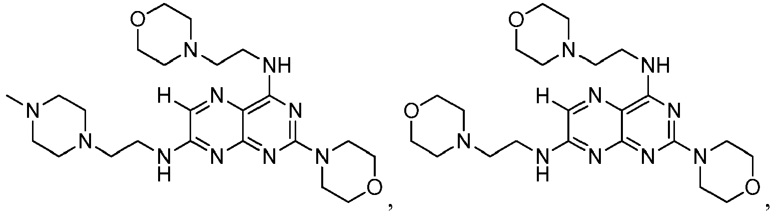

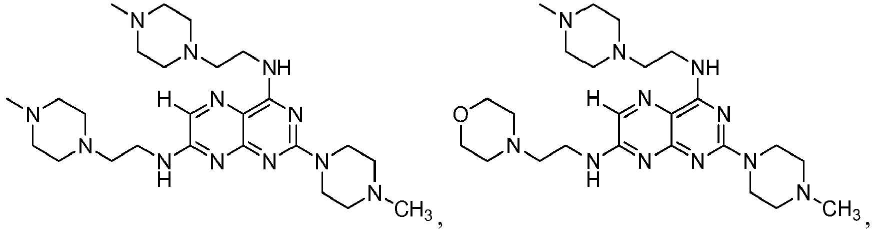

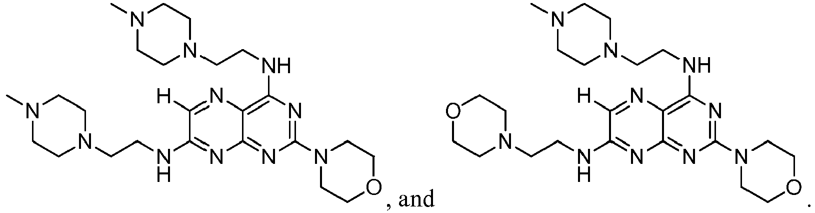

In certain specific embodiments, the compound has the structure of 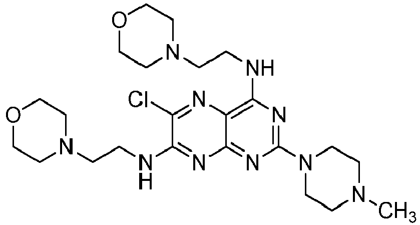 (6-chloro-2-(4-methylpiperazin-1-yl)-N⁴,N⁷-bis(2-morpholinoethyl)pteridine-4,7-diamine; "JB6121"). In certain specific embodiments, the compound has the structure of 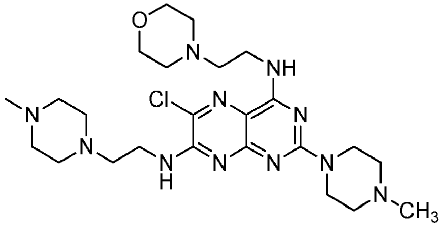 (6-chloro-2-(4-methylpiperazin-1-yl)-N⁷-(2-(4-methylpiperazin-1-yl)ethyl)-N⁴-(2-morpholinoethyl)pteridine-4,7-diamine).

For a small molecule compound to be considered as a proper drug candidate, a well-balanced overall pharmacological profile of the compound is desirable. For instance, in addition to potent in vitro and in vivo activities against a disease target, the drug candidate needs to exhibit acceptable safety profile, *e.g.,* low toxicity, and good bioavailability. Applicants have surprisingly and unexpectedly found that compounds having the structure of Formula (I), *e.g.,* JB6121, exhibited overall desirable PK/PD profiles, including potent antagonist activities agsint TLRs in vitro and in vivo, low or no toxicities in vivo, desirable cellular absorption, desirable in vivo stabilities, and/or desirable bioavalabilities and systemic exposure after administration of the compound in vivo. For instance, Applicants unexpectedly found that 6-chloro-2-(4-methylpiperazin-1-yl)-N⁴,N⁷-bis(2-morpholinoethyl)pteridine-4,7-diamine exhibited desirable in vivo and in vitro TLR antagonist activities, acceptable in vivo bioavailability (>20%), no toxicity in an AMES test in rats, low cardiovascular toxicities, low drug-drug interaction activities, and low receptor cross-activities.

More specifically, it has been unexpected found that compounds having the structure of Formula (I), *e.g.,* JB6121, have low or virtually no cardiovascular toxicities. A number of drugs have been withdrawn from late stage clinical trials due to these cardiotoxic effects, therefore it is important to identify and avoid compounds with cardiotoxic effects early in drug discovery. The cardiovascular toxicity of a compound can be measured using a standard human ether-a-go-go related gene (hERG) assay. The human ether-a-go-go related gene (hERG) encodes the inward rectifying voltage gated potassium channel in the heart (I_{Kr}), which is involved in cardiac repolarization. Inhibition of the hERG current causes QT interval prolongation resulting in potentially fatal ventricular tachyarrhythmia called *Torsade de Pointes.* A compound having an IC₅₀ of more than 10 µM in the hERG assay may be considered as free from any cardiovascular toxicity. In some embodiments, the compound of Formula (I) has an IC₅₀ of more than 10, 15, 20, 25, or 30 µM in the standard patch clamp hERG assay. For instance, 6-chloro-2-(4-methylpiperazin-1-yl)-N⁴,N⁷-bis(2-morpholinoethyl)pteridine-4,7-diamine exhibited no measurable hERG inhibition at or more than 30 µM. In comparison, JB6059, JB6116 and JB6131 displayed IC₅₀s equal to 6.2 µM, 13.1 µM and 8.1 µM, respectively, in the hERG assay. JB6059, JB6116 and JB6131 are analogs closely related to the compounds of Formula (I) but do not fall in the genus of Formula (I) (see Scheme 1 below). This lack of cardiovascular toxicity by the compound of Formula (I) is surprising and unexpected, especially considering the closely related analogs JB6059, JB6116 and JB6131 all possess certain degree of cardiovascular toxicities.

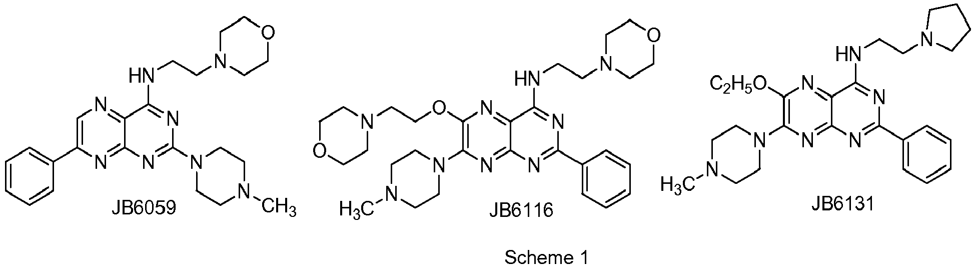

Additionally, it has been unexpectedly found that compounds having the structure of Formula (I), *e.g.,* JB6121, have low or virtually no hepatocyte toxicity. The hepatocyte toxicity may be measured as the percentage of hepatocyte viability after the hepatocyte is exposed to the compound. In some embodiments, the compounds of Formula (I), *e.g.,* JB6121, result in more than 75%, 80%, 85%, 90%, 95%, 97%, 98%, or 99% hepatocyte viability, or result in a hepatocyte viability in the range bounded by any two values disclosed herein, after the hepatocyte is exposed to the compound at 100 µM for 24h.

In another aspect, a compound of Formula Ia or a pharmaceutically acceptable salt thereof is described, 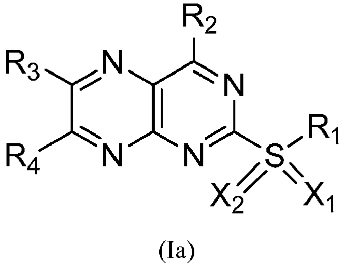 wherein
R₁ is hydrogen, alkyl, alkenyl, cycloalkyl, alkylcycloalkyl, aryl, alkylaryl, heterocycle, or alkylheterocycle;
X₁ and X₂ are each independently absent or O;
R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
each occurrence of R_{b} and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
provided that when R₂ is ORₐ, SRₐ, NR_{b}R_{c}, or NRₐ(CH₂)ₚNR_{b}R_{c}, at least one of X₁ and X₂ is O. In some embodiments, R₁ is alkyl, optionally substituted aryl, or optionally substituted heteroaryl. In any of the embodiments described herein, X₁ and X₂ may both be O.

In certain embodiments, R₁ is hydrogen, alkyl, alkenyl, cycloalkyl, alkylcycloalkyl, aryl, alkylaryl, heterocycle, or alkylheterocycle. Non-limiting examples of alkyl groups include Me, Et, Pr, i-Pr, n-Bu, i-Bu, t-Bu or sec-Bu.

In certain embodiments, X₁ and X₂ are each independently absent or O, with the proviso that when R₂ is ORₐ, SRₐ, NR_{b}R_{c}, or NRₐ(CH₂)ₚNR_{b}R_{c}, at least one of X₁ and X₂ is O.

In certain embodiments, R₂ is selected from the group consisting of halogen, ORₐ, SRₐ, OS(=O)₂Rₐ, OC(=O)Rₐ, NR_{b}R_{c}, and NRₐ(CH₂)ₚNR_{b}R_{c}, wherein p is 2-4. In certain embodiments, R₃ and R₄ are each selected from the group consisting of hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, and NRₐ(CH₂)ₚNR_{b}R_{c}.

In any of the embodiments described herein, R₂ may be selected from the group consisting of halogen, OS(=O)₂Rₐ, OC(=O)Rₐ, NR_{b}R_{c}, and NRₐ(CH₂)ₚNR_{b}R_{c}. In some embodiments, R₂ is Cl or Br. In some embodiments, R₂ is OS(=O)₂Rₐ, or OC(=O)Rₐ. In some embodiments, R₂ is NR_{b}R_{c} or NRₐ(CH₂)ₚNR_{b}R_{c}.

In any of the embodiments described herein, R₄ may be NR_{b}R_{c} or NRₐ(CH₂)ₚNR_{b}R_{c}. R₂ and R₄ may be the same or different. In some embodiments, R₂ and R₄ are each independently selected from the group consisting of: 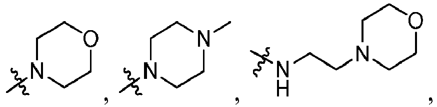 and

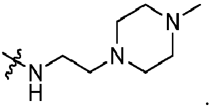

In another aspect, a compound is described, selected from the group consisting of: 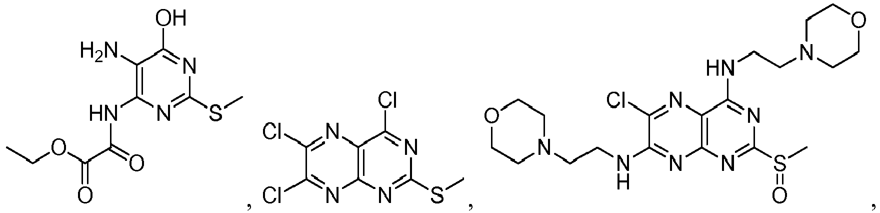 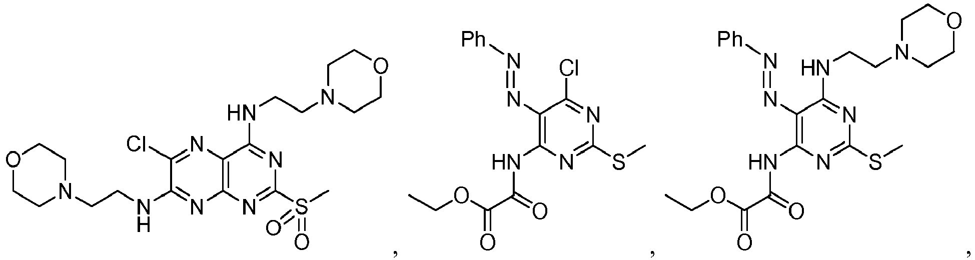 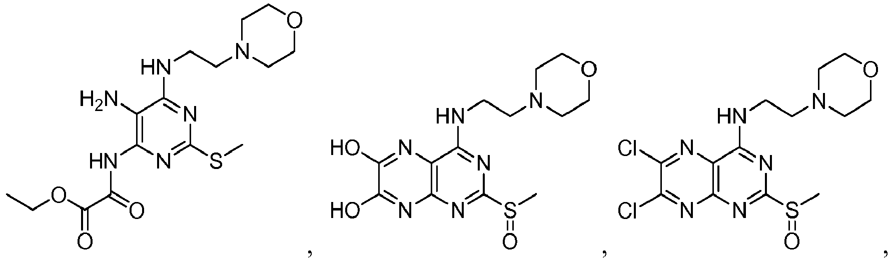 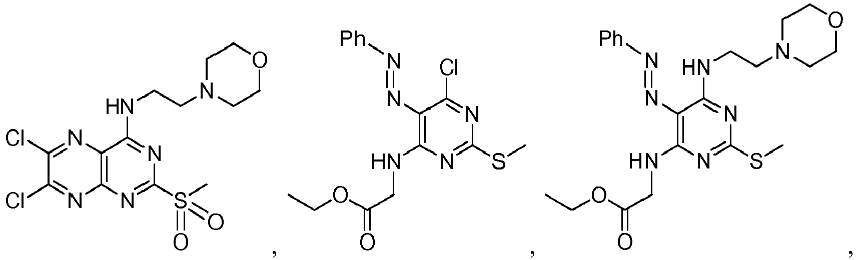 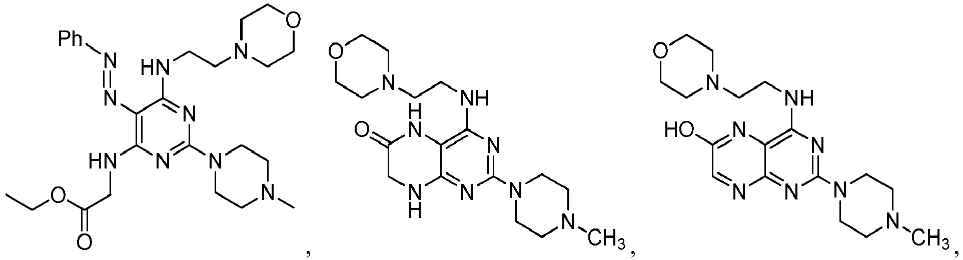 and

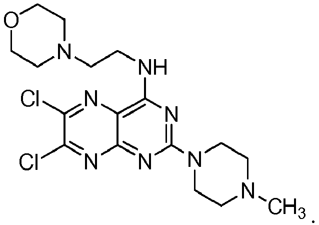

In yet another aspect, the present invention provides a compound of Formula (Ia) selected from Examples 1 through 74 as described in Table 1. The enumerated compounds in Table 1 are representative and non-limiting pteridine compounds of the invention.

**Table 1. Selected compound of Formula (Ia), wherein X₁ and X₂ are each independently O or absent.**

| Example No. | R₁ | R₂ | R₃ | R₄ |
|---|---|---|---|---|
| 1 | Me | Cl | H | Cl |
| 2 | Me | OH | Cl | H |
| 3 | Me | 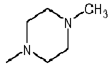 | H | Me |
| 4 | Ph | Cl | Me | H |
| 5 | Me | 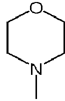 | Me | Me |
| 6 | Me | 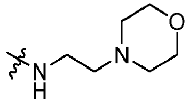 | 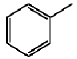 | Me |
| 7 | Ph | 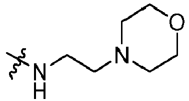 | H | 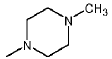 |
| 8 | Me | 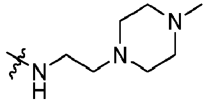 | Me | H |
| 9 | Ph | 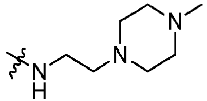 | Br | 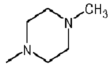 |
| 10 | Ph | 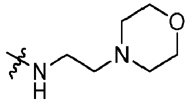 | Cl | 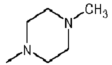 |
| 11 | Ph | 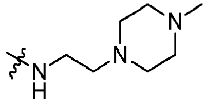 | H | SCH₃ |
| 12 | Ph | 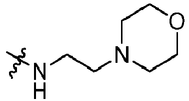 | H | SO₂CH₃ |
| 13 | Me | 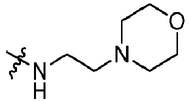 | Cl | OCH₃ |
| 14 | Ph | 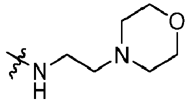 | H | OH |
| 15 | Me | 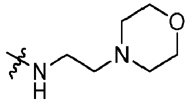 | H | H |
| 16 | NH₂ | 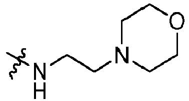 | Me | H |
| 17 | NH₂ | 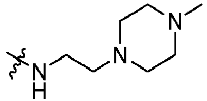 | H | Me |
| 18 | NH₂ | 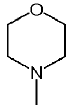 | H | H |
| 19 | NH₂ | 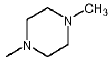 | H | Me |
| 20 | NH₂ | OH | H | H |
| 21 | NH₂ | SH | H | H |
| 22 | NH₂ | Cl | Me | Me |
| 23 | NH₂ | F | H | Me |
| 24 | NH₂ | Br | H | 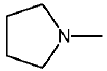 |

Additional pteridine analogs are described in the various aspects and embodiments disclosed in PCT Publication No. WO 2012/167046, the content of which is expressly incorporated by reference.

In yet another aspect, the present invention provides a pharmaceutical composition comprising at least one compound of Formulae I and Ia as described herein and a pharmaceutically-acceptable carrier or diluent.

In yet another aspect, the present invention provides a method for treating an autoimmune disease in a mammalian species in need thereof, the method comprising administering to the mammalian species a therapeutically effective amount of at least one compound of Formula I, 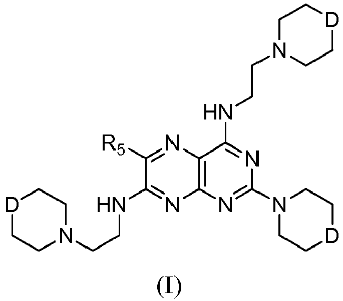 wherein
each occurrence of D is independently -O- or -N(Me)-; and
R₅ is H, F, or Cl.

In some specific embodiments, the compound has the structure of

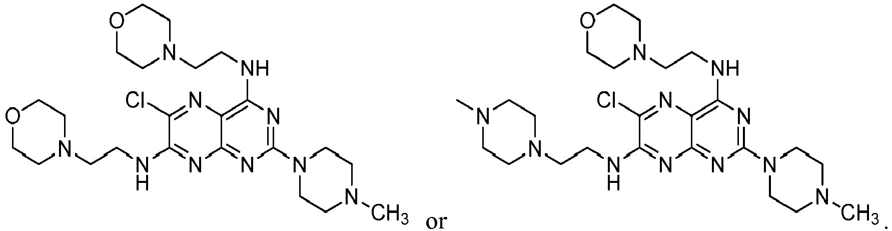

In yet another aspect, the present invention provides a method for treating an autoimmune disease in a mammalian species in need thereof, the method comprising administering to the mammalian species a therapeutically effective amount of at least one compound of Formula Ia, 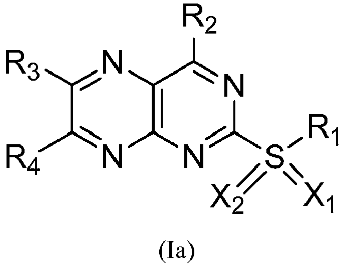 wherein
R₁ is hydrogen, alkyl, alkenyl, cycloalkyl, alkylcycloalkyl, aryl, alkylaryl, heterocycle, or alkylheterocycle;
X₁ and X₂ are each independently absent or O;
R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
provided that when R₂ is ORₐ, SRₐ, NR_{b}R_{c}, or NRₐ(CH₂)ₚNR_{b}R_{c}, at least one of X₁ and X₂ is O.

In certain embodiments, the pteridine composition is in the form of a hydrate or pharmaceutically acceptable salt. The pteridine composition can be administered to the subject by any suitable route of administration, including, without limitation, oral and parenteral. Parenteral routes of administration are as described above with respect to substituted 4-primary amino pteridines.

In certain embodiments, the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.

In some embodiments, the autoimmune disease is selected from the group consisting of systemic lupus erythematosus, rheumatoid arthritis, psoriasis, inflammatory bowel disease, Sjogren's syndrome, polymyositis, vasculitis, Wegener's granulomatosis, sarcoidosis, ankylosing spondylitis, Reiter's syndrome, psoriatic arthritis, and Behyet's syndrome. In one particular embodiment, the autoimmune disease is systemic lupus erythematosus. In another particular embodiment, the autoimmune disease is rheumatoid arthritis. In one particular embodiment the autoimmune disease is psoriasis. In yet another particular embodiment, the autoimmune disease is Sjogren's syndrome. In one embodiment, the subject is a human. In one embodiment the autoimmune disorder is an immune complex associated disease, as described above.

In yet another aspect, the present invention provides a method of inhibiting TLR-mediated immunostimulation in a mammalian species in need thereof, comprising administering to the mammalian species a therapeutically effective amount of at least one compound of Formula I, 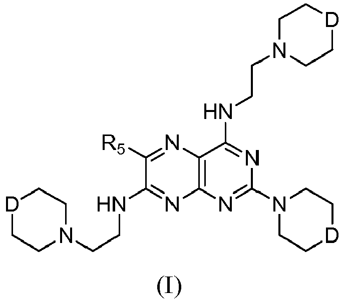 wherein
each occurrence of D is independently -O- or -N(Me)-; and
R₅ is H, F, or Cl.

In some specific embodiments, the compound has the structure of

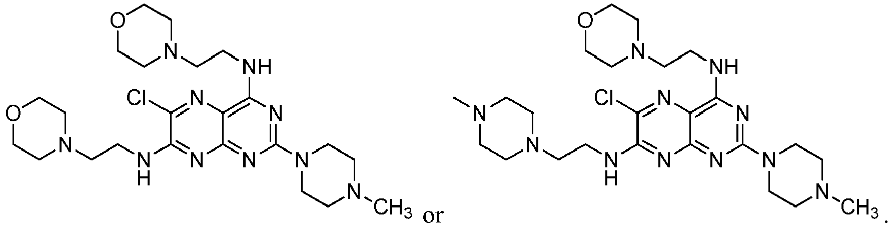

In yet another aspect, the present invention provides a method of inhibiting TLR-mediated immunostimulation in a mammalian species in need thereof, comprising administering to the mammalian species a therapeutically effective amount of at least one compound of Formula Ia, 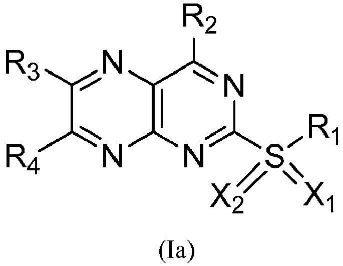 wherein
R₁ is hydrogen, alkyl, alkenyl, cycloalkyl, alkylcycloalkyl, aryl, alkylaryl, heterocycle, or alkylheterocycle;
X₁ and X₂ are each independently absent or O;
R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
provided that when R₂ is ORₐ, SRₐ, NR_{b}R_{c}, or NRₐ(CH₂)ₚNR_{b}R_{c}, at least one of X₁ and X₂ is O.

In some embodiments, the method of affecting TLR-mediated immunostimulation in a subject comprises administering to a subject having or at risk of developing TLR-mediated immunostimulation an effective amount of a compound of Formulae I-Ia, as provided herein, to inhibit TLR-mediated immunostimulation in the subject.

In yet another aspect, the present invention provides a method of inhibiting TLR-mediated immunostimulatory signaling, comprising contacting a cell expressing a TLR with an effective amount of at least one compound of Formula I, 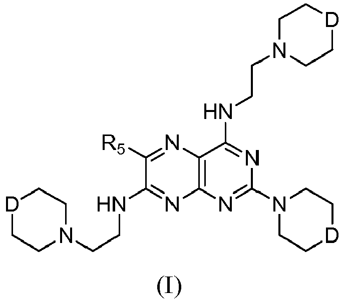 wherein
each occurrence of D is independently -O- or -N(Me)-; and
R₅ is H, F, or Cl.

In some specific embodiments, the compound has the structure of

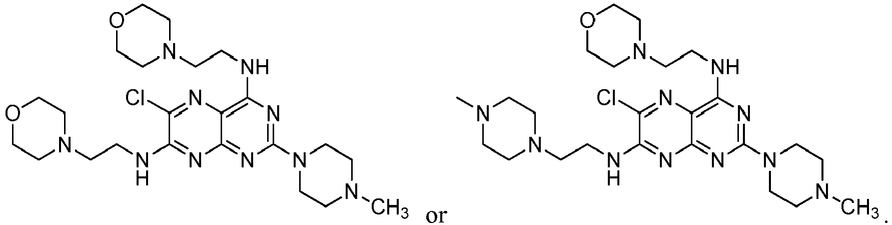

In yet another aspect, the present invention provides a method of inhibiting TLR-mediated immunostimulatory signaling, comprising contacting a cell expressing a TLR with an effective amount of at least one compound of Formula Ia, 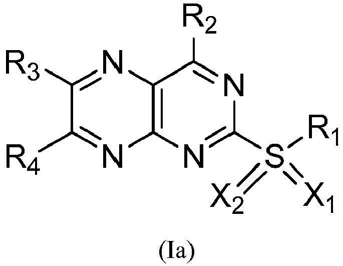 wherein
R₁ is hydrogen, alkyl, alkenyl, cycloalkyl, alkylcycloalkyl, aryl, alkylaryl, heterocycle, or alkylheterocycle;
X₁ and X₂ are each independently absent or O;
R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
provided that when R₂ is ORₐ, SRₐ, NR_{b}R_{c}, or NRₐ(CH₂)ₚNR_{b}R_{c}, at least one of X₁ and X₂ is O.

In some embodiments, the method of inhibiting TLR-mediated immunostimulatory signaling comprises contacting a cell expressing a TLR with an effective amount of a compound of Formulae I-Ia, as provided above, to inhibit TLR-mediated immunostimulatory signaling in response to a ligand for the TLR.

In some embodiments, the method of inhibiting TLR-mediated immunostimulatory signaling comprises contacting an immune cell expressing a functional TLR with
(a) an effective amount of a TLR signal agonist to stimulate signaling by the TLR in absence of a pteridine composition, and
(b) an effective amount of a pteridine composition having structural Formula I or Ia as described herein, to inhibit signaling by the TLR in response to the TLR signal agonist compared with the signaling by the TLR in response to the TLR signal agonist in absence of the pteridine composition.

In some specific embodiments, the pteridine composition used for inhibiting TLR-mediated immunostimulatory signaling has a structure of Formula I. In some specific embodiments, the pteridine composition is in the form of a hydrate or pharmaceutically acceptable salt. In some specific embodiments, the method for inhibiting TLR-mediated immunostimulatory signaling is performed in vitro or in vivo.

In some embodiments, the TLR is TLR9 and the TLR signal agonist is a TLR9 signal agonist. In these embodiments, the method is a method of inhibiting intracellular signaling by TLR9 in response to a TLR9 signal agonist. The TLR signal agonist in one embodiment is CpG DNA, which can be an oligodeoxynucleotide (ODN). In some embodiments, CpG ODN is ODN 2006. In other embodiments, CpG ODN belongs to any class of CpG ODN, including A-class (e.g., ODN 2216), B-class (e.g., ODN 2006), or C-class (e.g., ODN 2395).

In some embodiments, the TLR signal agonist is an immune complex that includes a nucleic acid.

In some embodiments, the methods as described herein are useful for altering TLR-mediated signaling. The methods are used to alter TLR-mediated signaling in response to a suitable TLR ligand or TLR signaling agonist. For example, the methods can be used to treat any variety of conditions involving autoimmunity, inflammation, allergy, asthma, graft rejection, graft-versus host disease (GvHD), infection, sepsis, cancer, and immunodeficiency. Generally, methods useful in the treatment of conditions involving autoimmunity, inflammation, allergy, asthma, graft rejection, and GvHD will employ small molecules that inhibit TLR-mediated signaling in response to a suitable TLR ligand or TLR signaling agonist. Generally, methods useful in the treatment of conditions involving infection, cancer, and immunodeficiency will employ small molecules that augment TLR-mediated signaling in response to a suitable TLR ligand. In some embodiments, the methods are used to inhibit or promote TLR-mediated signaling in response to a TLR ligand or TLR signaling agonist. In some embodiments, the methods are used to inhibit TLR-mediated immunostimulatory signaling in response to a TLR ligand or TLR signaling agonist. In some embodiments, the methods are used to inhibit or promote TLR-mediated immunostimulation in a subject. In some embodiments, the methods are used to inhibit TLR-mediated immunostimulation in a subject. In some embodiments, the methods are used to inhibit an immunostimulatory nucleic acid-associated response in a subject.

In some embodiments, the method useful for altering TLR-mediated signaling uses small molecule compositions of compounds of Formulae I and Ia. The compositions of the invention are used to alter TLR-mediated signaling in response to a suitable TLR ligand or TLR signaling agonist. For example, the small molecules can be used in methods to treat any of a variety of conditions involving autoimmunity, inflammation, allergy, asthma, graft rejection, GvHD, infection, sepsis, cancer, and immunodeficiency. Generally, methods useful in the treatment of conditions involving autoimmunity, inflammation, allergy, asthma, graft rejection, and GvHD will employ small molecules that inhibit TLR-mediated signaling in response to a suitable TLR ligand or TLR signaling agonist. Generally, methods useful in the treatment of conditions involving infection, cancer, and immunodeficiency will employ small molecules that augment TLR-mediated signaling in response to a suitable TLR ligand. In some instances the molecules can be used in a method to inhibit or promote TLR-mediated signaling in response to a TLR ligand or TLR signaling agonist. In some instances the small molecules can be used in a method to inhibit TLR-mediated immunostimulatory signaling in response to a TLR ligand or TLR signaling agonist. In some embodiments, the small molecules are used in a method to inhibit or promote TLR-mediated immunostimulation in a subject. In some embodiments, the small molecules are used in a method to inhibit TLR-mediated immunostimulation in a subject. In some embodiments, the small molecules are used to inhibit an immunostimulatory nucleic acid-associated response in a subject.

Furthermore, the methods as described herein can be combined with administration of additional agents to achieve synergistic effect on TLR-mediated immunostimulation. More specifically, whereas the agents described herein have been discovered to affect TLRs directly and thus directly affect TLR-bearing cells, e.g., antigen-presenting cells (APCs), such agents can be used in conjunction with additional agents which affect non-APC immune cells, e.g., T lymphocytes (T cells). Such an approach effectively introduces an immunomodulatory intervention at two levels: innate immunity and acquired immunity. Since innate immunity is believed to initiate and support acquired immunity, the combination intervention is synergistic.

In yet another aspect, a method of inhibiting an immunostimulatory nucleic acid-associated response in a subject is provided. The method comprises administering to a subject in need of such treatment an effective amount of a compound of Formulae I and Ia, as provided above, to inhibit an immunostimulatory nucleic acid-associated response in the subject.

In some embodiments, the subject being treated with the pteridine compounds as described herein has symptoms indicating an immune system disease. In other embodiments, the subject being treated with the pteridine compounds as described herein is free of any symptoms indicating an immune system disease.

In some embodiments, the TLR is TLR9. In some specific embodiments, the ligand for the TLR is an immunostimulatory nucleic acid. In other specific embodiments, the immunostimulatory nucleic acid is a CpG nucleic acid. In still other specific embodiments, the immunostimulatory nucleic acid a DNA containing immune complex.

In some embodiments, the TLR is TLR8. In some specific embodiments, the ligand for the TLR is a natural ligand for TLR8. In other specific embodiments, the ligand for the TLR is RNA. In still other specific embodiments, the ligand for the TLR is an immunostimulatory nucleic acid. In still other specific embodiments, the immunostimulatory nucleic acid is an RNA containing immune complex. In still other specific embodiments, the ligand for the TLR is an immunostimulatory imidazoquinoline. In still other specific embodiments, the ligand for the TLR is resiquimod (R848).

In some embodiments, the TLR is TLR7. In some specific embodiments, the ligand for the TLR is a natural ligand for TLR7. In other specific embodiments, the ligand for the TLR is an immunostimulatory nucleic acid. In one embodiment the ligand for the TLR is a RNA. In still other specific embodiments, the immunostimulatory nucleic acid is an RNA containing immune complex. In still other specific embodiments, the ligand for the TLR is an immunostimulatory imidazoquinoline. In still other specific embodiments, the ligand for the TLR is R848.

In some embodiments, the TLR is TLR3. In some specific embodiments, the ligand for the TLR is a double stranded RNA. In other specific embodiments, the ligand for the TLR is the immune complex as described herein. In still other specific embodiments, the ligand for the TLR is poly(I:C). In still other specific embodiments, the TLR is TLR9 and the TLR signal agonist is a TLR9 signal agonist. In still other specific embodiments, the TLR signal agonist is CpG DNA, which can be an oligodeoxynucleotide (ODN).

In some embodiments, the TLR signal agonist is an immune complex comprising a nucleic acid.

In yet another aspect, a method for inhibiting an immune response to an antigenic substance is provided. The method comprises contacting an immune cell expressing a functional Toll-like receptor with:
(a) an effective amount of an antigenic substance to stimulate an immune response to the antigenic substance in the absence of a pteridine composition, and
(b) an effective amount of a pteridine composition having structural Formulae I and Ia, as defined above, to inhibit an immune response to the antigenic substance compared with the immune response to the antigenic substance in absence of the pteridine composition.

In some embodiments, the immune response is an innate immune response. In other embodiments, the immune response includes an adaptive immune response. In some specific embodiments, the pteridine composition is in the form of a hydrate or pharmaceutically acceptable salt. In some specific embodiments, the method for inhibiting an immune response to an antigenic substance is performed in vitro or in vivo.

In some embodiments, the antigenic substance is an allergen. In other embodiments, the antigenic substance is an antigen that is or is derived from a microbial agent, including a bacterium, a virus, a fungus, or a parasite. In still other embodiments, the antigenic substance is a cancer antigen.

In certain embodiments, the functional TLR is naturally expressed by a cell. Non-limiting examples of cells expressing TLR include the RPMI 8226 cell line.

In one embodiment, the cell naturally expresses functional TLR and is an isolated cell from human multiple myeloma cell line RPMI 8226 (ATCC CCL-155; American Type Culture Collection (ATCC), Manassas, VA). This cell line was established from the peripheral blood of a 61 year old man at the time of diagnosis of multiple myeloma (IgG lambda type). Matsuoka Y et al. (1967) Proc Soc Exp Biol Med 125:1246-50. RPMI 8226 was previously reported as responsive to CpG nucleic acids as evidenced by the induction of IL-6 protein and IL-12p40 mRNA. Takeshita F et al. (2000) Eur J Immunol 30:108-16; Takeshita F et al. (2000) Eur J 30:1967-76. Takeshita et al. used the cell line solely to study promoter constructs in order to identify transcription factor binding sites important for CpG nucleic acid signaling. It is now known that RPMI 8226 cells secrete a number of other chemokines and cytokines including IL-8, IL-10 and IP-10 in response to immunostimulatory nucleic acids. Because this cell line expresses TLR9, through which immunostimulatory nucleic acids such as for example CpG nucleic acids mediate their effects, it is a suitable cell line for use in the methods of the invention relating to CpG nucleic acids as reference and test compounds, as well as to other TLR9 ligands.

Similar to peripheral blood mononuclear cells (PBMCs), the RPMI 8226 cell line has been observed to upregulate its cell surface expression of markers such as CD71, CD86 and HLA-DR in response to CpG nucleic acid exposure. This has been observed by flow cytometric analysis of the cell line. Accordingly, the methods provided herein can be structured to use appropriately selected cell surface marker expression as a readout, in addition to or in place of chemokine or cytokine production or other readouts described elsewhere herein.

The RPMI 8226 cell line has also been found to respond to certain small molecules including imidazoquinoline compounds. For example, incubation of RPMI 8226 cells with the imidazoquinoline compound R848 (resiquimod) induces IL-8, IL-10, and IP-10 production. It has recently been reported that R848 mediates its immunostimulatory effects through TLR7 and TLR8. The ability of RPMI 8226 to respond to R848 suggests that the RPMI 8226 cell line also expresses TLR7, as previously reported for normal human B cells.

The RPMI cell line can be used in unmodified form or in a modified form. In one embodiment, the RPMI 8226 cell is transfected with a reporter construct. Preferably, the cell is stably transfected with the reporter construct. The reporter construct generally includes a promoter, a coding sequence and a polyadenylation signal. The coding sequence can include a reporter sequence selected from the group consisting of an enzyme (e.g., luciferase, alkaline phosphatase, beta-galactosidase, chloramphenicol acetyltransferase (CAT), secreted alkaline phosphatase, etc.), a bioluminescence marker (e.g., green fluorescent protein (GFP, U.S. Pat. No. 5,491,084), etc.), a surface-expressed molecule (e.g., CD25), a secreted molecule (e.g., IL-8, IL-12 p40, TNF-α, etc.), and other detectable protein products known to those of skill in the art. Preferably, the coding sequence encodes a protein having a level or an activity that is quantifiable.

In certain embodiments, the functional TLR is artificially expressed (including over-expressed) by a cell, for example by introduction into the cell of an expression vector bearing a coding sequence for the functional TLR wherein the coding sequence is operably linked to a gene expression sequence. As used herein, a coding sequence and the gene expression sequence are said to be operably linked when they are covalently linked in such a way as to place the expression or transcription and/or translation of the coding sequence under the influence or control of the gene expression sequence. Two DNA sequences are said to be operably linked if induction of a promoter in the 5' gene expression sequence results in the transcription of the coding sequence and if the nature of the linkage between the two DNA sequences does not (1) result in the introduction of a frame-shift mutation, (2) interfere with the ability of the promoter region to direct the transcription of the coding sequence, or (3) interfere with the ability of the corresponding RNA transcript to be translated into a protein. Thus, a gene expression sequence would be operably linked to a coding sequence if the gene expression sequence were capable of effecting transcription of that coding sequence such that the resulting transcript is translated into the desired protein or polypeptide.

In some embodiments, a coding sequence refers to a nucleic acid sequence coding for a functional TLR. In some embodiments, a coding sequence refers to a nucleic acid sequence coding for a reporter.

A cell that artificially expresses a functional TLR can be a cell that does not express the functional TLR but for the TLR expression vector. For example, human 293 fibroblasts (ATCC CRL-1573) do not express TLR3, TLR7, TLR8, or TLR9. As described in the examples below, such cells can be transiently or stably transfected with a suitable expression vector (or vectors) so as to yield cells that do express TLR3, TLR7, TLR8, TLR9, or any combination thereof. Alternatively, a cell that artificially expresses a functional TLR can be a cell that expresses the functional TLR at a significantly higher level with the TLR expression vector than it does without the TLR expression vector.

For use in the methods of the instant invention, a cell that artificially expresses a functional TLR is preferably a stably transfected cell that expresses the functional TLR. Such a cell can also be stably transfected with a suitable reporter construct.

### Assays for Effectiveness

The methods of the invention can be assessed using any of a number of possible readout systems based upon a TLR/IL-1R signal transduction pathway. In some embodiments, the readout for the method is based on the use of native genes or, alternatively, transfected or otherwise artificially introduced reporter gene constructs which are responsive to the TLR/IL-1R signal transduction pathway involving MyD88, TRAF, p38, and/or ERK. Häcker H et al. (1999) EMBO J 18:6973-82. These pathways activate kinases including κB kinase complex and c-Jun N-terminal kinases. Thus reporter genes and reporter gene constructs particularly useful for the assays include, e.g., a reporter gene operably linked to a promoter sensitive to NF-κB. Examples of such promoters include, without limitation, those for NF-κB, IL-1β, IL-6, IL-8, IL-12 p40, IP-10, CD80, CD86, and TNF-α. The reporter gene operably linked to the TLR-sensitive promoter can include, without limitation, an enzyme (e.g., luciferase, alkaline phosphatase, β-galactosidase, chloramphenicol acetyltransferase (CAT), etc.), a bioluminescence marker (e.g., green-fluorescent protein (GFP, e.g., U.S. Pat. No. 5,491,084), blue fluorescent protein (BFP, e.g., U.S. Pat. No. 6,486,382), etc.), a surface-expressed molecule (e.g., CD25, CD80, CD86), or a secreted molecule (e.g., IL-1, IL-6, IL-8, IL-12 p40, TNF-α). In certain embodiments the reporter is selected from IL-8, TNF-α, NP-κB-luciferase (NF-κB-luc; Häcker H et al. (1999) EMBO J 18:6973-82), IL-12 p40-luc (Murphy TL et al. (1995) Mol Cell Biol 15:5258-67), and TNF-luc (Häcker H et al. (1999) EMBO J 18:6973-82). In assays relying on enzyme activity readout, a substrate can be supplied as part of the assay, and detection can involve measurement of chemiluminescence, fluorescence, color development, incorporation of radioactive label, drug resistance, or another marker of enzyme activity. For assays relying on surface expression of a molecule, detection can be accomplished using flow cytometry (e.g., FACS) analysis or functional assays. Secreted molecules can be assayed using enzyme-linked immunosorbent assay (ELISA) or bioassays. Many of these and other suitable readout systems are well known in the art and are commercially available.

### Reporter Constructs

A cell expressing a functional TLR and useful for the methods of the invention has, in some embodiments, an expression vector including an isolated nucleic acid which encodes a reporter construct useful for detecting TLR signaling. The expression vector, including an isolated nucleic acid which encodes a reporter construct useful for detecting TLR signaling, can include a reporter gene under the control of a promoter response element (enhancer element). In some embodiments, the promoter response element is associated with a minimal promoter responsive to a transcription factor that is activated as a consequence of TLR signaling. Examples of such minimal promoters include, without limitation, promoters for the following genes: AP-1, NF-κB, ATF2, IRF3, and IRF7. These minimal promoters contain corresponding promoter response elements sensitive to AP-1, NF-κB, ATF2, IRF3, and IRF7, respectively. In other embodiments the expression vector including an isolated nucleic acid which encodes a reporter construct useful for detecting TLR signaling can include a gene under the control of a promoter response element selected from response elements sensitive to IL-6, IL-8, IL-12 p40 subunit, a type I IFN, RANTES, TNF, IP-10, I-TAC, and interferon-stimulated response element (ISRE). The promoter response element generally will be present in multiple copies, e.g., as tandem repeats. For example, in one reporter construct, a coding sequence for luciferase is under the control of an upstream 6X tandem repeat of an NF-κB response element. In some embodiments, an ISRE-luciferase reporter construct useful in the invention (e.g., catalog no. 219092, Stratagene, Inc., La Jolla, CA) includes a 5x ISRE tandem repeat joined to a TATA box upstream of a luciferase reporter gene. As described herein, the reporter itself can be any gene product suitable for detection by methods recognized in the art. Such methods for detection can include, for example, measurement of spontaneous or stimulated light emission, enzyme activity, expression of a soluble molecule, expression of a cell surface molecule, etc.

Readouts typically involve the usual elements of Toll/IL-1R signaling, e.g., MyD88, TRAF, and IRAK molecules, although in the case of TLR3 the role of MyD88 is less clear than for other TLR family members. As described herein, such responses include the induction of a gene under control of a specific promoter such as an NF-κB promoter, increases in particular cytokine levels, increases in particular chemokine levels, etc. The gene under the control of the NF-κB promoter can be a gene which naturally includes an NF-κB promoter or it can be a gene in a construct in which an NF-κB promoter has been inserted. Genes and constructs which include the NF-κB promoter include but are not limited to IL-8, IL-12 p40, NF-κB-luc, IL-12 p40-luc, and TNF-luc.

Increases in cytokine levels can result from increased production, increased stability, increased secretion, or any combination of the foregoing, of the cytokine in response to the TLR-mediated signaling. Cytokines generally include, without limitation, IL-1, IL-2, IL-3, IL-4, IL-5, IL-6, IL-7, IL-10, IL-11, IL-12, IL-13, IL-15, IL-18, IFN-α, IFN-β, IFN-γ, TNF-α, GM-CSF, G-CSF, M-CSF. Th1 cytokines include but are not limited to IL-2, IFN-γ, and IL-12. Th2 cytokines include but are not limited to IL-4, IL-5, and IL-10.

Increases in chemokine levels can result from increased production, increased stability, increased secretion, or any combination of the foregoing, of the chemokine in response to the TLR-mediated signaling. Chemokines of particular significance in the invention include but are not limited to CCL5 (RANTES), CXCL9 (Mig), CXCL10 (IP-10), and CXCL11 (I-TAC), IL-8, and MCP-1.

### Abbreviations

- ACN: Acetonitrile
- EA: Ethyl acetate
- DMF: Dimethyl formamide
- PE: Petroleum ether
- DCM: Dichloromethane
- THF: Tetrahydrofuran
- HOBT: 1-Hydroxybenzotriazole
- EDCI: 1-Ethyl-3-(3-dimethylaminopropyl)carbodiimide
- HBTU: 2-(1H-Benzotriazole-1-yl)-1,1,3,3-tetramethyluronium hexafluorophosphate
- HATU: N-[(dimethylamino)(3H-1,2,3-triazolelo(4,4-b)pyridin-3-yloxy)methylene]-N-methylmethaneaminium hexafluorophosphate
- PyBOP: 1H-Benzotriazol-1-yloxytripyrrolidinophosphoniumhexafluorophosphate
- BOPC1: Bis(2-oxo-3-oxazolidinyl)phosphinic chloride
- BOP: Benzotriazol-1-yloxytris(diethylamino)phosphonium hexafluorophospahte
- TEA: Triethylamine
- DIPEA: Diisopropylethylamine
- DMAP: 4-Dimethylaminopyridine
- PCC: Pyridinium chlorochromate
- PDC: Pyridinium dichromate
- NBS: N-bromosuccinimide
- NCS: N-chlorosuccinimide
- NIS: N-iodosuccinimide
- 9-BBN: 9-Borabicyclo[3.3.1]nonane
- TsOH: p-Toluenesulfonic acid
- TFA: Trifluoroacetamide
- CDI: Carbonyldiimidazole

### Methods of Preparation

Following are general synthetic schemes for manufacturing compounds of the present invention. These schemes are illustrative and are not meant to limit the possible techniques one skilled in the art may use to manufacture the compounds disclosed herein. Different methods will be evident to those skilled in the art. Additionally, the various steps in the synthesis may be performed in an alternate sequence or order to give the desired compound(s). All documents cited herein are incorporated herein by reference in their entirety. For example, the following reactions are illustrations but not limitations of the preparation of some of the starting materials and compounds disclosed herein.

### Synthetic Route 1

Schemes below describe Synthetic Route 1 which may be used for the synthesis of compounds of the present invention, e.g., compounds having a structure of Formula I, Ia, or II. Various modifications to these methods may be envisioned by those skilled in the art to achieve similar results to that of the inventors given below. In the embodiments below, the synthetic route is described using a compound having the structure of Formula II as an example. However, this synthetic route can be used for the preparation of compounds having a structure of Formula I or Ia as well.

In yet another aspect, a method for the synthesis of a compound having the structure of Formula II is described, 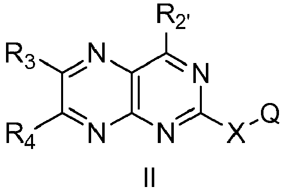 comprising:
(a) converting a compound having the structure of Formula III to a compound having the structure of Formula IV: 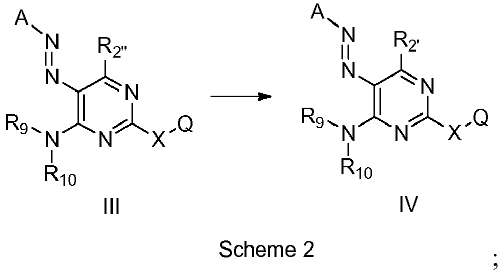 and
(b) converting the compound having the structure of Formula IV to the compound having the structure of Formula II: 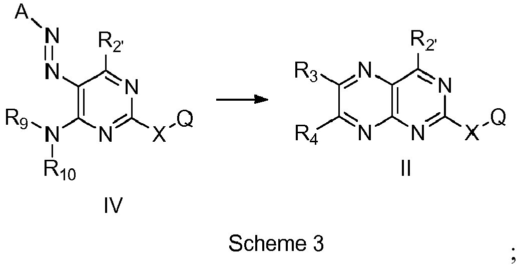 wherein
   each occurrence of X is independently absent or is an alkyl, cycloalkyl, aryl, or heterocycle;
   each occurrence of Q is independently H, (CH₂)_{q}NR_{b}R_{c}, NRₐ(CH₂)ₚNR_{b}R_{c}, OR₁, SR₁, 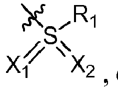 or CRₐR_{b}R_{c}, in which q is 0 or 1 and p is 2-4; and X₁ and X₂ are each independently absent or O;
   R₁ is hydrogen, alkyl, alkenyl, cycloalkyl, alkylcycloalkyl, aryl, alkylaryl, heterocycle, alkylheterocycle;
   R_{2"} is halogen, ORa, OS(=O)₂Rₐ, or OC(=O)Rₐ;
   R_{2'} is OH, NR_{b}R_{c}, or NRₐ(CH₂)ₚNR_{b}R_{c};
   A is aryl or heteroaryl;
   each occurrence of R₉ and R₁₀ is each independently hydrogen, OS(=O)₂Rₐ, CH₂C(=O)ORₐ, C(=O)C(=O)ORₐ, OC(=O)Rₐ, OC(=O)ORₐ, or R_{a'}, or alternatively R₉ and R₁₀ are taken together with the nitrogen atom to which that they are attached to form a mono- or bicyclic carbocycle or heterocycle, wherein the carbocycle or heterocycle is optionally substituted with oxo;
   R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
   each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
   wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl.

In certain embodiments, step (a) in Scheme 2 comprises a substitution reaction of the R_{2"} group in Formula III by a R_{2'} group in Formula IV. R_{2"} may be a leaving group such as halogen, ORₐ, OS(=O)₂Rₐ, or OC(=O)Rₐ. R_{2'} may be OH, NR_{b}R_{c}, or NRₐ(CH₂)ₚNR_{b}R_{c}. Step (a) may be carried out using a nucleophilic reagent R_{2'}H. Any suitable base, organic or inorganic, may be used in step (a). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

In certain embodiments, step (b) in Scheme 3 comprises removal of the 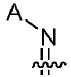 protection group in Formula IV and a cyclization reaction to form the pteridine ring system in Formula II. A can be any optionally-substituted aryl or heteroaryl. In certain embodiments, A is optionally-substituted phenyl. Non-limiting examples of conditions for removing the 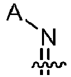 protection group include hydrogenation, e.g., using hydrogen in the presence of a catalyst such as Pd. Any other conditions known in the art can be used. Once, the 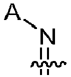 protection group is removed, a suitable NR₉R₁₀ can be cyclized onto the newly formed NH₂ group after the removal of the 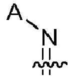 protection group. Such suitable, cyclizable NR₉R₁₀ substituents include, but are not limited to, NHCH₂C(=O)ORₐ and NHC(=O)C(=O)ORₐ. Such suitable, cyclizable NR₉R₁₀ substituents may be installed to the intermediates at any stage of the synthesis, e.g., in compound of Formula III, IV, or immediately prior to the cyclization to form the pteridine ring. Suitable solvents for the cyclization include DMSO, ethanol, water, THF, methylene chloride, acetonitrile, chloroform or toluene. In certain embodiments, a non-aromatic ring may be formed during cyclization and the step (b) further comprises the use of an aromatization reagent or dehydrogenation reagent to form the aromatic pteridine system. Non-limiting examples of such aromatization reagents and dehydrogenation reagents include

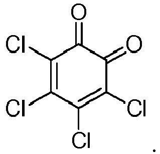

In certain embodiments, R₉ and R₁₀ are protecting groups selected from the group consisting of Fmoc-, Cbz-, Boc-, Ac-, CF₃(C=O)-, Benzyl, triphenylmethyl, and p-Toluenesulfonyl; or R₉ and R₁₀ are taken together with the nitrogen atom to which they are bonded to form 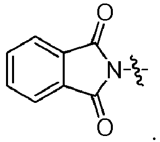 In these embodiments, protecting group R₉ and R₁₀ may be removed and suitable cyclization groups (e.g., R₉ or R₁₀ is CH₂C(=O)ORₐ or C(=O)C(=O)ORₐ) can be then installed.

In certain embodiments, the synthetic route further comprises a step of (a₁): 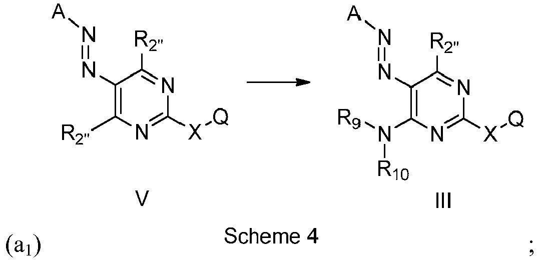 wherein each occurrence of R_{2"} is independent halogen, ORₐ, OS(=O)₂Rₐ, or OC(=O)Rₐ.

In certain embodiments, step (a₁) in Scheme 4 comprises a substitution reaction of the R_{2"} group in Formula V by a NR₉NR₁₀ group to form a compound of Formula III. R_{2"} may be a leaving group such as halogen, ORₐ, OS(=O)₂Rₐ, or OC(=O)Rₐ. Step (a₁) may be carried out using a nucleophilic reagent HNR₉NR₁₀. Any suitable base, organic or inorganic, may be used in step (a₁). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

In certain embodiments, the step (a₁) further comprises the steps of (a₂) and (a₃): 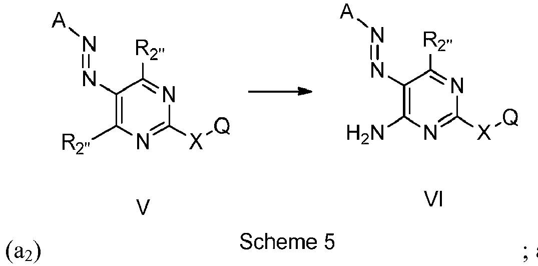 and 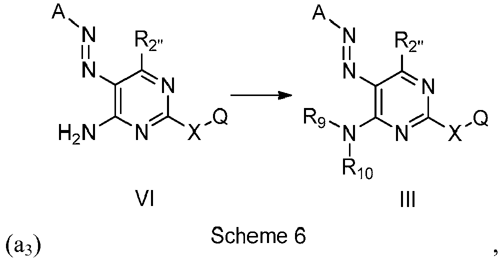 wherein at least one of R₉ and R₁₀ is not hydrogen. In step a₂, NH₃ may be used as a nucleophilic reagent to replace leaving group R_{2"} in Formula V. Any suitable base, organic or inorganic, may be used in step (a₂). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

In certain embodiments, in step a₃, R₉ and/or R₁₀ substituent may be introduced as a protecting group or cyclizable group described above, using conditions and reagents known in the art. Non-limiting examples of such reagents include ClOS(=O)₂Rₐ, halogen-CH₂C(=O)ORₐ, ClC(=O)C(=O)ORₐ, HOC(=O)Rₐ, ClC(=O)Rₐ, ClC(=O)ORₐ, and halogen-R_{a'}. Any suitable base, organic or inorganic, may be used in step (a₃). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

In certain embodiments, step (b) further comprises the steps of (b₁) and (b₂): 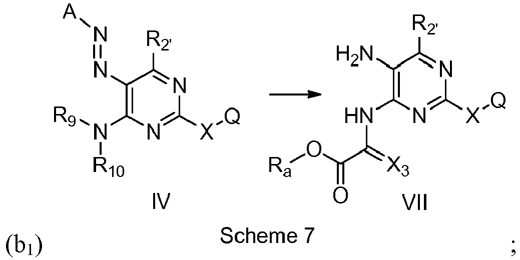 and 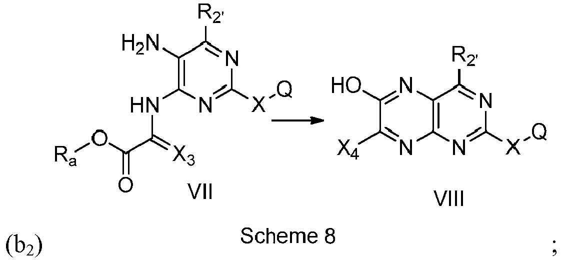 wherein X₃ is O or absent, X₄ is OH or absent, and Rₐ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl.

Thus, in certain embodiments, in step b₁, R₉ and/or R₁₀ substituent in Formula IV may be protecting groups which will be removed using any conditions and reagents known in the art. A cyclizable substituent RₐO(C=O)C(=X₃) may be then introduced using a suitable reagent such as RₐO(C=O)C(=X₃)Cl, to form a compound of Formula VII using conditions and reagents known in the art. X₃ may be O or absent. Any suitable base, organic or inorganic, may be used in the introduction of the cyclizable substituent RₐO(C=O)C(=X₃). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. The 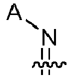 protection group may be removed in this step, before or after the removal of the protection group R₉ and/or R₁₀, or before or after the introduction of the cyclizable substituent RₐO(C=O)C(=X₃). A compound of Formula VII is then formed ready for cyclization.

In certain embodiments, in step b₂, the compound of Formula VII is cyclized to form a compound of Formula VIII. Any suitable base, organic or inorganic, may be used in the introduction of the cyclizable substituent RₐO(C=O)C(=X₃). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. The cyclization reaction may proceed at room temperature or elevated temperature. In certain embodiments, a non-aromatic ring may be formed during cyclization and the step (b) further comprises the use of an aromatization reagent or dehydrogenation reagent to form the aromatic pteridine system. Non-limiting examples of such aromatization reagents and dehydrogenation reagents include 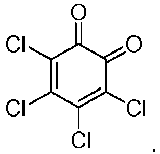 In some specific embodiments, R₉ is H and R₁₀ is -(C=O)ORₐ.

In certain embodiments, the method further comprises the steps of (b₃) and (b₄): 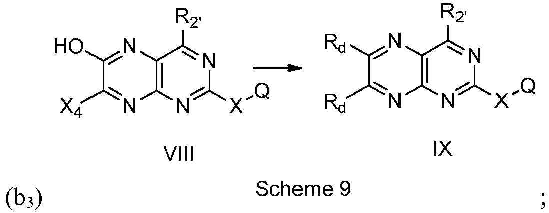 and 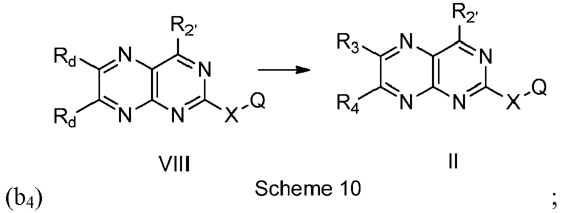 wherein each occurrence of R_{d} is independently halogen, OS(=O)₂Rₐ, or OC(=O)Rₐ.

In certain embodiments, in step b₃, the OH and/or X₄ group in the compound of Formula VIII may be converted to a leaving group R_{d}, using any suitable conditions and reagents known in the art. Non-limiting examples of the suitable reagents include Cl₂, Br₂, SOCl₂, POCl₃, ClOS(=O)₂Rₐ, HOC(=O)Rₐ and ClC(=O)Rₐ. Any suitable base, organic or inorganic, may be used in this step. Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. The reaction may proceed at room temperature or elevated temperature. In some embodiments, X₄ is absent in the compound of Formula VIII, and thus R_{d} in the compound of Formula IX and R₄ in the compound of Formula II may be H.

In this route, because R_{2'} and R₄ can be introduced at different stages of the synthesis, R_{2'} and R₄ can be two different groups. The synthetic route enables easy and convenient synthesis of a compound of Formula II where R_{2'} and R₄ are different, without the need for difficult column and/or HPLC separations of regioisomers. Of course, a compound of Formula II where R_{2'} and R₄ are the same can be made using this synthetic route as well.

In certain embodiments, X is absent. In other embodiments, X is selected from the group consisting of alkyl, cycloalkyl, aryl, and heterocycle. In some embodiments, X is-(CH₂)ₘ-, wherein m is 2-4. In other embodiments, X is aryl. Non-limiting examples of aryl include optionally substituted phenyl and napthyl. In still other embodiments, X is a heterocycle. In some embodiments, X is a saturated heterocyclic. Non-limiting examples of saturated heterocycle include piperizine. In other embodiments, X is an unsaturated heterocyclic. Non-limiting examples of unsaturated heterocycles include pyridine, pyrazine, pyrimidine, and pyridazine.

In certain embodiments, Q is H, (CH₂)_{q}NR₁R₂, NR₁(CH₂)ₚNR_{b}R_{c}, OR₁, SR₁, CR₁R₂R_{2'},or CHR₁R₂, in which q is 0 or 1 and p is 2-4. R₁, R₂, and R_{2'} are each independently hydrogen, alkyl, alkenyl, cycloalkyl, alkylcycloalkyl, aryl, alkylaryl, heterocycle, alkylheterocycle, or R₁ and R₂ together with the nitrogen atom to which they are bonded form a heterocycle, which may be optionally substituted by from one to four groups which may be the same or different selected from (C₁-C₄)alkyl, phenyl, benzyl, C(=O)R₁₂, (CH₂)ₚORₐ, and (CH₂)ₚNR_{b}R_{c}, in which p is 2-4. In some embodiments, Q is H, OR₁, SR₁, or CHR₁R_{2.} In other embodiments, Q is -(CH₂)_{q}NR₁R₂. In some specific embodiments, Q is -(CH₂)₂NR₁R₂. In some specific embodiments, Q is 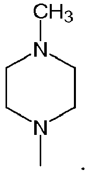 In other embodiments, Q is 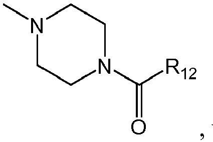 wherein R₁₂ is alkyl, aryl, or heterocycle. In some embodiments, C(=O)R₁₂ is 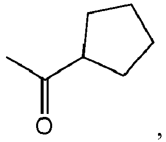 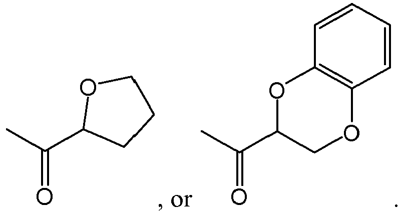 In still other embodiments, Q is NH(CH₂)ₚNR_{b}R_{c}. In some specific embodiments, Q is NH(CH₂)₂N(CH₃)₂, NH(CH₂)₂N(CH₂CH₃)₂, or NH(CH₂)₂N(CH₃)(CH₂CH₃). In still other embodiments, Q is alkyl. Non-limiting examples of alkyl include methyl, ethyl, propyl, *iso*-propyl, butyl, *sec*-butyl, and *tert*-butyl.

In some embodiments, X is absent and Q is (CH₂)_{q}NR_{b}R_{c}, NRₐ(CH₂)ₚNR_{b}R_{c}, OR₁, SR₁, or

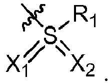

In some embodiments, X is a phenyl group. In these embodiments, Q is attached to phenyl group at the ortho, meta, or para position relative to the pteridine core. In some specific embodiments, Q is 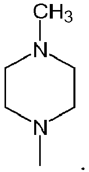 In some specific embodiments, Q is 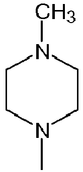 attached to the phenyl group at the para position relative to the pteridine core. In some specific embodiments, -X-Q is 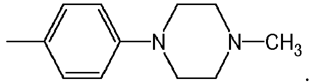 In other specific embodiments, -X-Q is 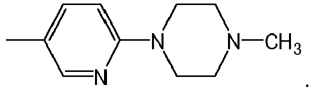 In still other specific embodiments, -X-Q is 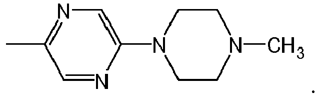 In still other specific embodiments, -X-Q is 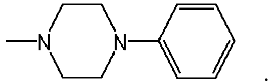 In these embodiments, Y is NH, S, or O and L is -(CH₂)ₘ- wherein m is 2-6. Other substituent groups are as described herein.

### Synthetic Route 2

Schemes below describe Synthetic Route 2 which may be used for the synthesis of compounds of the present invention, e.g., compounds having a structure of Formula I, la, or II. Various modifications to these methods may be envisioned by those skilled in the art to achieve similar results to that of the inventors given below. In the embodiments below, the synthetic route is described using a compound having the structure of Formula II as an example. However, this synthetic route can be used for the preparation of compounds having a structure of Formula I or Ia as well.

In yet another aspect, a method for the synthesis of a compound having the structure of Formula II is described, 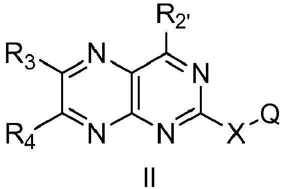 comprising:
(a) converting a compound having the structure of Formula X to a compound having the structure of Formula XI: 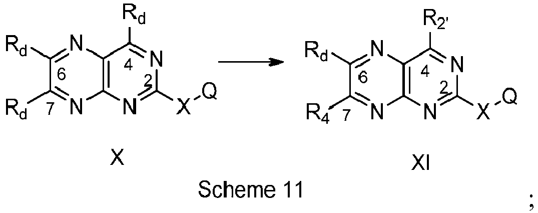 and
(b) converting the compound having the structure of Formula XI to the compound having the structure of Formula II: 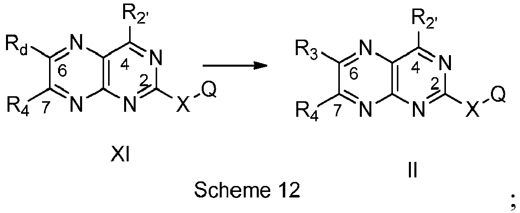 wherein
   each occurrence of X is independently absent or is an alkyl, cycloalkyl, aryl, or heterocycle;
   each occurrence of Q is independently H, R_{d}, (CH₂)_{q}NRₐR_{b}, NRₐ(CH₂)ₚNR_{b}R_{c}, OR₁, SR₁, 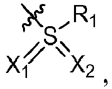 or CRₐR_{b}R_{c}, in which q is 0 or 1 and p is 2-4; and X₁ and X₂ are each independently absent or O;
   R₁ is hydrogen, alkyl, alkenyl, cycloalkyl, alkylcycloalkyl, aryl, alkylaryl, heterocycle, alkylheterocycle;
   each occurrence of R_{d} is independently halogen, OS(=O)₂Rₐ, or OC(=O)Rₐ;
   R_{2'} is OH, NR_{b}R_{c}, or NRₐ(CH₂)ₚNR_{b}R_{c};
   R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
   each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
   wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   with the proviso that step (b) can be omitted if R_{d} at the 6 position of the compound of Formula XI and R₃ are the same.

In certain embodiments, step (a) in Scheme 11 comprises a substitution reaction of the R_{d} groups at the 4- and 7 position in Formula X by a R_{2'} and R₄ group, respectively, to form a compound of Formula XI. R_{2'} may be OH, NR_{b}R_{c}, or NRₐ(CH₂)ₚNR_{b}R_{c}; R₃ and R₄ each may be independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4. Step (a) may be carried out using nucleophilic reagents R_{2'}H and R₄H, sequentially in either order, or in one pot. Any suitable base, organic or inorganic, may be used in step (a). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

When R_{2'} and R₄ are the same, no separation of region-isomers is necessary. When R_{2'} and R₄ are different, however, column chromatography purification and/or HPLC purification may be necessary to separate regioisomers. This is mainly due to the similar reactivities of the 4 and 7 positions of the pteridine ring.

In certain embodiments, step (a) in Scheme 11 may further comprise steps (a₁) and (a₂): 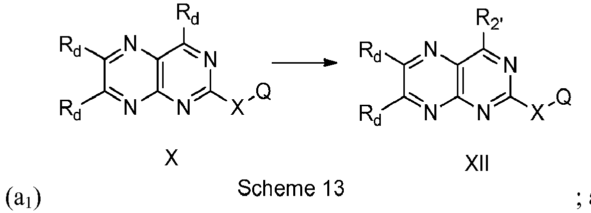 and 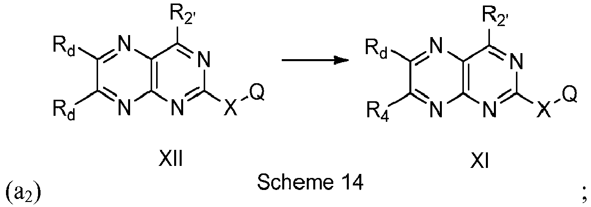 wherein step (a₁) further compresses purifying the compound having the structure of Formula XII. Thus, the R_{d} group at the 4 position may be firstly replaced with R_{2'} using a nucleophilic reagent R_{2'}H. Any suitable base, organic or inorganic, may be used in step (a₁) in Scheme 13. Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. This reaction may generate by-product where the R_{d} group at the 7 position is replaced with R_{2'}. Thus, further purification by HPLC and/or column may be necessary.

Subsequently, the R_{d} group at the 7 position may be replaced with R₄ using a nucleophilic reagent R₄H. Any suitable base, organic or inorganic, may be used in step (a₂) in Scheme 14. Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

In certain embodiments, step (a) in Scheme 11 may further comprise steps (a₁) and (a₂): 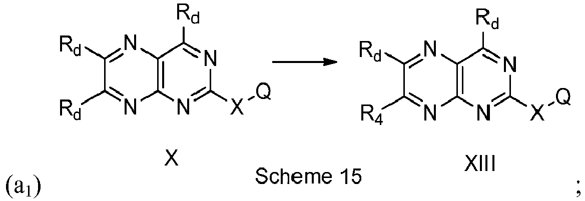 and 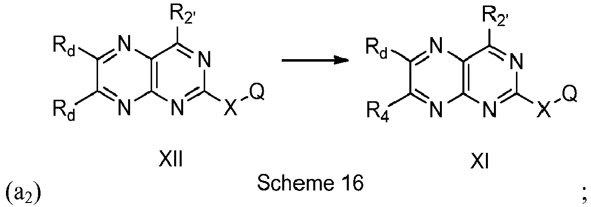 wherein step (a₁) further compresses purifying the compound having the structure of Formula XII. Thus, R_{d} group at the 7 position may be firstly replaced with R₄ using a nucleophilic reagent R₄H. Any suitable base, organic or inorganic, may be used in step (a₁) in Scheme 15. Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. This reaction may generate by-product where the R_{d} group at the 4 position is replaced with R₄. Thus, further purification by HPLC and/or column may be necessary.

Subsequently, R_{d} group at the 4 position may be replaced with R_{2'} using a nucleophilic reagent R_{2'}H. Any suitable base, organic or inorganic, may be used in step (a₂) in Scheme 16. Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene.

In certain embodiments, step (a) in Scheme 11 comprises a one-pot synthetic step (a₁ₓ): 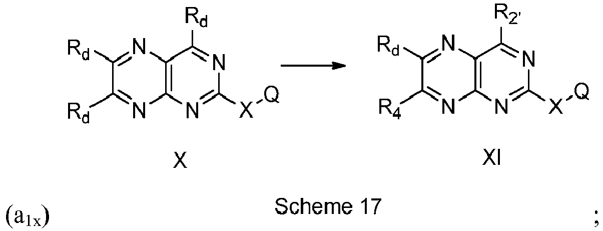 wherein step (a₁ₓ) further comprises purifying the compound having the structure of Formula XI. Thus, the one-pot synthesis may use nucleophilic reagents R_{2'}H and R₄H together, under conditions described herein for the nucleophilic substitution. A purification by a column chromatography purification or HPLC purification may be needed.

In certain embodiments, the substituent -X-Q in Formulae X and XI is R_{d}, and step (a) in Scheme 11 comprises converting a compound having the structure of Formula X' to a compound having the structure of Formula XI': 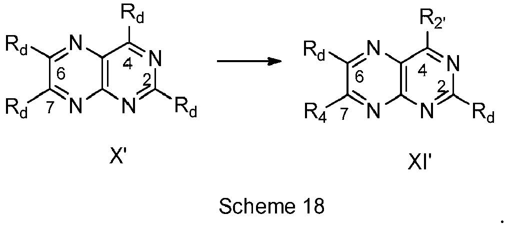 Thus, as shown in Scheme 18, substitution reactions of the R_{d} groups at the 4- and 7 position in Formula X' by a R_{2'} and R₄ group, respectively, form a compound of Formula XI'. R_{2'} may be OH, NR_{b}R_{c}, or NRₐ(CH₂)ₚNR_{b}R_{c}; R₃ and R₄ each may be independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4. The reaction in Scheme 18 may be carried out using nucleophilic reagents R_{2'}H and R₄H, sequentially in either order, or in one pot. Any suitable base, organic or inorganic, may be used in step (a). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. In certain specific embodiments, R_{2'} and R₄ are the same. In one embodiment, R_{2'} and R₄ are both

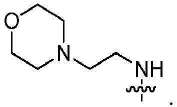

In certain embodiments, the method further comprises step (a'): converting a compound having the structure of Formula XI" to a compound having the structure of Formula XI''' as shown in Scheme 19.

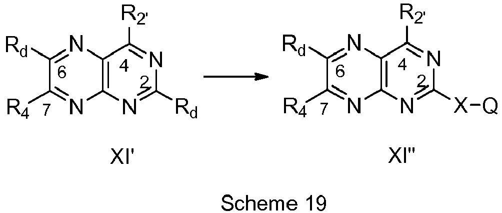

In certain specific embodiments, -X-Q is NRₐR_{b}, NRₐ(CH₂)ₚNR_{b}R_{c}, OR₁, or SR₁. In one embodiment, -X-Q is 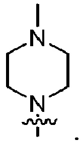 The reaction in Scheme 19 may be carried out using nucleophilic reagents Q-X-H. Any suitable base, organic or inorganic, may be used in step (a). Non-limiting examples of suitable bases include Na₂CO₃, K₂CO₃, NaOH, KOH, CsOH, sodium hydride, potassium carbonate, triethylamine, and diisopropylethylamine. Suitable solvents for this reaction include DMSO, ethanol, water, THF, methylene chloride, acetonitrile, chloroform or toluene. In certain specific embodiments, R_{2'} and R₄ are the same. In certain specific embodiments, R_{2'} and R₄ are both 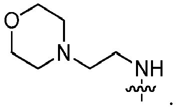 In certain specific embodiments, R_{d} and R₃ are both Cl.

In certain specific embodiments, the method comprises the following two steps: 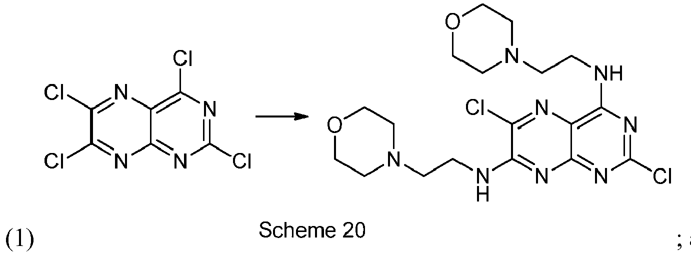 and

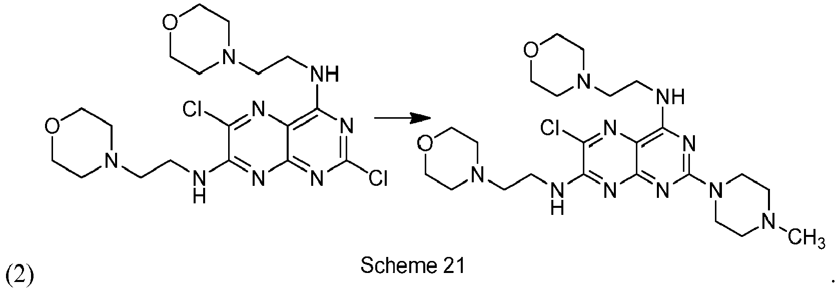

In certain specific embodiments, the method further comprises preparing the compound having the structure of 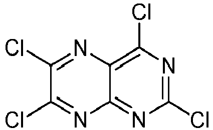 by the following steps:

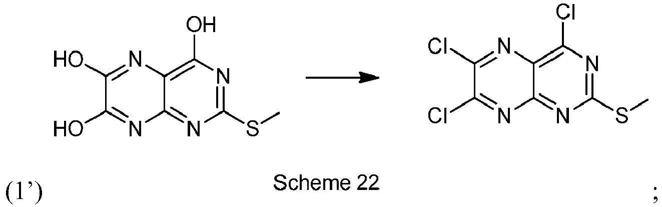

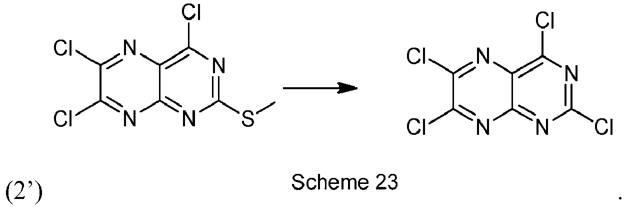

In certain specific embodiments, the method further comprises preparing the compound having the structure of 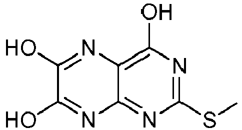 by the following steps: 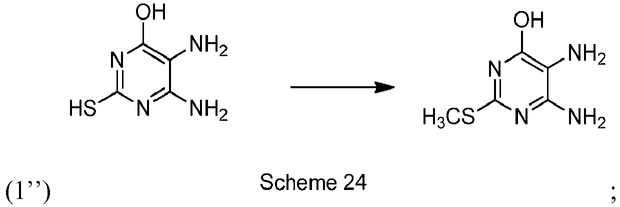 and

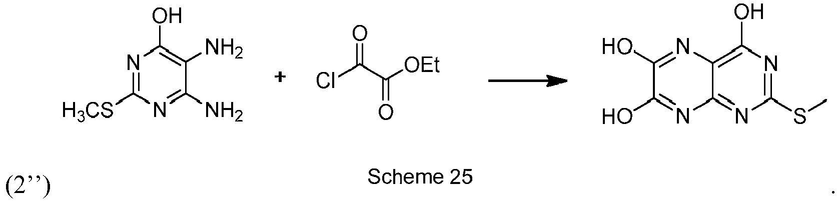

In some embodiments, any of the reactions described in any of schemes 1-25 may be carried out at room temperature or at 0 °C. In other embodiments, any of the reactions described in any of schemes 1-19 may be carried out at an elevated temperature. Non-limiting examples of the elevated temperature include about 30, 35, 40, 45, 50, 55, 60, 65, 70, 75, 80, 90, 100, 110, 120, 130, 140, 150, 160, 170, 180, 190, or 200 °C, or in a range bounded by any of the two values disclosed herein.

In certain embodiments, X is absent. In other embodiments, X is selected from the group consisting of alkyl, cycloalkyl, aryl, and heterocycle. In some embodiments, X is-(CH₂)ₘ-, wherein m is 2-4. In other embodiments, X is aryl. Non-limiting examples of aryl include optionally substituted phenyl and napthyl. In still other embodiments, X is a heterocycle. In some embodiments, X is a saturated heterocycle. Non-limiting examples of saturated heterocycle include piperizine. In other embodiments, X is an unsaturated heterocycle. Non-limiting examples of unsaturated heterocycles include pyridine, pyrazine, pyrimidine, and pyridazine.

In certain embodiments, Q is H, (CH₂)_{q}NR₁R₂, NR₁(CH₂)ₚNR_{b}R_{c}, OR₁, SR₁, CR₁R₂R_{2'}, or CHR₁R₂, in which q is 0 or 1 and p is 2-4. R₁, R₂, and R_{2'} are each independently hydrogen, alkyl, alkenyl, cycloalkyl, alkylcycloalkyl, aryl, alkylaryl, heterocycle, alkylheterocycle, or R₁ and R₂ together with the nitrogen atom to which they are bonded form a heterocycle, which may be optionally substituted by from one to four groups which may be the same or different selected from (C₁-C₄)alkyl, phenyl, benzyl, C(=O)R₁₂, (CH₂)ₚORₐ, and (CH₂)ₚNR_{b}R_{c}, in which p is 2-4. In some embodiments, Q is H, OR₁, SR₁, or CHR₁R₂. In other embodiments, Q is -(CH₂)_{q}NR₁R₂. In some specific embodiments, Q is -(CH₂)₂NR₁R₂. In some specific embodiments, Q is 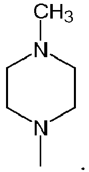 In other embodiments, Q is 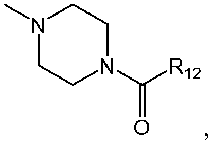 wherein R₁₂ is alkyl, aryl, or heterocycle. In some embodiments, C(=O)R₁₂ is 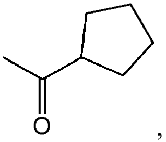 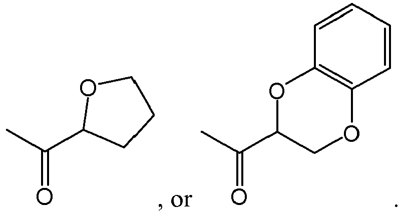 In still other embodiments, Q is NH(CH₂)ₚNR_{b}R_{c}. In some specific embodiments, Q is NH(CH₂)₂N(CH₃)₂, NH(CH₂)₂N(CH₂CH₃)₂, or NH(CH₂)₂N(CH₃)(CH₂CH₃). In still other embodiments, Q is alkyl. Non-limiting examples of alkyls include methyl, ethyl, propyl, *iso*-propyl, butyl, *sec*-butyl, and *tert*-butyl.

In some embodiments, X is a phenyl group. In these embodiments, Q is attached to phenyl group at the ortho, meta, or para position relative to the pteridine core. In some specific embodiments, Q is 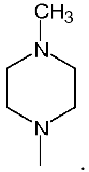 In some specific embodiments, Q is 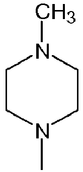 attached to the phenyl group at the para position relative to the pteridine core. In some specific embodiments, -X-Q is 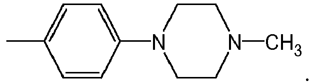 In other specific embodiments, -X-Q is 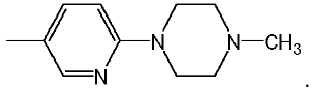 In still other specific embodiments, -X-Q is 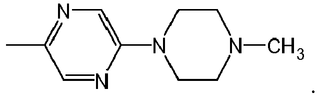 In still other specific embodiments, -X-Q is 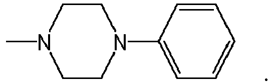 In these embodiments, Y is NH, S, or O and L is -(CH₂)ₘ- wherein m is 2-6. Other substituent groups are as described herein.

In addition, other compounds of Formulae I, la, and II may be prepared by the procedures generally known to those skilled in the art. In particular, the following examples provide additional methods for preparing compounds of this invention.

The invention will now be further described by the working examples below, which are preferred embodiments of the invention. These examples are illustrative rather than limiting, and it is to be understood that there may be other embodiments that fall within the spirit and scope of the invention as defined by the claims appended hereto.

### Pharmaceutical Compositions

This invention also provides a pharmaceutical composition comprising at least one of the compounds as described herein or a pharmaceutically acceptable salt or solvate thereof, and a pharmaceutically acceptable carrier.

In yet another aspect, a pharmaceutical composition is described, comprising at least one a compound of Formula I, la, or II or a pharmaceutically acceptable salt thereof, and a pharmaceutically-acceptable carrier or diluent. In some specific embodiments, the compound has the structure of

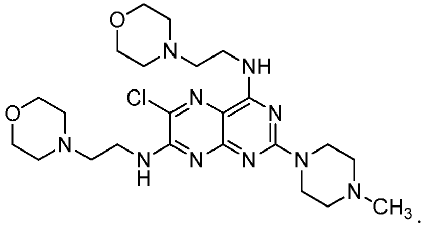

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject pharmaceutical agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as butylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations. The term "carrier" denotes an organic or inorganic ingredient, natural or synthetic, with which the active ingredient is combined to facilitate the application. The components of the pharmaceutical compositions also are capable of being comingled with the compounds of the present invention, and with each other, in a manner such that there is no interaction which would substantially impair the desired pharmaceutical efficiency.

As set out above, certain embodiments of the present pharmaceutical agents may be provided in the form of pharmaceutically acceptable salts. The term "pharmaceutically-acceptable salt", in this respect, refers to the relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al., (1977) "Pharmaceutical Salts", J. Pharm. Sci. 66:1-19.)

The pharmaceutically acceptable salts of the subject compounds include the conventional nontoxic salts or quaternary ammonium salts of the compounds, *e.g*., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, butionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge *et al.,* supra.)

Wetting agents, emulsifiers and lubricants, such as sodium lauryl sulfate, magnesium stearate, and polyethylene oxide-polybutylene oxide copolymer as well as coloring agents, release agents, coating agents, sweetening, flavoring and perfuming agents, preservatives and antioxidants can also be present in the compositions.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient, which can be combined with a carrier material to produce a single dosage form will generally be that amount of the compound which produces a therapeutic effect. Generally, out of 100%, this amount will range from about 1% to about 99% of active ingredient, preferably from about 5% to about 70%, most preferably from about 10% to about 30%.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouthwashes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, sodium carbonate, and sodium starch glycolate; solution retarding agents, such as paraffin; absorption accelerators, such as quaternary ammonium compounds; wetting agents, such as, for example, cetyl alcohol, glycerol monostearate, and polyethylene oxide-polybutylene oxide copolymer; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxybutylmethyl cellulose), lubricant, inert diluent, preservative, disintegrant (for example, sodium starch glycolate or cross-linked sodium carboxymethyl cellulose), surface-active or dispersing agent. Molded tablets, may be, made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxybutylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions, which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isobutyl alcohol, ethyl carbonate, ethyl acetate, benzyl alcohol, benzyl benzoate, butylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Additionally, cyclodextrins, e.g., hydroxybutyl-β-cyclodextrin, may be used to solubilize compounds.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar--agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active pharmaceutical agents of the invention.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and butane.

Transdermal patches have the added advantage of providing controlled delivery of a compound of the present invention to the body. Such dosage forms can be made by dissolving, or dispersing the pharmaceutical agents in the proper medium. Absorption enhancers can also be used to increase the flux of the pharmaceutical agents of the invention across the skin. The rate of such flux can be controlled, by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle. One strategy for depot injections includes the use of polyethylene oxide-polypropylene oxide copolymers wherein the vehicle is fluid at room temperature and solidifies at body temperature.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly (anhydrides). Depot injectable formulations are also prepared by entrapping the drug in liposomes or microemulsions, which are compatible with body tissue.

When the compounds of the present invention are administered as pharmaceuticals, to humans and animals, they can be given *per se* or as a pharmaceutical composition containing, for example, 0.1% to 99.5% (more preferably, 0.5% to 90%) of active ingredient in combination with a pharmaceutically acceptable carrier.

The compounds and pharmaceutical compositions of the present invention can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, the compound of the present invention may be administered concurrently with another anti-inflammatory or immunesupressant agent); such as but not limited to NSAIDS, DMARDS, steroids, or biologics such as antibody therapies) or they may achieve different effects (e.g., control of any adverse effects).

The compounds of the invention may be administered intravenously, intramuscularly, intraperitoneally, subcutaneously, topically, orally, or by other acceptable means. The compounds may be used to treat arthritic conditions in mammals (e.g., humans, livestock, and domestic animals), race horses, birds, lizards, and any other organism, which can tolerate the compounds.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### Administration to a Subject

Some aspects of the invention involve administering an effective amount of a composition to a subject to achieve a specific outcome. The small molecule compositions useful according to the methods of the present invention thus can be formulated in any manner suitable for pharmaceutical use.

The formulations of the invention are administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the compound can be administered to a subject by any mode allowing the compound to be taken up by the appropriate target cells. "Administering" the pharmaceutical composition of the present invention can be accomplished by any means known to the skilled artisan. Specific routes of administration include but are not limited to oral, transdermal (e.g., via a patch), parenteral injection (subcutaneous, intradermal, intramuscular, intravenous, intraperitoneal, intrathecal, etc.), or mucosal (intranasal, intratracheal, inhalation, intrarectal, intravaginal, etc.). An injection can be in a bolus or a continuous infusion.

For example the pharmaceutical compositions according to the invention are often administered by intravenous, intramuscular, or other parenteral means. They can also be administered by intranasal application, inhalation, topically, orally, or as implants, and even rectal or vaginal use is possible. Suitable liquid or solid pharmaceutical preparation forms are, for example, aqueous or saline solutions for injection or inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of present methods for drug delivery, see Langer R (1990) Science 249:1527-33, which is incorporated herein by reference.

The concentration of compounds included in compositions used in the methods of the invention can range from about 1 nM to about 100 µM. Effective doses are believed to range from about 10 picomole/kg to about 100 micromole/kg.

The pharmaceutical compositions are preferably prepared and administered in dose units. Liquid dose units are vials or ampoules for injection or other parenteral administration. Solid dose units are tablets, capsules, powders, and suppositories. For treatment of a patient, depending on activity of the compound, manner of administration, purpose of the administration (*i.e.,* prophylactic or therapeutic), nature and severity of the disorder, age and body weight of the patient, different doses may be necessary. The administration of a given dose can be carried out both by single administration in the form of an individual dose unit or else several smaller dose units. Repeated and multiple administration of doses at specific intervals of days, weeks, or months apart are also contemplated by the invention.

The compositions can be administered *per se* (neat) or in the form of a pharmaceutically acceptable salt. When used in medicine the salts should be pharmaceutically acceptable, but non-pharmaceutically acceptable salts can conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Compositions suitable for parenteral administration conveniently include sterile aqueous preparations, which can be isotonic with the blood of the recipient. Among the acceptable vehicles and solvents are water, Ringer's solution, phosphate buffered saline, and isotonic sodium chloride solution. In addition, sterile, fixed oils are conventionally employed as a solvent or suspending medium. For this purpose any bland fixed mineral or non-mineral oil may be employed including synthetic mono- or diglycerides. In addition, fatty acids such as oleic acid find use in the preparation of injectables. Carrier formulations suitable for subcutaneous, intramuscular, intraperitoneal, intravenous, etc. administrations can be found in Remington's Pharmaceutical Sciences, Mack Publishing Company, Easton, PA.

The compounds useful in the invention can be delivered in mixtures of more than two such compounds. A mixture can further include one or more adjuvants in addition to the combination of compounds.

A variety of administration routes is available. The particular mode selected will depend, of course, upon the particular compound selected, the age and general health status of the subject, the particular condition being treated, and the dosage required for therapeutic efficacy. The methods of this invention, generally speaking, can be practiced using any mode of administration that is medically acceptable, meaning any mode that produces effective levels of response without causing clinically unacceptable adverse effects. Preferred modes of administration are discussed above.

The compositions can conveniently be presented in unit dosage form and can be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product.

Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di-and tri-glycerides; hydrogel release systems; silastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Pat. Nos. 4,452,775, 4,675,189, and 5,736,152, and (b) diffusional systems in which an active component permeates at a controlled rate from a polymer such as described in U.S. Pat. Nos. 3,854,480, 5,133,974 and 5,407,686. In addition, pump-based hardware delivery systems can be used, some of which are adapted for implantation.

### Equivalents

The representative examples which follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art. The following examples contain important additional information, exemplification and guidance which can be adapted to the practice of this invention in its various embodiments and equivalents thereof.

### EXAMPLES

**Example 1. Synthesis of** 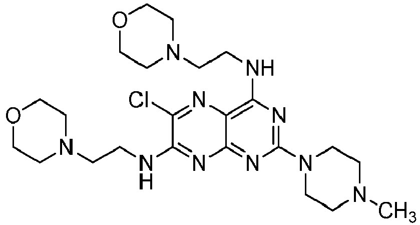 **using Synthetic Route 1**

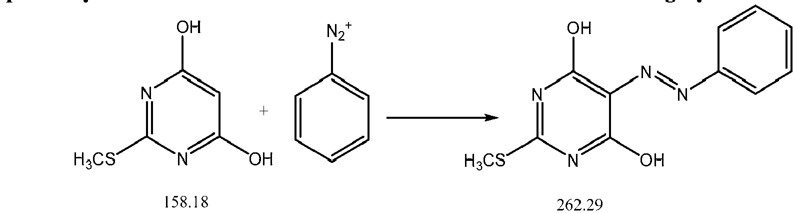

Solution 1: 2-methylthio-4,6-dihydroxypyrimidine (15.8 gm, 0.10 moles) was dissolved in water (200 mL) containing sodium hydroxide (24 gm, 0.60 moles). Once dissolution was complete, the solution was cooled on ice and ice chips (100 gm) were added. Solution 2: Aniline (9.3 gm, 0.10 moles) and concentrated hydrochloric acid (20 mL) were added to water (150mL) and ice (150 gm). To this solution was added a solution of sodium nitrite (6.9 gm, 0.10 moles) dissolved in water (50 mL). The addition was done dropwise and with stirring using a separatory funnel with the spout situated below the surface of the aniline solution. Once the addition was complete the diazonium solution was stirred at 0°C for ten minutes.

Solution 2 was then added to solution 1 with stirring at 0°C. The resulting red solution was stirred for 30 minutes and was then acidified with concentrated hydrochloric acid causing the azopyrimidine to separate as a bright yellow solid. The solid was isolated by filtration and was washed well with water before being dried. The yield was 25 gm (95.3%). 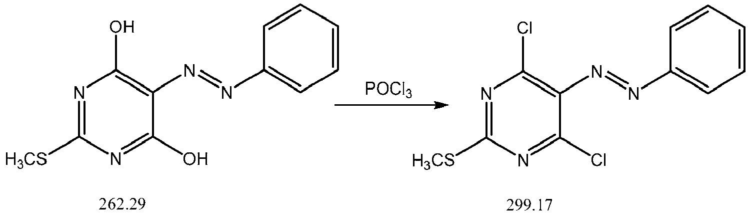 The azopyrimidine (25 gm, 9.53 X 10⁻² moles) was stirred in phosphorous oxychloride (150 mL) and diisopropylethylamine (35 mL) was slowly added. The solution warmed to about 50°C. This solution was heated to reflux for 5 minutes and was then stirred at room temperature overnight. The excess phosphorous oxychloride was removed under reduced pressure. The residual red solid was dissolved in chloroform (250 mL) and this solution was stirred with water (200 mL). Solid sodium hydrogen carbonate was added in portions until the evolution of carbon dioxide stopped. After stirring for an additional 15 minutes, the chloroform solution was isolated and dried over magnesium sulfate. After filtering to remove the drying agent, the chloroform was evaporated under reduced pressure. The resulting solid was stirred in water (400 mL) and solid sodium hydrogen carbonate was added in portions until the evolution of carbon dioxide stopped and the pH held at 7.0 - 8.0. The solid 4,6-dichloro-2-methylthio-5-phenylazopyrimidine was isolated by filtration, washed with water and dried. The red product was obtained in a yield of 28 gm (98%).

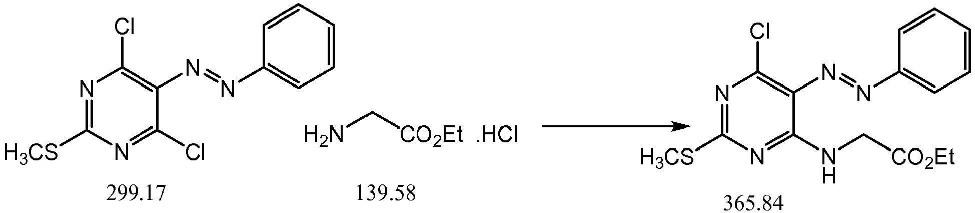

A mixture of 4,6-dichloro-2-methylthio-5-phenylazopyrimidine (5.98 gm, 0.02 moles) and glycine ethyl ester hydrochloride (2.79 gm, 0.02 moles) was stirred in ethanol (100 mL). To this was added diisopropylethylamine (5.17 gm, 6.97 mL, 0.04 moles). This caused the formation of a dark yellow solution. After stirring for a few minutes, solid began to separate. After stirring at room temperature for 1 hour, the reaction was stored in the freezer overnight. The solid which had crystallized was isolated by filtration and was washed with ethanol and then hexane before being dried. The yield of the yellow product was 6.3 gm (86.1%).

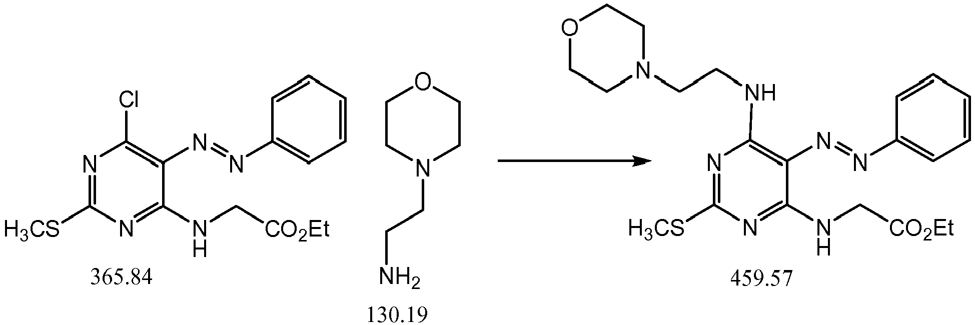

The chloropyrimidine (17.36 gm, 4.75 X 10⁻² moles) and N-(2-aminoethyl)morpholine (12.36 gm, 9.5 X 10⁻² moles) were combined in 2-butanol (100 mL) and the mixture was brought to reflux. After 30 minutes at reflux, the solution was cooled to room temperature causing the product to crystallize from solution. After cooling on ice, the product was isolated by filtration and was washed with 2-propanol before being dried. The yield of the yellow product was 18.7 gm (85.7%).

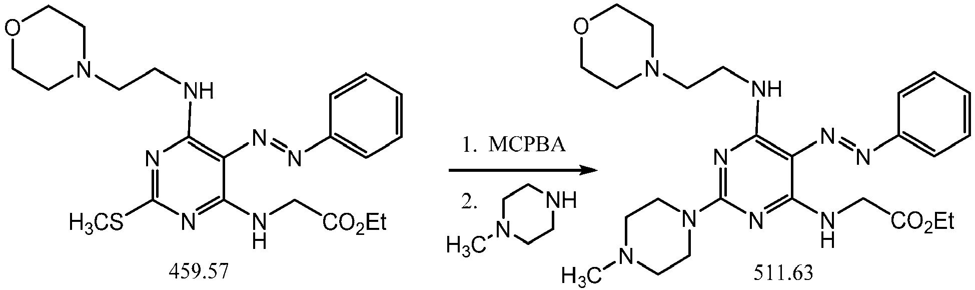

A solution of the methylthiopyrimidine (39.54 gm, 8.6 X 10⁻² moles) was dissolved in chloroform (200 mL). This solution was cooled on ice as a solution of m-chloroperoxybenzoic acid (40.1 gm of 77% acid/23% water, 0.172 moles) dissolved in chloroform (450 mL) was slowly added through a dropping funnel. After the addition was complete, the reaction was stirred at room temperature overnight. To this was added finely powdered potassium carbonate (30 gm) and stirring was continued for 2 hours. The solids were removed by filtration through Celite and the Celite was washed well with chloroform. The combined filtrates were evaporated under reduced pressure. The remaining material was stirred with 2-propanol (200 mL) and N-methylpiperazine (17.23 gm, 19.1 mL, 0.172 moles) was added. After being stirred at reflux for 1 hour, the reaction was stirred at room temperature overnight. The 2-propanol was removed by evaporation at reduced pressure and the remaining material was partitioned between chloroform (400 mL) and 20% potassium carbonate solution (200 mL). The chloroform solution was dried over magnesium sulfate. After filtration to remove the drying agent, the filtrates were evaporated at reduced pressure. The resulting solid was stirred in diethyl ether before being isolated by filtration and dried. The yield of product was 36.0 gm (81.8%).

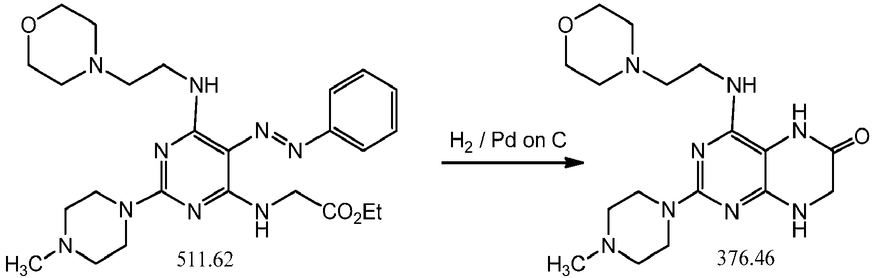

The Azopyrimidine (25 gm, 5.44 X 10⁻² moles) was dissolved in a mixture of methanol (150 mL) and THF (100 mL). To this was added 10% palladium on carbon (50% water, 15 gm). This was hydrogenated on a Parr shaker under 50 PSI of hydrogen. After 4 hours, hydrogen consumption had stopped and the solution had turned from dark yellow to colorless. The reaction was kept under 50 PSI of hydrogen for 24 hours to allow the completion of ring closure. Then the catalyst was removed by filtration and the filtrates were evaporated under reduced pressure. Diethyl ether (100 mL) was added to the remaining material and this was stirred for 30 minutes causing the product to crystallize. The solid product was isolated by filtration, washed with ether and dried. The yield was 14.1 gm (78%).

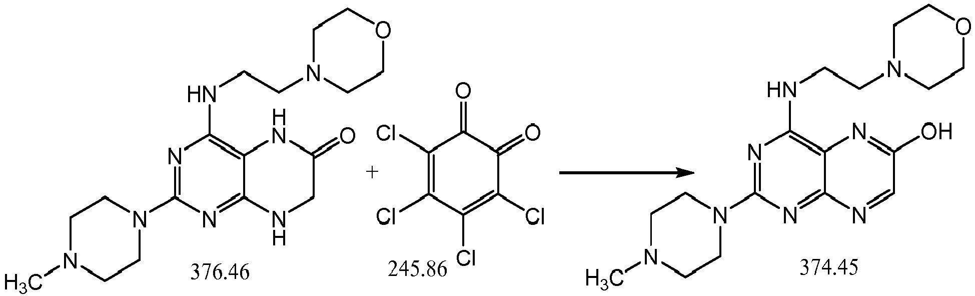

A solution of the dihydropteridinone (1.5 gm, 4.0 X 10⁻³ moles) in warm 40% methanol in tetrahydrofuran (100 mL) was stirred as a solution of O-chloranil (984 mg, 4.0 X 10⁻³ moles) in tetrahydrofuran(60 mL) was slowly added through an addition funnel. After the addition was complete, the yellow solution was stirred for 15 minutes and then 4M hydrogen chloride in dioxane (1.0 mL, 4.0 X 10⁻³ moles) was added. This was stirred at room temperature for 1 hour during which time solid separated. The reaction was cooled in the freezer and the solid was then collected by filtration. After washing with diethyl ether, the solid was dried. The yield was 1.05 gm, 70.1%.

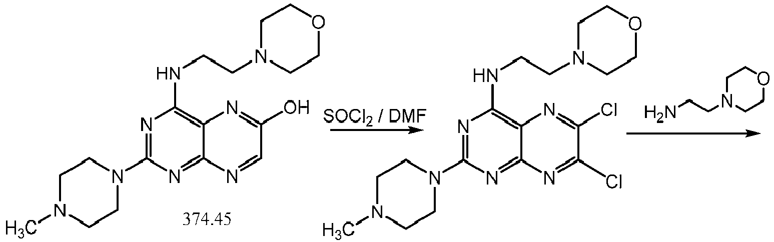

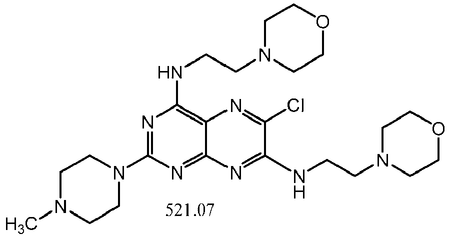

The 6-hydroxypteridine (385 mg, 1.03 X 10⁻³ moles) was heated at reflux in thionyl chloride (10 mL) and dimethylformamide (1 mL). After 3 hours, the reaction was cooled and the thionyl chloride was removed under reduced pressure. The remaining material was partitioned between methylene chloride (100 mL) and saturated sodium hydrogen carbonate (100 mL). The methylene chloride solution was dried over magnesium sulfate before being filtered and evaporated under reduced pressure. To the remaining material was added 2-butanol (5 mL) and N-(2-aminoethyl)morpholine 269 mg, 2.06 X 10⁻³ moles). The resulting solution was heated at reflux for 2 hours. After cooling, the reaction was partitioned between methylene chloride (50 mL) and 2% potassium carbonate (50 mL). The methylene chloride solution was dried over magnesium sulfate before filtered and evaporated under reduced pressure. The remaining material was purified by chromatography on silica using 25% methanol in chloroform as eluent. The fractions containing the product were pooled and evaporated under reduced pressure. The product, JB6121, was isolated in a yield of 300 mg (55.9%).

**Example 2. First Synthesis of** 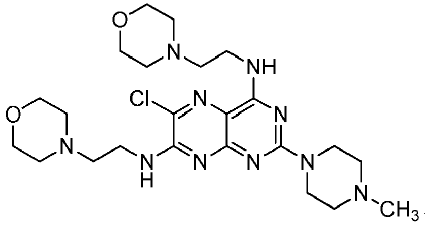 **using Synthetic Route 2**

### Step 1. Alkylation, nitrosation and reduction as a one pot reaction to prepare 5,6-diamino-4-hydroxy-2-methylthiopyrimidine

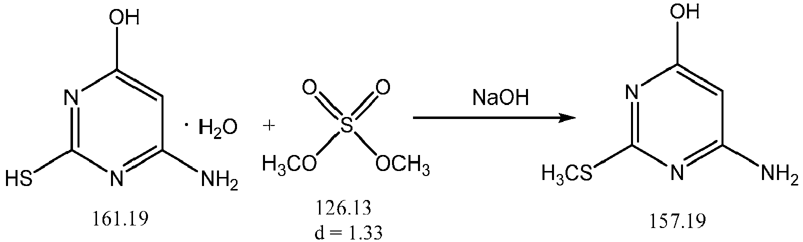

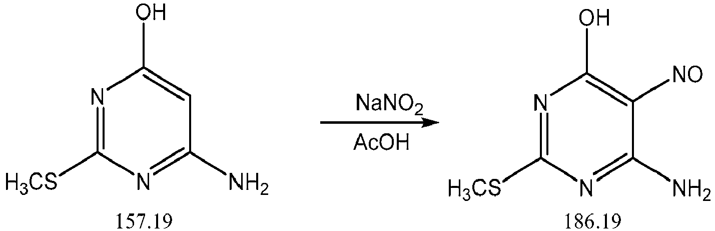

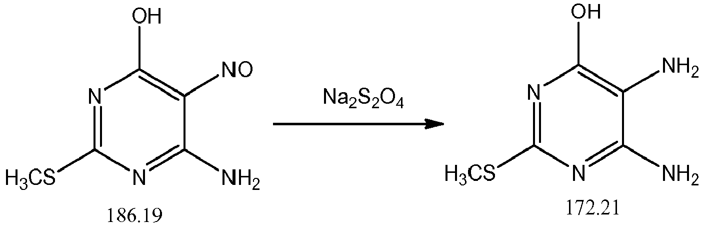

To a solution of sodium hydroxide (30 gm, 0.75 moles) in water (280 mL) was added 4-amino-6-hydroxy-2-mercaptopyrimidine monohydrate (60 gm, 0.37 moles). Once the pyrimidine had dissolved, the solution was stirred on ice and cooled to <10°C. Dimethyl sulfate (32.6 gm, 24.5 mL, 0.258 moles) was added through a dropping funnel over the course of 1 hour keeping the temperature below 10°C. Once the addition was complete, the reaction was slowly warmed to 90°C and was kept at this temperature overnight. A TLC was run in 25% methanol in chloroform to follow the reaction. Addition of more dimethyl sulfate quickly formed the desired 2-methylthio-4-amino-6-hydroxypyrimidine but also caused the formation of more 2-methylthio-4-methoxy-6-aminopyrimidine. Using less than a mole of dimethyl sulfate and heating the reaction to make use of the sodium methylsulfate reduced the byproduct formation. This reaction is slow and therefore a prolonged heating period may be required. To run the TLC, a few drops of the reaction mixture were added to 1.0 mL of methanol and a drop of acetic acid. This solution was spotted on the silica plate, dried and run in 25% methanol in chloroform. 4-amino-6-hydroxy-2-mercaptopyrimidine, Rf = 0.26, 2-methylthio-4-hydroxy-6-aminopyrimidine, Rf = 0.51, 2-methylthio-4-methoxy-6-aminopyrimidine, Rf = 0.66.

Once cooled to room temperature, some solid had separated. The reaction was filtered and the solid was stirred in a solution of sodium hydroxide (10 gm, 0.25 moles) in water (100 mL). A small amount of solid which remained was removed by filtration and the combined aqueous filtrates were used for nitrosation without isolation of the 2-methylthio-4-amino-6-hydroxypyrimidine.

The solution of 4-amino-6-hydroxy-2-methylthio-pyrimidine (0.37 moles), from above, was stirred as a solution of sodium nitrite (29.2 gm, 0.42 moles) in water (100 mL) was added. This solution was cooled on ice to 15°C and stirred as acetic acid (65 gm, 0.1.08 moles) was slowly added through a dropping funnel keeping the temperature below 20°C. A white solid immediately began to separate but as the acetic acid addition continued, the suspension became thicker and turned blue in color. Once all of the acetic acid had been added, the blue suspension was stirred at room temperature overnight. TLC, run in 25% methanol in chloroform on silica plates with sample preparation as described in note 1, showed the nitrosation reaction was slow after the acetic acid addition and stirring overnight was performed to allow the reaction to go to completion. To this suspension was added sodium hydroxide (59.8 gm, 1.5 moles) in water (200 mL). The slurry changed from blue to pink. The slurry was heated to 45°C. To this warm slurry was added sodium dithionite (165 gm, 0.95 moles) over a 30 minute period keeping the temperature below 55°C using cooling as necessary. Once all of the sodium dithionite had been added, a pale yellow suspension had formed. This was heated to 70°C for 30 minutes before being allowed to cool to room temperature overnight. The resulting slurry was filtered and the solid was washed with water. After drying, the product, 2-methylthio-4,5-diamino-6-hydroxypyrimidine was obtained as a tan solid (Purity by HPLC = 91%, MS, Mw = 173) in a yield of 34.5 gm, 54.1%. This material was recrystallized from boiling water as yellow needles.

### Step 1a. Alternative method to prepare 5,6-diamino-4-hydroxy-2-methylthiopyrimidine

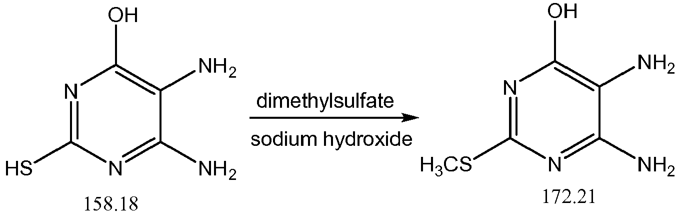

To a solution of sodium hydroxide (25.3 gm, 0.63 moles) in water (250 mL) was added 5,6-diamino-4-hydroxy-2-mercaptopyrimidine (50 gm, 0.32 moles, Aldrich #D17807). Once the pyrimidine had dissolved, the solution was stirred on ice and cooled to <10°C. Dimethyl sulfate (27.9 gm, 20.9 mL, 0.22 moles) was added through a dropping funnel over the course of 1 hour, keeping the temperature below 10°C. Once the addition was complete, the reaction was warmed to room temperature and then slowly warmed to 90°C and was kept at this temperature for 15 hours. Once cooled to room temperature, the resulting slurry was treated with acetic acid (20 mL) to a pH 0f 5. The reaction slurry was heated to boiling and filtered to remove a small amount of insoluble solid. The hot filtrates were cooled on ice and the solid which crystallized was isolated by filtration, washed with water and dried. The yield of 5,6-diamino-4-hydroxy-2-methylthiopyrimidine was 29.5 gm, 54.2%.

### Step 2: Pteridine formation

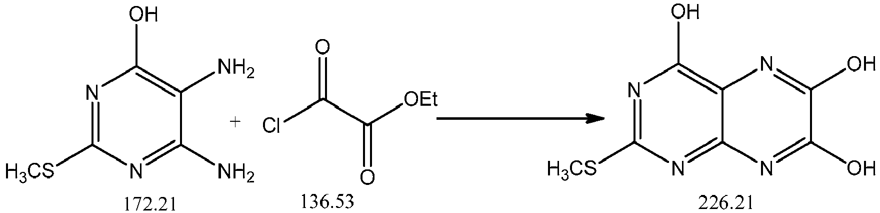

A mixture of powdered 2-methylthio-4-hydroxy-5,6-diaminopyrimidine (48.2 gm, 2.80 X 10⁻¹ moles) and N-methylpyrrolidinone (150 mL) was heated at 65°C until the pyrimidine had dissolved. The solution was stirred in a cold water bath and when the temperature had reached 40°C, pyridine (26.4 gm, 27 mL, 3.34 X 10⁻¹ moles) was added and then ethyl oxalylchloride (45.6 gm, 37.2 mL, 3.34 X 10⁻¹ moles) was slowly added through an addition funnel. (When cooled to 45°C, some solid separated but this quickly dissolved upon addition of the pyridine and some of the ethyl oxalylchloride.) The temperature was kept between 40°C and 50°C by rate of addition and through the use of the cooling bath. After the addition was complete, the reaction was stirred at room temperature for one hour. TLC (silica, 25% methanol / CHCl3) showed loss of the diamine (Rf = 0.35) with the formation of a single product (Rf = 0.69). The solution was brought to 115°C in an oil bath. After about 2 hours, solid began to separate. The reaction was held at 115°C bath temperature (108°C pot temperature) for 4 hours. TLC (silica, 25% methanol / CHCl3) showed complete loss of the compound at Rf = 0.69 with formation of a new product at Rf = 0.24. After the heating period, the reaction was stirred at room temperature overnight. The stirring overnight at room temperature was not necessary and external cooling to 10°C for 30 minutes was sufficient.

The solid was isolated by filtration and was washed with NMP and then acetone (200 mL) before being dried. The yield of 2-methylthio-4,6,7-trihydroxypteridine was 46.6 gm, (73.6%). Purity by HPLC = 88%. MS, Mw = 225.

### Step 3: Formation of JB6136

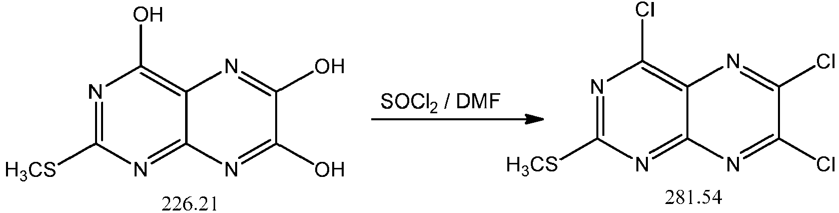

A mixture of 2-methylthio-4,6,7-trihydroxypteridine (15.7 gm, 6.94 X 10⁻² moles), thionyl chloride (75 mL) and dimethyl formamide (4.8 mL) was stirred at room temperature for 30 minutes. This slurry was heated to reflux. After 2 hours a dark yellow solution had formed. Heating was continued for an additional 4 hours. Some of the thionyl chloride (20 mL) was removed under reduced pressure at 50°C before solid separated. To the remaining mixture, chloroform (100 mL) was added and this was stirred until a solution had formed. The solution was then poured onto ice and water and this mixture was stirred to decompose the remaining thionyl chloride. The layers were separated and the chloroform solution was washed with cold water (100 mL) followed by saturated sodium hydrogen carbonate. Then the solution was dried over magnesium sulfate before being filtered to remove the drying agent. The filtrates were used without further purification for the next step.

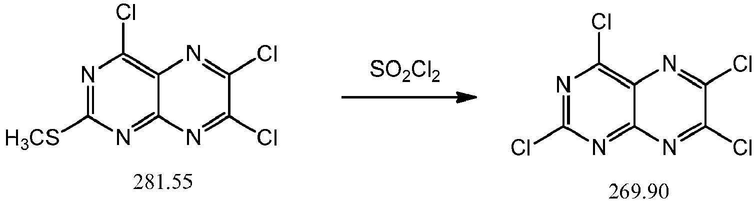

The solution of 2-methylthio-4,6,7-trichloropteridine in chloroform from above was cooled on ice and stirred as sulfuryl chloride (16.4 gm, 9.8 mL , 1.22 X 10⁻¹ moles) was slowly added through a dropping funnel. The solution was then stirred at room temperature overnight. The volatiles were evaporated under reduced pressure to a volume of 20 mL. To the remaining material was added heptane (100 mL) which caused solid to separate. The slurry was concentrated to a volume of 25 mL under reduced pressure. Additional heptane (100 mL) was added and the slurry was again concentrated to a volume of 25 mL under reduced pressure. The remaining heptane was decanted from the solid which was washed with a small amount of heptane. The solid was dissolved in chloroform (100 mL) and this solution, containing 2,4,6,7-tetrachloropteridine was used for the next step without purification.

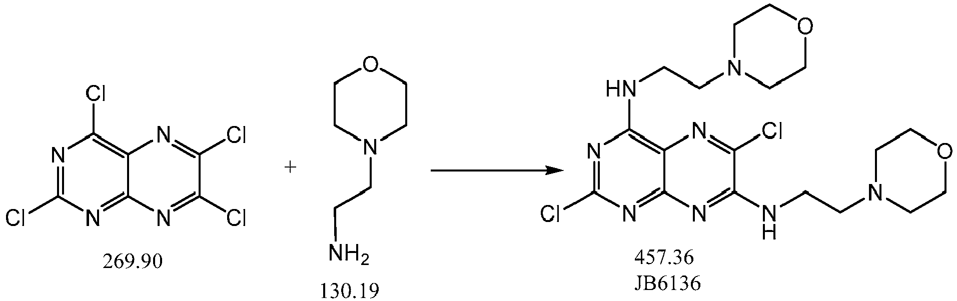

The tetrachloropteridine solution from above was stirred and cooled on ice. To this was added a solution of N-(2-aminoethyl)morpholine (18.1 gm, 0.139 moles) dissolved in chloroform (50 mL) through a dropping funnel with stirring. The resulting solution was stirred overnight at room temperature. The solution was washed with 10% potassium carbonate (100 mL) and then with water (100 mL). After drying over magnesium sulfate, the chloroform solution was filtered and concentrated under reduced pressure to a volume of about 25 mL. To the remaining solution was added ethyl acetate (100 mL). This solution was concentrated under reduced pressure to about 25 mL. Again, ethyl acetate (100 mL) was added and the solution was concentrated under reduced pressure to about 50 mL. This concentrate was stirred at room temperature causing the dichloropteridine to crystallize. The compound is slow to crystallize, and stirring at room temperature overnight is helpful in maximizing yield. The solid was isolated by filtration, washed with cold ethyl acetate and dried to provide JB6136 (This material can be recrystallized from ethyl acetate) as a tan powder in the amount of 13.0 gm (41% from the 2-methylthio-4,6,7-trihydroxypteridine). Purity by HPLC = 99.4%. MS, Mw = 457.

### Step 4: Preparation of JB6121 tri-hydrochloride

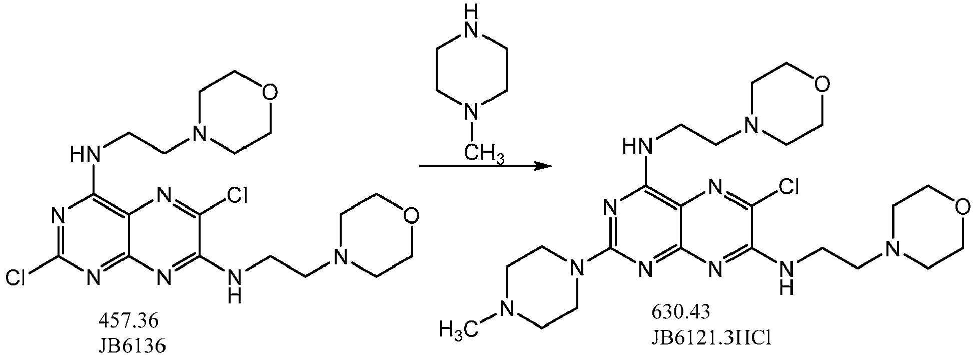

A mixture of the dichloropteridine (34.7 gm, 7.58 X 10⁻² moles) and N-methylpiperazine (9.1 gm, 10.1 mL, 9.1 X 10⁻² moles) in methanol (375 mL) was brought to reflux which provided an orange solution. After refluxing for 15 hours, TLC (silica, 15% methanol in chloroform) showed loss of starting material (Rf = 0.45) and formation of a blue fluorescent compound (Rf = 0.15). The solution was cooled to 85°C and concentrated hydrochloric acid (31.4 mL, 3.6 X 10⁻¹ moles) was added. Upon cooling to room temperature, the hydrochloride salt crystallized. After cooling on ice, the solid was isolated by filtration, washed with cold methanol (100 mL), followed by diethyl ether (100 mL) and then dried under vacuum at 45°C. The yield was 32.5 gm (68.0% overall from the dichloropteridine) as a white solid. Evaporation of the filtrates gave an aqueous oil which was stirred with 2-propanol (200 mL). After sitting overnight, a semi-solid mass had formed. The 2-propanol was decanted from the mass which was dissolved in boiling methanol (200 mL). Upon cooling in the refrigerator overnight a second crop of JB6121 hydrochloride was obtained in a yield of 6.0 gm (12.6%). The use of anhydrous HCl in methanol may increase the yield of the initial crop of JB6121 hydrochloride. Purity by HPLC = 99.5%. MS, Mw = 521. TLC: (silica, 25% methanol in methylene chloride) - one spot, Rf = 0.65.

**Example 3. Second Synthesis of** 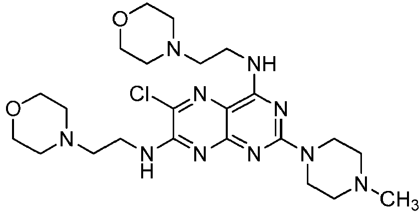 **using Synthetic Route 2**

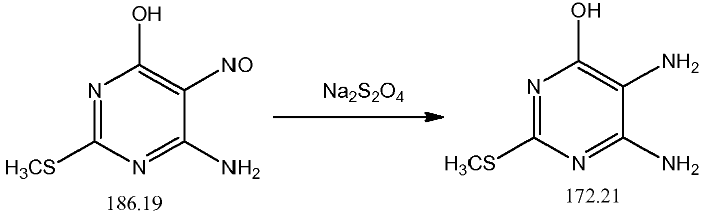

Powdered 2-methylthio-4-hydroxy-5-nitroso-6-aminopyrimidine (12.6 gm, 6.77 X 10⁻² moles) was stirred in a solution of sodium hydroxide (8.1 gm, 2.03 X 10⁻² moles) in water (150 mL). This blue suspension was stirred as sodium dithionite (25 gm) was added in portions over 30 minutes. After the addition was complete, the blue color had faded and a white suspension had formed. After stirring overnight at room temperature, the solid was isolated by filtration, washed with water and dried. The yield of product was 11.4 gm, (98%).

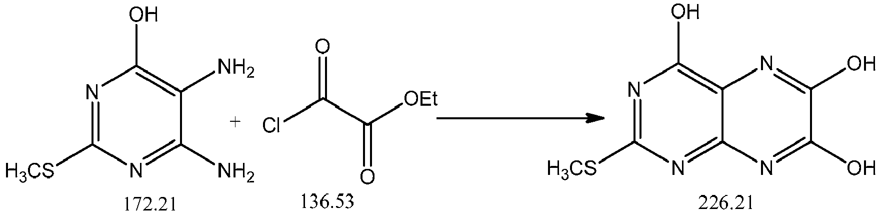

A mixture of 2-methylthio-4-hydroxy-5,6-diaminopyrimidine (31.4 gm, 0.183 moles) and pyridine (500 mL) was heated until the pyrimidine had dissolved. The solution was stirred in a cold water bath and when the temperature had reached 40°C. ethyl oxalylchloride (34.9 gm, 28.5 mL, 0.256 moles) was slowly added through an addition funnel. After the addition was complete, the reaction was stirred at room temperature for one hour. TLC (silica, 25% methanol / CHCl3) showed complete loss of the diamine (Rf = 0.35) with the formation of a single product (Rf = 0.69). The solution was brought to reflux. After about 15 minutes, solid began to separate. The reaction was held at reflux for 2 hours. TLC (silica, 25% methanol / CHCl3) showed complete loss of the compound at Rf = 0.69 with formation of a new product at Rf = 0.24. After cooling to room temperature, the solid was isolated by filtration and was washed with acetone before being dried. The crude solid, which contained pyridine hydrochloride, was dissolved in water (300 mL) containing sodium hydroxide (15 gm, 0.375 moles). To this solution was added concentrated hydrochloric acid (60 mL) to a pH of 3. The precipitated solid was isolated by filtration, washed with water and dried. The yield of the pteridine was 27.5 gm, (66.4%)

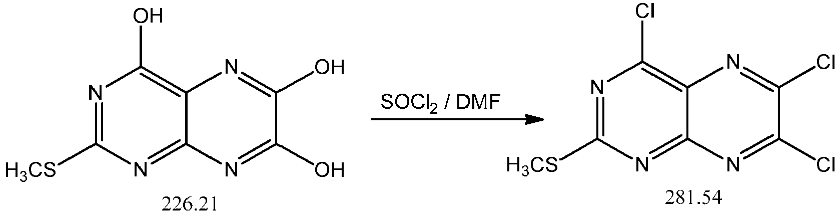

A mixture of 2-methylthio-4,6,7-trihydroxypteridine (2.0 gm, 8.84 X 10⁻³ moles), thionyl chloride (20 mL) and dimethyl formamide (4 mL) was stirred overnight at room temperature. The resulting slurry was heated to 75°C. for 4 hours. The resulting yellow solution was cooled and excess thionyl chloride was removed under reduced pressure. The remaining solid was dissolved in methylene chloride and the solution was stirred as ice was added. The cooled (ice) mixture was stirred as sodium bicarbonate was added until the pH of the aqueous was 7 - 8. The methylene chloride was dried over magnesium sulfate before being filtered and evaporated under reduced pressure. The remaining material was stirred in diethyl ether which caused the precipitation of a small amount of dark material. This was removed by filtration and the ether was evaporated under reduced pressure to provide the 2-methylthio-4,6,7-trichloropteridine as a bright yellow solid in a yield of 2.12 gm, (85%).

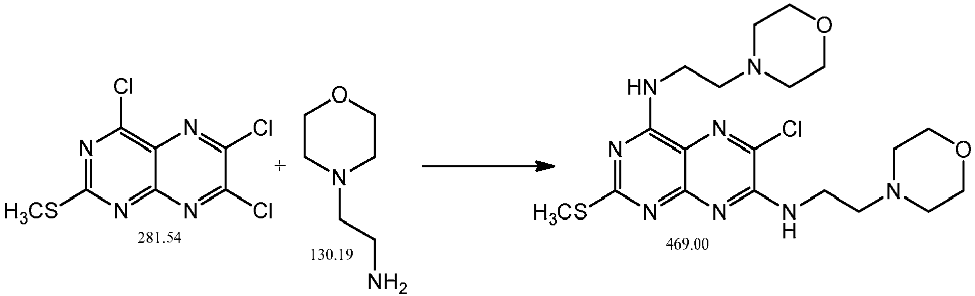

A solution of 2-methylthio-4,6,7-trichloropteridine (2.12.gm, 7.53 X 10⁻³ moles) in methylene chloride (50 mL) was stirred as N-(2-aminoethyl)morpholine (3.45 gm, 2.65 X 10⁻² moles) was added. The resulting solution was stirred overnight at room temperature. The reaction solution was diluted with methylene chloride (50 mL) and was washed with 5% potassium carbonate solution (50 mL). The methylene chloride solution was dried over magnesium sulfate before being filtered and evaporated under reduced pressure. The remaining oil quickly solidified upon cooling. This solid was recrystallized from 2-propanol (50 mL) to provide the product in a yield of 2.4 gm (69%).

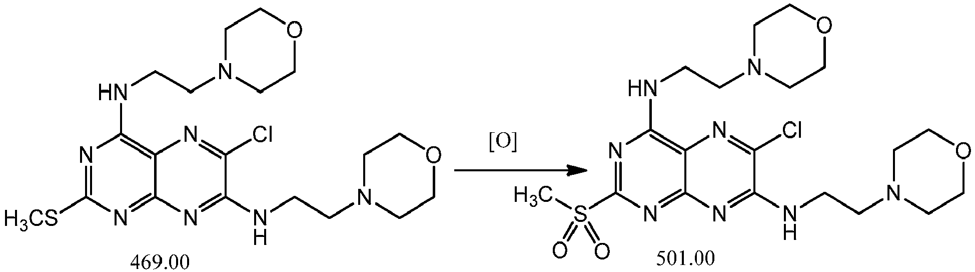

The methylthiopyrimidine (3.75 gm, 8.0 X 10⁻³ moles) was dissolved in acetic acid (4.6 mL) and was cooled on ice. To this was added hydrogen peroxide (30%, 3.4 mL) and sodium tungstate dihydrate. (792 mg, 2.40 X 10⁻³ moles). This solution was stirred on ice for 2 hours at which point more acetic acid (4.6 mL) and hydrogen peroxide (30%, 3.4 mL) were added. The resulting solution was kept in the freezer overnight. The reaction was diluted in cold water and neutralized by the careful addition of potassium carbonate. Once neutralized, the product sulfone was extracted into methylene chloride (2 X 100 mL). The organic solution was dried over magnesium sulfate before being filtered and evaporated under reduced pressure. The sulfone was used for the next step without further purification.

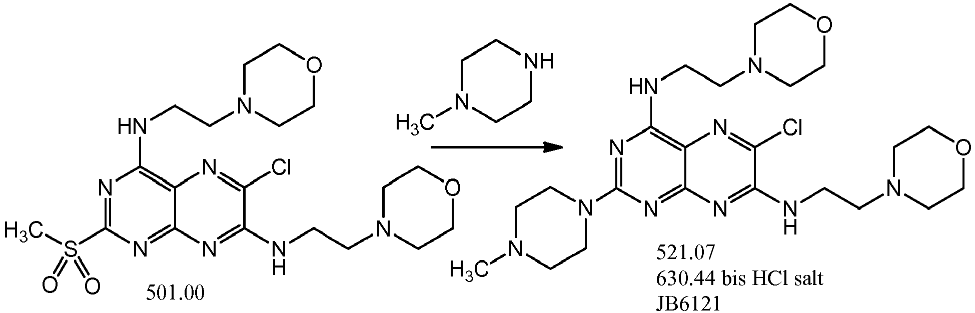

The pteridine sulfone (677 mg, 1.35 X 10⁻³ moles) and N-methylpiperazine (203 mg, 225 µL, 2.0 X 10⁻³ moles) were combined in n-butanol (10 mL). This was heated at reflux for 10 hours. TLC (silica, 15% methanol in methylene chloride) showed loss of the sulfone with a major product consistent with JB6121 along with several minor products. After cooling, the reaction was partitioned between methylene chloride (50 mL) and 5% potassium carbonate. The methylene chloride solution was dried over magnesium sulfate before being filtered and evaporated under reduced pressure. The remaining material was dissolved in boiling ethanol (10 mL) and concentrated hydrochloric acid (382 µL) was added. Upon cooling a solid separated which was isolated by filtration. After being washed with ethanol, the solid was dried to give JB6121 tris hydrochloride in a yield of 400 mg, (47%).

### Example 4. hERG patch clamp assay and in vitro IC₅₀s of pteridine analogs against TLRs. hERG (automated patch-clamp)

The hERG inhibition assay uses a high throughput single cell planar patch clamp approach. Chinese hamster ovary cells transfected with the hERG gene (CHO-hERG) are dispensed into the PatchPlate. Amphotericin is used as a perforating agent to gain electrical access to the cells. The hERG tail current is measured prior to the addition of the test compound by perforated patch clamping. Following addition of the test compound at a defined concentration or range of concentrations a second recording of the hERG current is performed. The degree of inhibition (%) is obtained by measuring the tail current amplitude, which is induced by a one second test pulse to - 40 mV after a two second pulse to + 20 mV, before and after drug incubation (the difference current is normalized to control and multiplied by 100 to obtain the percent of inhibition).

Concentration (log) response curves were fitted to a logistic equation (three parameters assuming complete block of the current at very high test compound concentrations) to generate estimates of the 50 % inhibitory concentration (IC50). The concentration-response relationship of each compound is constructed from the percentage reductions of current The data is usually categorized into the following classification bands:

| | |
|---|---|
| Highly potent | IC₅₀ < 0.1µM |
| Potent | IC₅₀ between 0.1 and 1µM |
| Moderately potent | IC₅₀ between 1µM and 10µM |
| Weak or no inhibition | IC₅₀>10µM |

TLR7, -8 and -9 are members of a subset of the TLR family of transmembrane proteins that recognize microbial-associated nucleic acid motifs to stimulate the production of transcriptions factors, such as NF-κB, and inflammatory cytokines. TLR7, expressed by plasmacytoid dendritic cells (pDCs), is activated by ssRNA oligos to produce, among other things, the type-1 interferon, IFNα. Similarly, TLR9 is also expressed by pDCs, as well as B-cells, and induces expression of IFNα, but unlike TLR7, TLR9 is stimulated by CpG-DNA. TLR8, while activated by ssRNA, differs from TLR7 in both localization and down-stream signaling; TLR8 is localized to myeloid dendritic cells (mDCs) and leads to the production of inflammatory cytokines, such as TNFα. By tailoring the stimulus / readout to the TLR of interest, we are able to measure the activity of small molecule antagonists against either TLR7, - 8, or -9 in primary human peripheral blood mononuclear cells (PBMCs).

Toll-like Receptors (TLR) are a family of transmembrane proteins, related to the *Drosophila* protein Toll, and function as key mediators of the innate immune response through the recognition of pathogen-associated microbial patterns (PAMPs). Recognition of PAMPs, such as lipopolysaccharides (LPS), microbial DNA, or RNA, by TLRs activates the Toll/Interlukin-1 receptor (IL-1R) signaling cascade, via MyD88, leading to the production of transcription factors, including NF-κB, which subsequently causes the transcription / translation of immune response genes, such as Interleukin (IL)-6, IL-8, type 1 interferon (IFN), and tumor necrosis factor (TNF). While there is some redundancy in the function and activation of TLRs, individual TLRs can be distinguished by their unique expression patterns, as well as the specific PAMPs that they recognize.

TLR7, -8, -9, along with TLR3, constitute a subfamily of TLRs that, while being structurally similar to TLR1-6, are localized within the endosome, rather than on the cell surface, and are found primarily in immune-rich tissues, such as spleen and peripheral blood cells. The TLRs in this subgroup respond to pathogen associated oligonucleotides, either DNA, or RNA, and can be distinguished based on the specific nucleic acid motif recognized, expression pattern, and downstream effector signaling. Both human TLR7 and -9 are expressed in plasmacytoid dendritic cells (pDC), while TLR8 is found in myeloid dendritic cells (mDC). TLR9 is also expressed by peripheral B cells. Neutrophils, the most abundant immune cells in human blood, have been shown to express all human TLRs, except TLR3. Activation of either TLR7, or TLR9, by single-stranded RNA oligonucleotides, or deoxycytidylate-phosphate-deoxyguanylate (CpG) DNA, respectively, leads to the production of IFNα. Similar to TLR7, TLR8 is also activated by single-stranded RNA leading to the activation of Jun-N Terminal kinase (JNK) signaling and the production of TNFα.

Previous research has suggested that anti-malarial drugs, specifically quinolines, can inhibit TLR7, -8, and -9 activity in human peripheral blood mononuclear cells (PBMCs). To determine the efficacy of our small molecule, antagonist compounds against TLR7, -8, and -9, we developed *in vitro* assays to screen for antagonist activity in PBMCs. By taking advantage of the distinct TLR signaling pathways, we are able to determine the relative activity of our antagonists both in relation to other small-molecule antagonists and against TLR7, -8, or -9, explicitly.

PBMCs were isolated from unpurified buffy coat from healthy human donors (Blood Research Components, LLC). For antagonist testing, cells were plated on 96-well U-bottom plates at 3x10⁶ cells/ml. Antagonist compounds were added to cells in duplicate wells and titrated from 10µM in 1:3 serial dilutions (8 antagonist concentrations tested in total). After approximately 30 minutes, cells were stimulated with agonist compound (see table2 for agonist concentrations). After stimulation, cells were incubated overnight at 37°C. To increase the stability of the ssRNA, 3µg/ml DOTAP (Roche) was added to ORN1075 agonist solutions. All dilutions were made in RPMI-1640 media (Sigma, cat.# R7388) + 5% human male AB sera (Sigma, cat.# H4522) + 1X Penicillin / Streptomycin solution (VWR, cat.# 82026-730).

**Table 2: Summary of TLR agonists for human PBMC assays. Summary of agonists used to stimulate TLR7, -8, or -9 activity for antagonists testing in primary human cells.**

| **Cell Type** | **TLR** | **Agonist** | **Concentration** |
|---|---|---|---|
| **PBMC** | **TLR7** | **ssRNA (ORN1075)** | **0.5µM** |
| **PBMC** | **TLR8** | **ssRNA (ORN1075)** | **0.5µM** |
| **PBMC** | **TLR9** | **CpG-dsDNA (ODN2395)** | **0.25uM** |

### ELISAs

TLR activity was measured in PBMCs and neutrophils by ELISA using cytokine readouts specific for the TLR of interest.

### TNFα ELISA.

TNFα was used as a readout for TLR8 activity in PBMCs and neutrophils stimulated with ORN1075 (See Table 3 for concentrations) using the ELISA Max™ Deluxe Set Human TNFα kit (BioLegend, cat.#430206). Supernatant from PBMCs was diluted 1:50 and from neutrophils at 1:3 in IX Assay Diluent (BioLegend). ELISAs were run according to the kit product specifications. Absorbance at 450nM was recorded using a Molecular Devices SpectraMax340PC plate reader. The IC₅₀ for each antagonist was subsequently calculated following the method described below.

**Table 3: Summary table ELISAs used to test each TLR-agonist combination.***

| **Cell Type** | **TLR** | **Agonist** | **Activity Readout (for ELISA)** | **Analyte Dilution** |
|---|---|---|---|---|
| **PBMC** | **TLR7** | **ssRNA (ORN1075)** | **IFNα** | **1:3** |
| **PBMC** | **TLR8** | **ssRNA (ORN1075)** | **TNFα** | **1:50** |
| **PBMC** | **TLR9** | **CpG-dsDNA (ODN2395)** | **IFNα** | **1:3** |

| | | | | |
|---|---|---|---|---|
| *TLR7 and TLR8 are activated by single-stranded RNA, signaling the production of Type I Interferons or Tumor Necrosis Factor, respectively. TLR9 is activated by unmethylated, double-stranded CpG-DNA motifs and results in the production of Type I Interferons, such as IFNα. | | | | |

### IFNα ELISA

IFNα was measured as a readout for TLR7 and TLR9 activity in PBMCs stimulated with either ORN1075, or ODN2395, respectively, using a Human Pan-IFNα ELISA kit (MABTech, cat.#3425-1H-20). PBMC supernatant was diluted 1:3 in PBS + 0.05%tween-20 + 0.1%BSA. Absorbance at 450nM was recorded using a Molecular Devices SpectraMax340PC plate reader. The IC₅₀ for each antagonist was subsequently calculated following the method described below.

### Calculating the IC₅₀ of Antagonists from ELISA Datasets and Biological Profile Comparisons

Raw ELISA datasets for each 96-well plate, consisting of absorbance readings at 450nM, were first imported in Microsoft Excel to calculate the average absorbance for each standard or antagonist concentration. The averaged data was then imported into SigmaPlot v.11.0 where a standard curve was calculated and used to extrapolate the relative concentration of TLR readout (TNFα, IFNα, etc.) produced. The dose-response curve of each antagonist was then plotted with SigmaPlot v.11.0 on a Log scale. Concentrations of TLR readout were converted to a percentage relative to the average maximum concentration for that ELISA plate in Microsoft Excel. The IC₅₀ for each antagonist was then determined with the SigmaPlot regression wizard using a 4-parameter Hill equation. Each antagonist was tested in a minimum of 3 independent experiments and IC₅₀ results were compiled and averaged using Microsoft Excel.

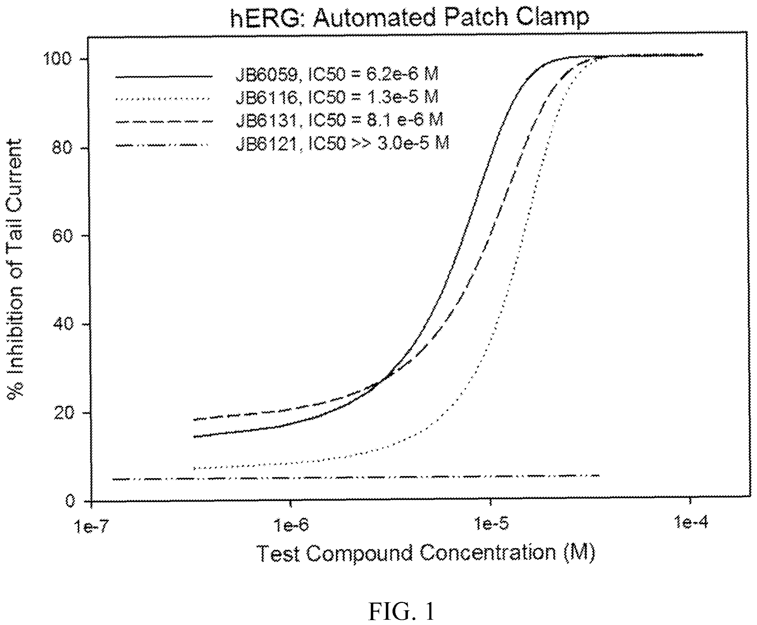

Compounds JB6059, JB6116, JB6131 and 6-chloro-2-(4-methylpiperazin-1-yl)-N⁴,N⁷-bis(2-morpholinoethyl)pteridine-4,7-diamine were tested. 6-chloro-2-(4-methylpiperazin-1-yl)-N⁴,N⁷-bis(2-morpholinoethyl)pteridine-4,7-diamine displayed no measurable hERG inhibition up to 30 µM. However, JB6059, JB6116 and JB6131 displayed and IC₅₀ equal to 6.2 µM, 13.1 µM and 8.1 µM respectively, see FIG. 1.

In TLR inhibition assay JB6121 (6-chloro-2-(4-methylpiperazin-1-yl)-N⁴,N⁷-bis(2-morpholinoethyl)pteridine-4,7-diamine) was shown to be more potent across the sum of TLR9, TLR8 and TLR7. *See,* Tables 4a and Scheme 18.

**Table 4a. IC₅₀s of pteridine compounds against TLRs.**

| Compound ID | TLR9 IC50, nM | TLR8 IC50, nM | TLR7 IC50, nM |
|---|---|---|---|
| JB6059 | 305 | 273 | 143 |
| JB6116 | 297 | 658 | 81 |
| JB6121 | 146 | 514 | 25 |
| JB6131 | 326 | 78 | 64 |
| JB6147 | 8675 | 7300 | 223 |
| JB6140 | 101 | 754 | 238 |
| JB6143 | 54 | 263 | 24 |
| JB6160 | 60 | 219 | 28 |
| JB6172 | 167 | 246 | 62 |
| JB6180 | 114 | 151 | 502 |
| JB6185 | 61 | 136 | 74 |

| Table 4b. hERG and % hepatocyte viability. | | |
|---|---|---|
| Compound ID | hERG IC50, µM | % Hepatocyte viability 24h at 100µM |
| JB6059 | 5 µM | 69% |
| JB6116 | <30 µM | 69% |
| JB6121 | >30 µM | 92% |
| JB6131 | <10 µM | 11% |

| | | % Hepatocyte viability 24h at 50 µM |
|---|---|---|
| JB6140 | <1 µM | 59% |
| JB6143 | >30 µM | 74% |
| JB6160 | >30 µM | 47% |
| JB6172 | 1 µM | 51% |
| JB6180 | 0.3 µM | 13% |
| JB6185 | <0.3 µM | 0% |

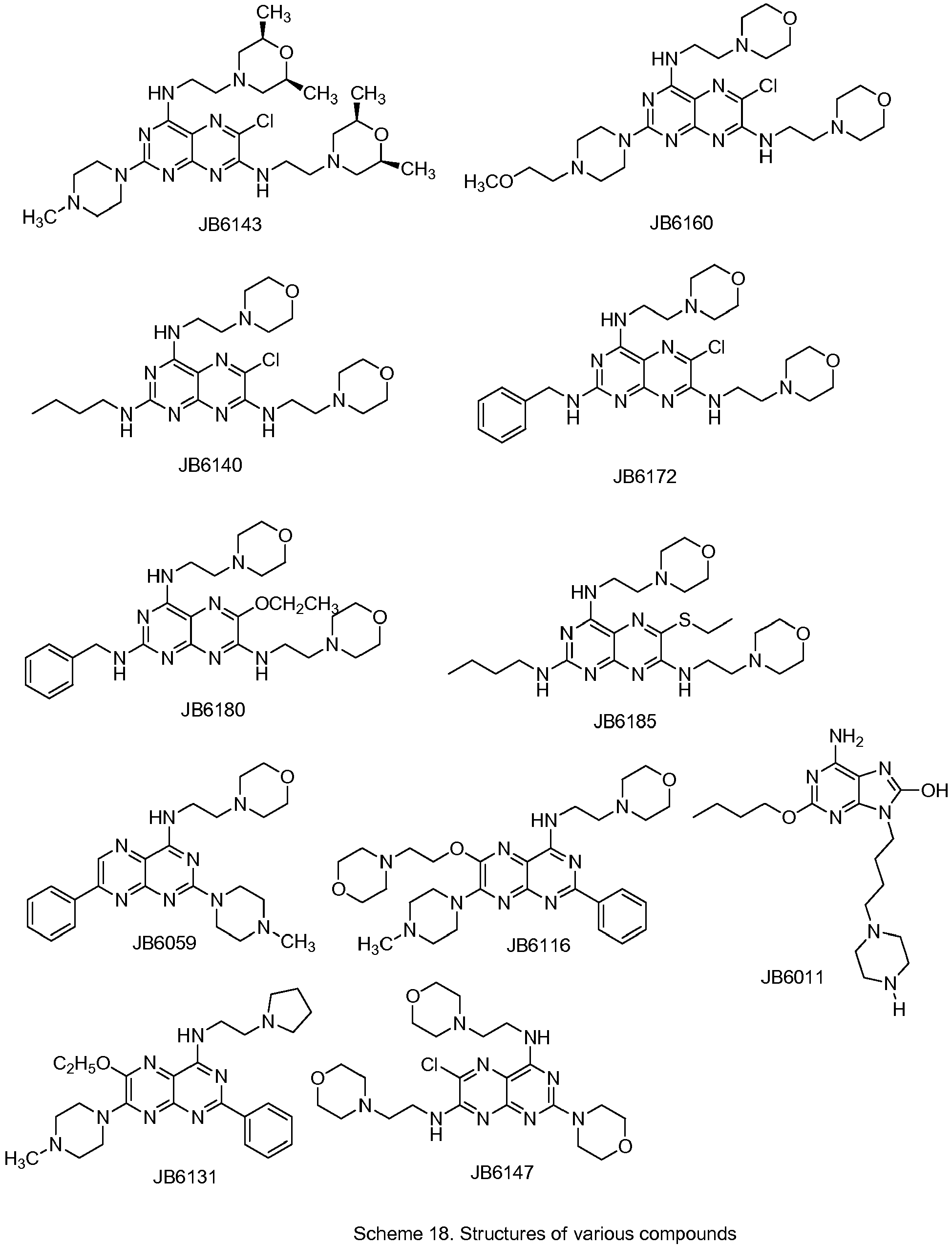

As shown above, it has been surprisingly found that the compound of Formula (I), *e.g.,* JB6121, exhibited surprisingly superior cardio safety profile in a hERG patch clamp assay and superior hepatocyte safety in a hepatocyte viability test, in comparison with other compounds. *See,* Tables 4b and Scheme 18. The hepatocyte cell viability assays were performed by CEREP, Seattle, WA, according to Mosmann, T., 1983, J. Immunol. Met. 65:55-62.

### Example 5. Pharmacological data for 6-chloro-2-(4-methylpiperazin-1-yl)-N4,N7-bis(2-morpholinoethyl)pteridine-4,7-diamine.

### Antagonist/Agonist Competitive Inhibition

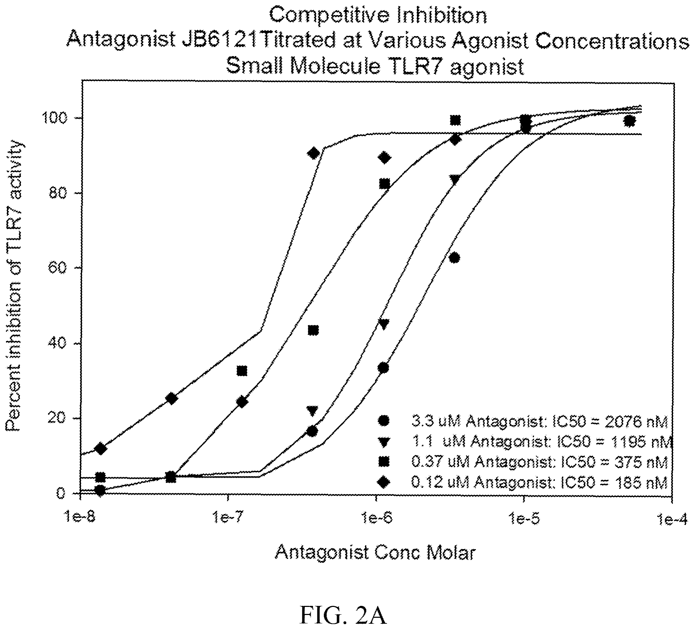

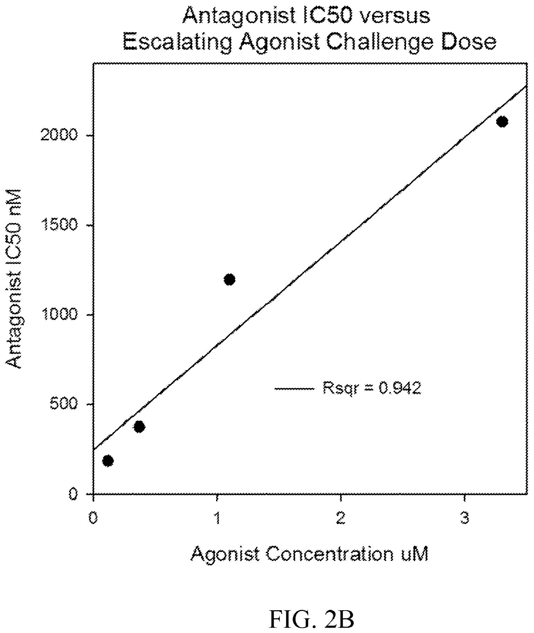

In preliminary results (i.e., one assay result - repeat assays in process), a cross titration of antagonist versus agonist was performed to determine the form of antagonist binding. JB6121 was titrated from 10uM to 4 nM against various concentrations of challenge agonist. The small molecule TLR7 agonist JB6011 was used. The graph below, while crude, implies that the inhibition of the TLR7 agonist by JB6121 is competitive in nature due to the parallel tracking of the curves as the agonist dose is increased. The agonist range for this experiment was between 0.12 and 3.3 µM or about a 30-fold range. These results, while representing only one assay, appear to be supported with various antagonists. A comparable result has been achieved with the antagonist JB6140, a similar antagonist molecule with a higher logD and a higher potency versus JB6011 than JB6121 (See, FIGS. 2A and 2B).

### In vivo pharmacology

JB6121 was tested in vivo for TLR9 knockdown activity. The ID₅₀ for IP injection was determined to be 55.27 µg injection dose and the PO gavage was 59.99 µg delivery dose. This roughly translates to an ID50 of 2.4 mg/kg dose for a 25 gram mice.

Mice were treated with test antagonist at varying doses at T = 0 hr. Agonist treatment (CpG-DNA) dosed one hour later, T = 1 hr for IP groups and two hours later, T = 2 hr for PO groups. Necropsy performed 3 hours post agonist treatment, T = 4 hr (IP groups) or T = 5 hr (PO groups). Serum cytokines induced by the TLR agonist are measured by ELISA. Groups size per dose, n=3. The mean +/- SD of two independent ELISA's at differing serum dilutions is plotted.

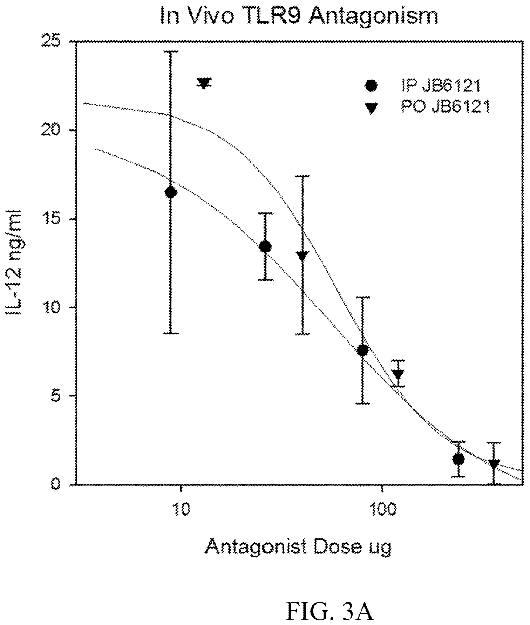

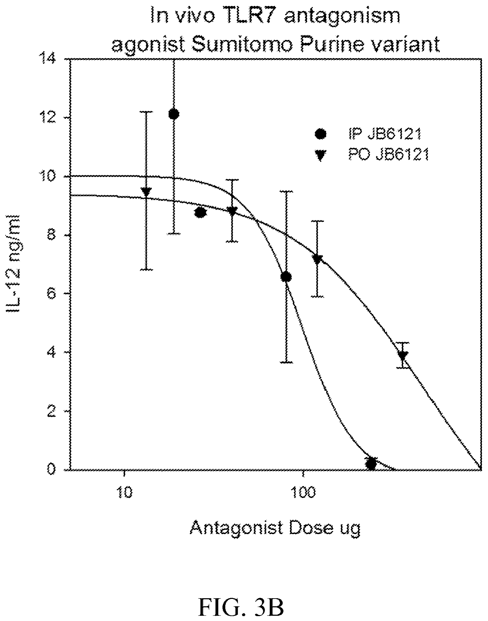

JB6121 was also tested in vivo for TLR7 knockdown activity utilizing the TLR7 small molecule agonist JB6011 as the agonist challenge. The ID50 for IP injection was determined to be 98.34 µg injection dose and the PO gavage was 286 µg delivery dose, see Figures 3A and 3B below. This roughly translates to a 3.92 mg/kg IP dose and 11.44 mg/kg PO dose for a 25 gram mouse.

### CEREP preliminary ADMET data

Preliminary screening measures were conducted by CEREP. All values fall within acceptable limits (Table 5). Solubility assays where performed by CEREP, Seattle, WA, according to Lipinski, C.A. et al., 1997, Adv. Drug Del. Rev., 46:3-26. Protein binding assays where performed by CEREP, Seattle, WA, according to Banker, M.J. et al., 2003, J.Pharm. Sci., 92:967-974. Permeability assays where performed by CEREP, Seattle, WA according to Hildalgo, I.J., 1998, Gastroenterology, 96:736-749. Metabolic stability assays where performed by CEREP, Seattle, WA, according to Obach, R.S. et al., 1997, J. Pharmacol. Exp. Ther., 283:46-58. P-glycoprotein inhibition assays where performed by CEREP, Seattle, WA, according to Polli, J.W. et al., 2001, J. Pharm. Exp. Ther., 299:620-628. Cell viability assays where performed by CEREP, Seattle, WA, according to Mosmann, T., 1983, J. Immunol. Met. 65:55-62. hERG automated patch clamp assays where performed by CEREP, Seattle, WA, according to Mathes, C., 2006, Expert Opin. Ther. Targets, 10:230-241.

**Table 5. in vitro ADMET data for JB6121.**

| | |
|---|---|
| Aqueous Solubility, PBS, pH 7.4, in µM | >200 µM |
| Aqueous Solubility, simulated Gastric Fluid, in µM | >200 µM |
| Aqueous Solubility, simulated Intestinal Fluid, µM | 194.9 µM |
| LogD, pH 7.4 | 0.995 |
| Protein Binding, Human Plasma, % bound | 87% |
| A-B Permeability, CaCo-2 cells, pH 6.5/7.4, 10⁻⁶ cm/sec | 1.2 |
| P-glycoprotein, % inhibition at 100 µM | 1.0% |
| Metabolic Stability, Human Liver Microsomes at 60 min. % remaining | 75% |
| Cell Viability, Hep-G2 cells, % inhibition at 100 µM | 8.5% |
| hERG, automated patch clamp, % inhibition at 10 µM | 11.0% |

### JB6121 Cytochrome p450 inhibition

JB6121 was also tested for CYP inhibition, see Table 6 below. Assays where performed by CEREP, Seattle, WA, according to Atkinson, A et al., 2005, Drug Metab. Dispos. 33:1637-1647.

**Table 6.**

| Cytochrome p450 | Test conc. | Mean % Control |
|---|---|---|
| CYP1A2 | 10 µM | 97.0 |
| CYP2B6 | 10 µM | 58.2 |
| CYP2C8 | 10 µM | 87.7 |
| CYP2C9 | 10 µM | 46.7 |
| CYP2C19 | 10 µM | 92.9 |
| CYP2D6 | 10 µM | 80.9 |
| CYP2E1 | 10 µM | NT |
| CYP3A4, BFC substrate | 10 µM | 94.1 |
| CYP3A4, BzRes substrate | 10 µM | 104.8 |

### JB6121 Receptor Cross-reactivity

The receptor cross-reactivity profile is composed of 71 binding and 27 enzyme assays. The binding assay panel is broadly defined with roughly an equal number of selective, central and peripheral therapeutically relevant targets. The enzyme assay panel includes the most representative targets from diverse enzyme families with a particular focus on phosphatases and specific enzymes involved in cell cycle regulation. Assays where performed by CEREP, Seattle, WA, with protocols similar to Cordeaux, Y. et al., 2004 Brit. J. Pharmacol., 143:705-714. JB6121 was tested for receptor cross reactivity at 10 µM for approximately 100 receptors and enzymes. Any % inhibition greater than 50% (IC50< 10 µM) is highlighted below in Table 7.

**Table 7**

| Receptor/Enzyme | Tested Conc. | % Ligand Inhibition |
|---|---|---|
| M (non-selective) Muscarinic Receptor | 10 µM | 63.1 |
| COMT (catechol-O-methyl transferase) | 10 µM | 53.5 |

The above data implies that JB6121 may be able to block radio-ligand binding to the M non-selective muscarinic receptor and COMT. For the non-selective muscarinic receptor, the activity was most-likely IC50 > 5.0 µM and for COMT very close to 10 µM.

### JB6121 Pharmacokinetics

A pharmacokinetic study was performed by Charles River Wilmington MA, in rats and the doses where 2 mg/kg IV and 10 mg/kg PO and whole blood levels were measured for 72h. The calculated pharmacokinetic parameters for 24h are given below in Table 8.

Six (6) male Sprague-Dawley rats were selected from the Testing Facility's rodent colony. The animals were enrolled in the study based on acceptable health as determined by a Testing Facility veterinarian and based on patency of femoral and jugular vein catheters. Fasting was performed for at least 16 hours prior to dosing. Food was also withheld until after the 4 hour time point collection. The animals were placed into 2 groups of 3 animals per group. Each animal in Group 1 received prepared JB6121 by intravenous dose administration at a target dose level of 2 mg/kg and at a dose volume of 1 mL/kg. Immediately following intravenous dosing, the inter-dwelling catheter was flushed with 0.5 mL of saline. Each animal in Group 2 received prepared JB6121 by oral gavage dose administration at a target dose level of 10 mg/kg and at a dose volume of 10 mL/kg. Throughout dosing and at all sample collection time points, the animals were observed for any clinically relevant abnormalities; no abnormalities were noted.

Whole blood samples (0.300 mL; K₂EDTA anticoagulant) were collected from each animal through a jugular vein catheter. Whole blood samples were collected from all animals at 30 minutes and at 1, 2, 4, 6, 12, 24, 48, and 72 hours after dose administration.

Whole blood samples were transferred to the Charle River Bioanalytical Sciences Laboratory, Wilmington MA, for analysis. Pharmacokinetic parameters were estimated using Watson pharmacokinetic software (Version No. 7.2) using a non-compartmental approach consistent with the IV or PO route of administration.

**Table 8.**

| Route | IV | Route | PO |
|---|---|---|---|
| Dose mg/kg | 2.0 | Dose mg/kg | 10.0 |
| Subject | Rat | Subject | Rat |
| T1/2 Hour | 9.71 | Bioavailability % | 20.6 |
| CL ml/min/kg | 16.4 | Tmax hours | 3.33 |
| Vdss ml/kg | 21600 | Cmax ng/ml | 149 |
| AUC h*ng/ml | 2040 | AUC h*ng/ml | 2100 |
| AUC Extrap H*ng/ml | 2230 | AUC Extrap h*ng/ml | 2310 |
| Terminal points | 3 | Terminal points | 3 |

### JB6121 PK/PD

The objective of this study was to collect serum following ODN CpG 1668 administration and to determine the pharmacokinetics of JB6121 following oral administration to Sprague-Dawley rats for 7 days. There was a pre-test to establish the appropriate agonist dose for a TLR9 ligand, CpG DNA ODN 1668, in rats using serum IL-12 as the monitored cytokine, groups 1-3, see table 6. Whole blood samples for serum were collected terminally from each animal 3 hours following dosing with ODN CpG 1668. Serum IL-12 was determined by ELISA, data not shown. An agonist dose of 1mg/kg was selected for the next phase of the study. For groups administered JB6121 (Groups 4-7), formulations were corrected for base/salt components and not corrected for purity. On the initial day of dose administration, JB6121 formulations were prepared using sterile PBS to the adjusted target dosing concentrations using a factor of 0.8265 to correct for salt content. Dosing was performed as outlined in the following Table 9.

**Table 9. Experimental Design**

| Gr^{a} | No. of Males | Test Article(s) | Dose (mg/kg) | Adjusted^{b} Dose Conc. (mg/mL) | Dose Volume (mL/kg) | Vehicle | Route | Dosing Regimen |
|---|---|---|---|---|---|---|---|---|
| 1 | 3 | ODN CpG 1668 | 0.11 | 0.11 | 1 | PBS | IP | Once on Day 1 |
| 2 | 3 | ODN CpG 1668 | 0.33 | 0.33 | 1 | PBS | | |
| 3 | 3 | ODN CpG 1668 | 1 | 1 | 1 | PBS | | |
| 4 | 5 | JB6121 | 0.39 | 0.047 | 10 | PBS | PO | PO once daily for 7 days IP two hours following PO dose on Day 7 |
| | | ODN CpG 1668 | 1 | 1 | 1 | PBS | IP | |
| 5 | 5 | JB6121 | 1.56 | 0.19 | 10 | PBS | PO | |
| | | ODN CpG 1668 | 1 | 1 | 1 | PBS | IP | |
| 6 | 5 | JB6121 | 6.25 | 0.76 | 10 | PBS | PO | |
| | | ODN CpG 1668 | 1 | 1 | 1 | PBS | IP | |
| 7 | 5 | JB6121 | 25 | 3. 03 | 10 | PBS | PO | |
| | | ODN CpG 1668 | 1 | 1 | 1 | PBS | IP | |

| | | | | | | | | |
|---|---|---|---|---|---|---|---|---|
| IP = Intraperitoneal, PO = Oral Gavage ^{a} Groups 1-3 were dosed and collected serum samples analyzed prior to dose administration for Groups 4-7. These serum data were used to assign doses (and concentrations) for ODN CpG 1668 formulations (Groups 4-7). ^{b} Adjustment applied to JB6121 formulations ONLY. Dose concentrations were adjusted by a factor of 0.8265 to correct for salt content. | | | | | | | | |

Animals were assigned to four groups (groups 4-7) and administered JB6121 at 0.39, 1.56, 6.25, or 25 mg/kg in a volume of 10 mg/mL once per day by oral gavage for 7 days. On Day 7, two hours following the JB6121 dose (approximate Cmax), ODN CpG 1668 was administered as a single IP dose at 1 mg/kg in a volume of 1 mL/kg. Whole blood samples for bioanalysis and serum samples for serum cytokine analysis were collected by tail vein bleeding or terminally by cardiac puncture at t = 2, 3, 4 and 5 h. The results of bioanalytical measurement for JB6121 are presented in Table 10 below.

**Table 10. Mean whole blood concentrations of jb6121 in sprague-dawley rats.**

| | Time^{A} (h) | Group 4 JB6121 0.39 mg/kg (ng/mL) | Group 5 JB6121 1.56 mg/kg (ng/mL) | Group 6 JB6121 6.25 mg/kg (ng/mL) | Group 7 JB6121 25 mg/kg (ng/mL) |
|---|---|---|---|---|---|
| | 2 | 2.50 | 3.93 | 27.0 | 401 |
| | 3 | 3.88 | 8.20 | 94.8 | 711 |
| | 4 | 5.57 | 7.80 | 77.8 | 487 |
| | 5 | 6.47 | 7.76 | 63.8 | 366 |
| AUC₍₀₋ₜ₎ | ng·h/mL | 9.68 | 21.3 | 217 | 1570 |
| AUC Extrap | ng·h/mL | 133 | 231 | 609 | 2770 |
| AUC₍₀₋ₜ₎/D | ng·h/mL/mg/kg/day | 24.8 | 13.6 | 34.8 | 63 |
| Cmax | ng/mL | 5.24 | 10.9 | 95 | 711 |
| Cmax/D | ng/mL/mg/kg/day | 13.4 | 6.96 | 15.2 | 28.4 |
| tmax | h | 3.8 | 4 | 3.2 | 3 |
| T½ | h | 8.75 | 21.2 | 4.09 | 2.2 |

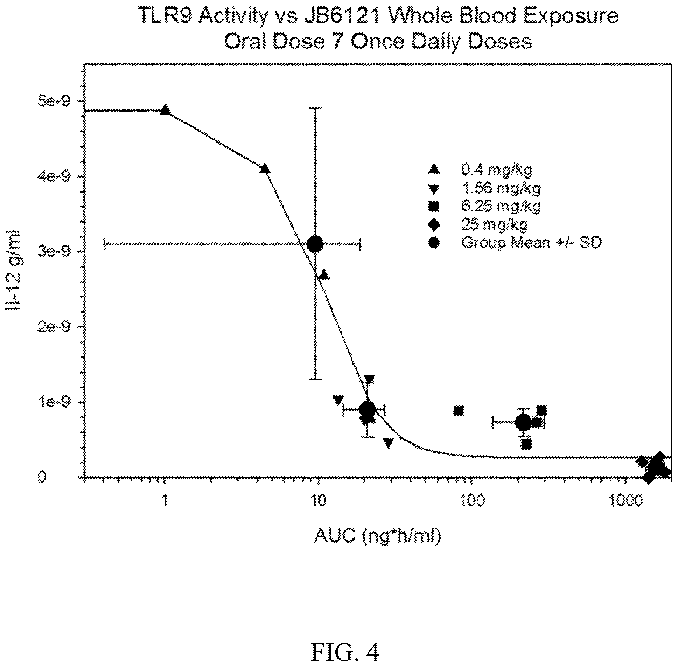

Serum IL-12 was monitored at three hours post agonist challenge as a measure of pharmacodynamic JB6121 activity, see FIG. 4. The apparent ID50 post 7-day exposure is approximately 11ng*h/ml AUC or roughly 0.5 mg/kg dose. This value roughly correlates with the 2.4 mg/kg ID50 determined in the in vivo TLR9 knockdown assay (above section 4.2, In vivo Pharmacology) considering 7-day accumulation of approximately 2 fold (see 7 day toxicokinetics measures versus 24h PK whole blood exposures) and an allometric scaling of 2 between rats and mice, if appropriate. This would lead to a fourfold greater, 0.5 mg/kg versus 2.4 mg/kg, expected activity in the 7-day rat versus the 1-day mouse. However, due to the strong variability in the whole blood exposure at the 0.4 mg/kg dose one can simply state that > 80% inhibition of TLR9 was achieved at the 1.56 mg/kg dose in this study. The 1.56 mg/kg dose resulted in a mean Cmax blood exposure of 10.9 ng/ml JB6121 post the 7th dose. We also used multiplex analysis to measure additional cytokines and chemokines in the serum samples. The measurements delivered results for MIP-1a, MIP-2, MCP-1 and IP-10. IP-10 yielded the highest quality estimate of ID50, ≈ 2.8 mg/kg, in reasonable agreement with the IL-12 ELISA results detailed above.

### JB6121 Toxicology

### Genotoxicity

A non-GLP AMES test study was performed by BioRelience Inc, MD according to Maron, D.M. and Ames, B.N., 1983, Mutation Res., 113:173-215. The test was performed in five bacterial strains plus or minus S9 fraction to 5000 µg.

### Cardiovascular

The hERG assay was conducted by Cyprotec Inc. in its Watertown, MA laboratories. The concentration range tested was 0.0096-30µM. No concentration-dependent inhibition was observed, IC₅₀ > 30 µM.

The hERG inhibition assay uses a high throughput single cell planar patch clamp approach. Chinese hamster ovary cells transfected with the hERG gene (CHO-hERG) are dispensed into the PatchPlate. Amphotericin is used as a perforating agent to gain electrical access to the cells. The hERG tail current is measured prior to the addition of the test compound by perforated patch clamping. Following addition of the test compound, n= 4 cells per concentration, final DMSO concentration = 0.25%), a second recording of the hERG current is performed.

Post-compound hERG currents are expressed as a percentage of pre-compound hERG currents (% control current) and plotted against concentration for each compound. Where concentration dependent inhibition is observed the Hill equation is used to fit a sigmoidal line to the data and an IC₅₀ (concentration at which 50% inhibition is observed) is determined. The assays were performed according to Haverkamp, W. et al., 2000, Eur Heart J., 21:1216-1231, Kiss, L. et al., 2003, Assay Drug Dev. Technol., 1:127-135 and Schroeder, K. et al., 2003, J. Biomol Screen, 8:50-64.

### In vivo Toxicity

A 7-day non-GLP tox study was performed in rats. Dosing was once daily by oral gavage. There were four dose groups; 0, 10, 30 and 100 mg/kg. The following parameters and end points were evaluated in this study: clinical signs, body weights, body weight changes, food consumption, clinical pathology parameters (hematology, coagulation, clinical chemistry, and urinalysis), toxicokinetic parameters, gross necropsy findings, organ weights, and histopathologic examinations.

There were no test article-related effects on mortality, clinical observations, body weights, body weight changes, food consumption, hematology parameters, coagulation parameters, clinical chemistry parameters, urinalysis parameters, or gross necropsy observations. Relative to spleen weights, the differences in the mean values among the dose groups were not statistically different when measuring absolute spleen weight (P = 0.444), % spleen of body weight (P = 0.278), or % spleen of brain weight (P = 0.502) in males. There was, however, a trend toward increasing mean spleen weight with escalating dose. The dose-related increase in spleen weights in males at ≥ 30 mg/kg/day coincided with an increased incidence of hematopoiesis at ≥ 30 mg/kg/day. There was also a slight increase in hematopoiesis in the liver of males at 100 mg/kg/day. Increased hematopoiesis is frequently an adaptive (non-adverse) response, which regresses following removal of the initiating stimulus. However, because no changes that could initiate such a response were observed in the limited tissues examined, the possibility that this could be a direct effect of JB6121 cannot be ruled out. Once daily oral administration of JB6121 for 7 days was well tolerated in rats at levels of 10, 30, and 100 mg/kg/day. Mean spleen weights were increased in males by at least 10% at ≥ 30 mg/kg/day, which coincided with increased hematopoiesis in the spleen. Because there were no apparent adverse effects of the increased hematopoiesis on hematology parameters, liver enzymes, or the spleen or liver tissue, the no-observed-adverse-effect level (NOAEL) was considered to be 100 mg/kg/day for males and females while the no-observed-effect level (NOEL) was considered to be 10 mg/kg/day in males and 100 mg/kg/day in females.

### Toxicokinetics Results

Clinical signs and body weight changes in the TK animals were generally similar to the main toxicology phase animals. Select toxicokinetic parameters from Days 1 and 7 are included below in Table 11.

**Table 11. Summary of Toxicokinetic Parameters - Day 1.**

| **Dose (mg/kg)** | **Sex** | | **Cmax (ng/mL)** | | **Tmax (hr)** | | **T1/2 (hr)** | | **AUC (ng·hr/mL)** | | **F(0-x) (%)** |
|---|---|---|---|---|---|---|---|---|---|---|---|
| 10 | Male | | 122 | | 3.00 | | 14.1 | | 743 | | 13.3 |
| | Female | | 92.0 | | 3.00 | | 5.43 | | 357 | | 8.10 |
| 30 | Male | | 582 | | 3.00 | | 9.39 | | 4700 | | 28.0 |
| | Female | | 643 | | 3.00 | | 8.80 | | 4920 | | 37.2 |
| 100 | Male | | 3950 | | 3.00 | | 11.9 | | 43800 | | 78.5 |
| | Female | | 3980 | | 3.00 | | 9.39 | | 40300 | | 91.0 |
| | | | | | | | | | | | |

| **Dose (mg/kg)** | | **Sex** | | **CL (mL/min/kg)** | | **Vdss (mL/kg)** | | **T1/2 (hr)** | | **AUC (ng·hr/mL)** | |
|---|---|---|---|---|---|---|---|---|---|---|---|
| 5 I.V. | | Male | | 24.7 | | 18100 | | 10.2 | | 2790 | |
| | | Female | | 31.5 | | 23100 | | 10.8 | | 2210 | |

**Table 12. Summary of Toxicokinetic Parameters - Day 7**

| **Dose (mg/kg)** | **Sex** | **Cmax (ng/mL)** | **Tmax (hr)** | **T1/2 (hr)** | **AUC (ng·hr/mL)** |
|---|---|---|---|---|---|
| 10 | Male | 181 | 3.00 | 19.9 | 1910 |
| | Female | 260 | 3.00 | 18.2 | 2120 |
| 30 | Male | 805 | 6.00 | 18.4 | 11100 |
| | Female | 639 | 6.00 | 14.3 | 8890 |
| 100 | Male | 6280 | 3.00 | 40.4 | 91200 |
| | Female | 5310 | 3.00 | 14.1 | 80200 |

More than dose proportional increase in Cmax and AUC was observed on Day 1 and Day 7 when the dose was increased from 10 mg/kg to 100 mg/kg. On Day 1, a 10-fold increase in dose resulted in a 32-fold and 43-fold increase in male and female Cmax, respectively and a 59-fold and 113-fold increase in male and female AUC₀₋₂₄ₕ, respectively. Similarly, on Day 7, a 10-fold increase in dose resulted in a 24-fold and 20-fold increase in male and female Cmax, respectively and a 48-fold and 38-fold increase in male and female AUC0-24h, respectively. The disproportional increase in systemic exposure could be due to saturation of "first pass" metabolism and/or inhibition of an efflux transporter in the GI tract, hepatocytes and/or renal tubule cells, depending on the pathways of elimination.

After 7-days of daily oral administration, JB6121 accumulated in whole blood after all three doses with an average D7/D1 Cmax ratio of 1.71 and an AUC0-24h ratio of 2.79; however, some or all of the accumulation is expected due to the half-life of JB6121 and once a day dosing. There was a trend that male rats have slightly higher Cmax and AUC0-24h compared to female rats consistent with a lower clearance after IV administration.

Due to the more than dose proportional increase in AUC 0-24h when the oral dose was increased from 10 to 100 mg/kg, the calculated oral absolute bioavailability (assuming dose proportionality after IV administration between 5 mg/kg to 100 mg/kg) on Day 1 was dose dependent averaging 13.3%, 28.0% and 78.5% after doses of 10, 30 and 100 mg/kg, respectively.

### Pooled 7-Day Whole Blood Exposure Data

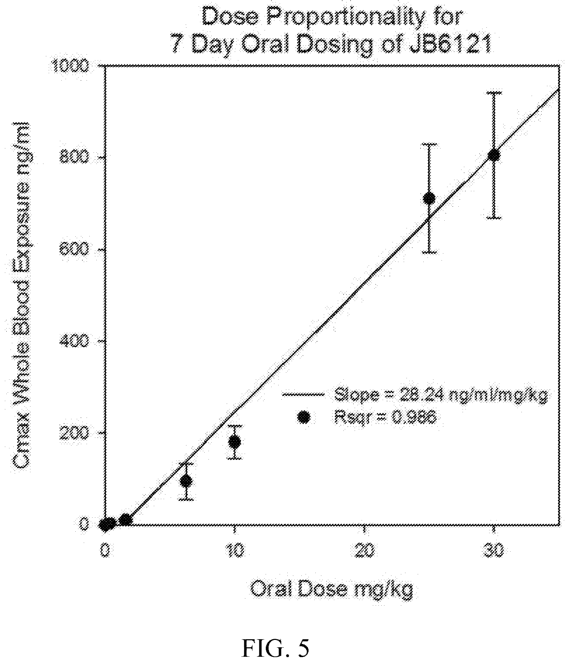

If one pools the 7-day exposure data from the PK/PD study and the toxicokinetics study, plotting Cmax versus dose yields a dose proportional linear curve between 0 and 30 mg/kg administered dose. The line has a correlation coefficient of 0.986 and a slope of 28.24 ng/ml/mg/kg. The 100 mg/kg dose is not dose proportional with the previously lower dose 30 mg/kg by a factor of 2.36 and is not depicted. Cmax was used for this analysis because AUC was not comparable between the studies due to the shorter duration of blood sampling in the PK/PD study (see, FIG. 5).
The invention further includes the subject matter of the claims of WO2016/029077 from which this application was derived, the content of which is reproduced below as numbered paragraphs "para".
1. A compound of Formula I or a pharmaceutically acceptable salt thereof, 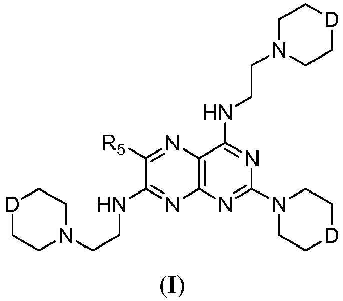 wherein
   each occurrence of D is independently -O- or -N(Me)-; and
   R₅ is H, F, or Cl.
2. The compound of para 1, having the structure selected from the group consisting of 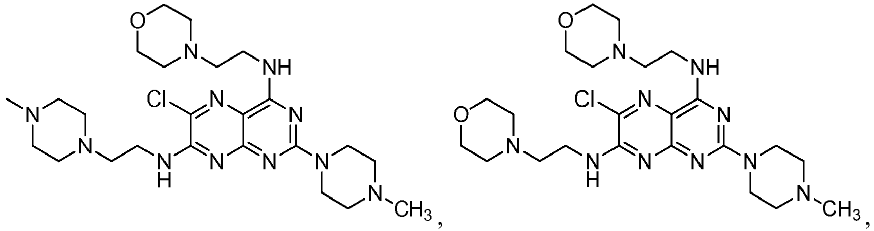 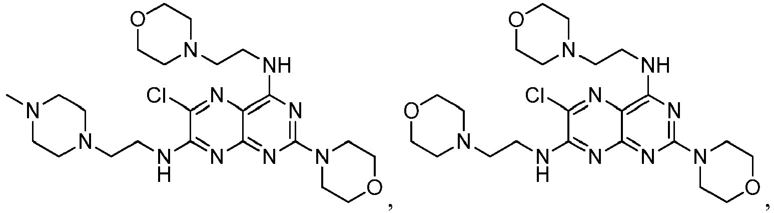 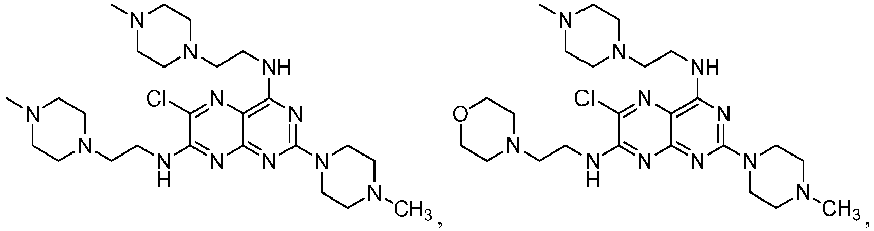 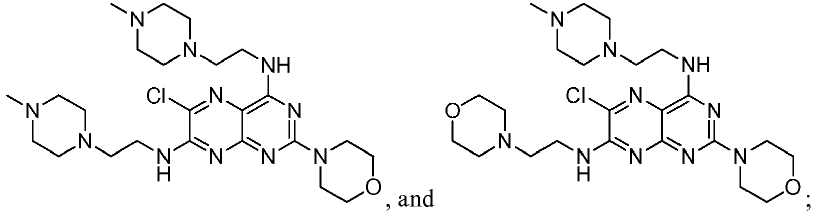 or a pharmaceutically acceptable salt thereof.
3. The compound of para 1 or 2, having the structure selected from the group consisting of 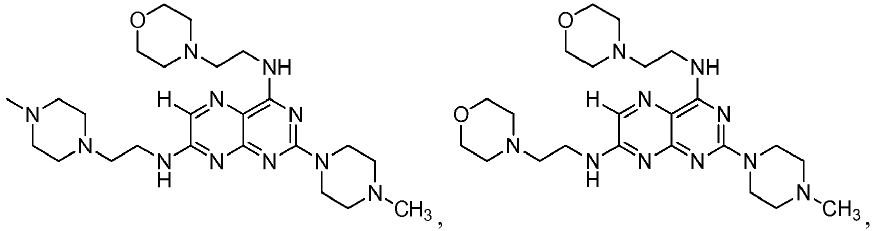 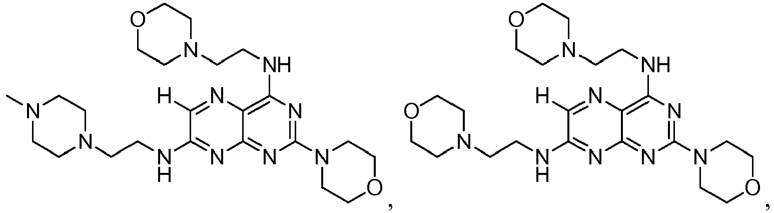 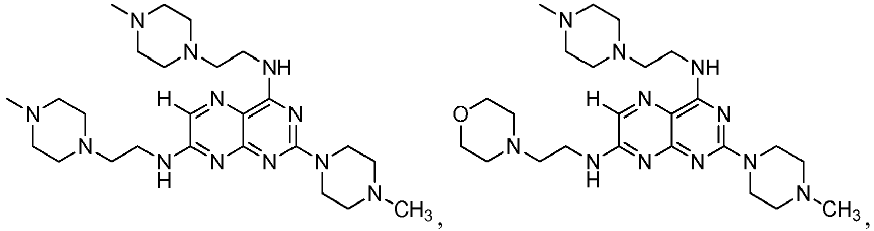 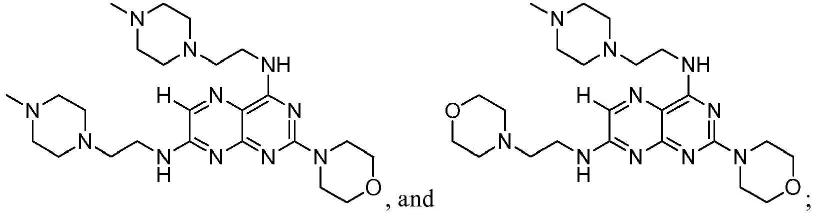 or a pharmaceutically acceptable salt thereof.
4. The compound of para 1, having the structure of 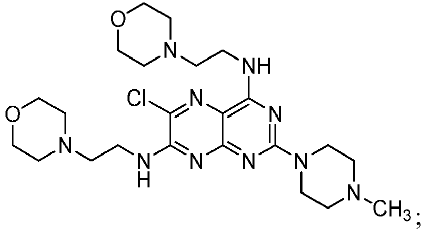 or a pharmaceutically acceptable salt thereof.
5. The compound of para 1, having the structure of 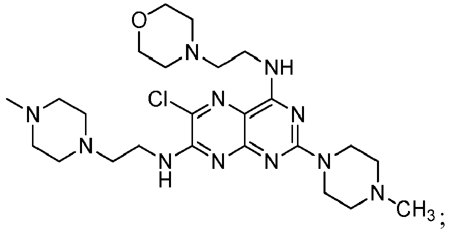 or a pharmaceutically acceptable salt thereof.
6. The compound of any one of paras 1-5, wherein the compound has an IC₅₀ of more than 10, 15, 20, 25, or 30 µM in a standard human ether-a-go-go related gene (hERG) patch clamp assay.
7. The compound of any one of paras 1-6, wherein the compound results in more than 75% hepatocyte viability in a hepatocyte viability assay after the hepatocyte has been exposed to 100 µM of the compound for 24 h.
8. A compound of Formula Ia or a pharmaceutically acceptable salt thereof, 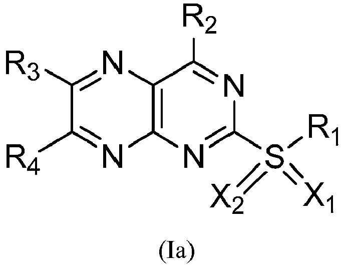 wherein
   R₁ is hydrogen, alkyl, alkenyl, cycloalkyl, alkylcycloalkyl, aryl, alkylaryl, heterocycle, or alkylheterocycle;
   X₁ and X₂ are each independently absent or O;
   R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
   R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
   each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
   each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
   wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
   provided that when R₂ is ORₐ, SRₐ, NR_{b}R_{c}, or NRₐ(CH₂)ₚNR_{b}R_{c}, at least one of X₁ and X₂ is O.
9. The compound of para 8, wherein R₁ is alkyl, optionally substituted aryl, or optionally substituted heteroaryl.
10. The compound of para 8 or 9, wherein X₁ and X₂ are both O.
11. The compound of any one of paras 8-10, wherein R₂ is Cl or Br.
12. The compound of any one of paras 8-10, wherein R₂ is OS(=O)₂Rₐ, or OC(=O)Rₐ.
13. The compound of any one of paras 8-10, wherein R₂ is NR_{b}R_{c} or NRₐ(CH₂)ₚNR_{b}R_{c}.
14. The compound of any one of paras 8-13, wherein R₄ is NR_{b}R_{c} or NRₐ(CH₂)ₚNR_{b}R_{c}.
15. The compound of any one of paras 8-14, wherein R₂ and R₄ are the same or different.
16. The compound of any one of paras 8-15, wherein R₂ and R₄ are each independently selected from the group consisting of: 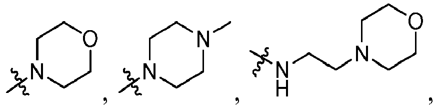 and 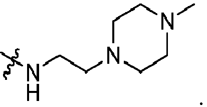
17. A method for the synthesis of a compound having the structure of Formula II, 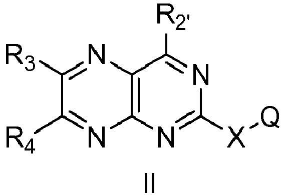 comprising:
   (a) converting a compound having the structure of Formula III to a compound having the structure of Formula IV: 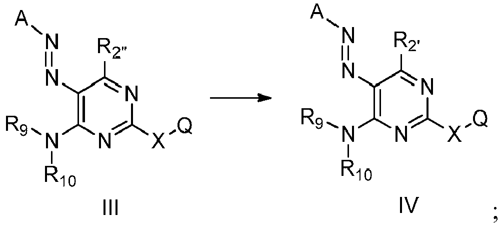 and
   (b) converting the compound having the structure of Formula IV to the compound having the structure of Formula II: 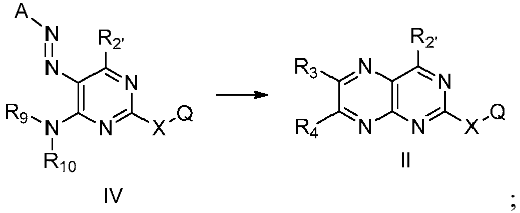 wherein
      each occurrence of X is independently absent or is an alkyl, cycloalkyl, aryl, or heterocycle;
      each occurrence of Q is independently H, (CH₂)_{q}NR_{b}R_{c}, NRₐ(CH₂)ₚNR_{b}R_{c}, OR₁, SR₁, 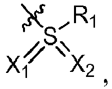 or CRₐR_{b}R_{c}, in which q is 0 or 1 and p is 2-4; and X₁ and X₂ are each independently absent or O;
      R₁ is hydrogen, alkyl, alkenyl, cycloalkyl, alkylcycloalkyl, aryl, alkylaryl, heterocycle, alkylheterocycle;
      R_{2"} is halogen, ORₐ, OS(=O)₂Rₐ, or OC(=O)Rₐ;
      R_{2'} is OH, NR_{b}R_{c}, or NRₐ(CH₂)ₚNR_{b}R_{c};
      A is aryl or heteroaryl;
      each occurrence of R₉ and R₁₀ is each independently hydrogen, OS(=O)₂Rₐ, CH₂C(=O)ORₐ, C(=O)C(=O)ORₐ, OC(=O)Rₐ, OC(=O)ORₐ, or R_{a'}, or alternatively R₉ and R₁₀ are taken together with the nitrogen atom to which that they are attached to form a mono- or bicyclic carbocycle or heterocycle, wherein the carbocycle or heterocycle is optionally substituted with oxo;
      R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
      each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
      each occurrence of R_{b} and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
      wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl.
18. The method of para 17, wherein X is absent and Q is (CH₂)_{q}NR_{b}R_{c}, NRₐ(CH₂)ₚNR_{b}R_{c}, OR₁, SR₁, or 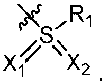
19. The method of para 17 or 18, wherein R_{2'} and R₄ are the same.
20. The method of para 17 or 18, wherein R_{2'} and R₄ are different.
21. The method of any one of paras 17-20, wherein R₉ and R₁₀ are selected from the group consisting of Fmoc-, Cbz-, Boc-, Ac-, CF₃(C=O)-, Benzyl, triphenylmethyl, and p-Toluenesulfonyl; or R₉ and R₁₀ are taken together with the nitrogen atom to which they are bonded to form 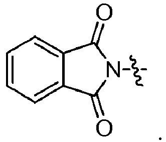
22. The method of any one of paras 17-20, wherein A is phenyl.
23. The method of any one of paras 17-20, further comprising a step of (a₁): 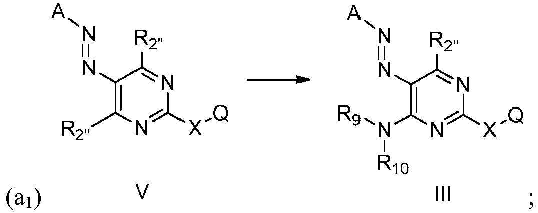 wherein each occurrence of R_{2"} is independent halogen, ORₐ, OS(=O)₂Rₐ, or OC(=O)Rₐ.
24. The method of para 23, wherein the step (a₁) further comprises the steps of (a₂) and (a₃): 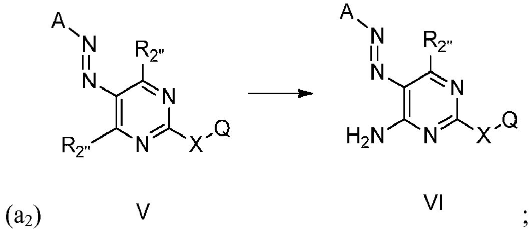 and 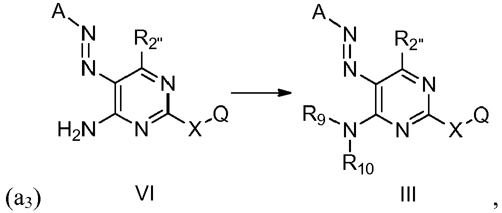 wherein at least one of R₉ and R₁₀ is not hydrogen.
25. The method of any one of paras 17-24, wherein step (b) further comprises the steps of (b₁) and (b₂): 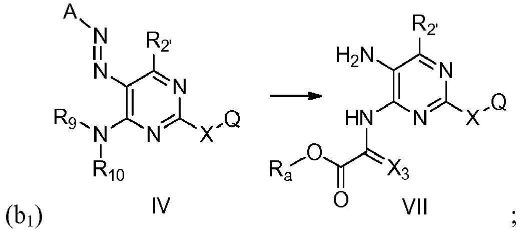 and 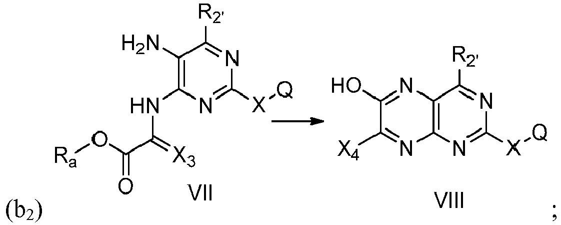 wherein X₃ is O or absent, X₄ is OH or absent, and Rₐ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl.
26. The method of para 25, wherein R₉ is H and R₁₀ is -(C=O)ORₐ.
27. The method of para 25 or 26, further comprising the steps of (b₃) and (b₄): 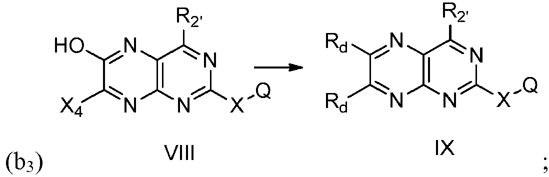 and 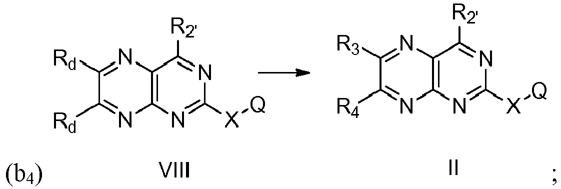 wherein each occurrence of R_{d} is independently H, halogen, OS(=O)₂Rₐ, or OC(=O)Rₐ.
28. A method for the synthesis of a compound having the structure of Formula II, 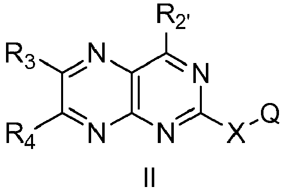 comprising:
   (a) converting a compound having the structure of Formula X to a compound having the structure of Formula XI: 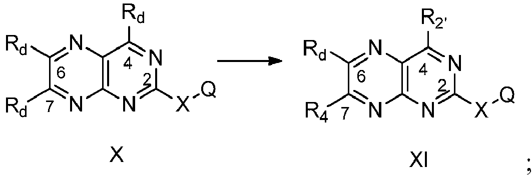 and
   (b) converting the compound having the structure of Formula XI to the compound having the structure of Formula II: 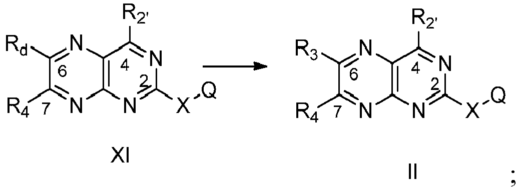 wherein
      each occurence of X is independently absent or is an alkyl, cycloalkyl, aryl, or heterocycle;
      each occurence of Q is independently H, R_{d}, (CH₂)_{q}NRₐR_{b}, NRₐ(CH₂)ₚNR_{b}R_{c}, OR₁, SR₁, 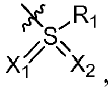 or CRₐR_{b}R_{c}, in which q is 0 or 1 and p is 2-4; and X₁ and X₂ are each independently absent or O;
      R₁ is hydrogen, alkyl, alkenyl, cycloalkyl, alkylcycloalkyl, aryl, alkylaryl, heterocycle, alkylheterocycle;
      each occurrence of R_{d} is independently halogen, OS(=O)₂Rₐ, or OC(=O)Rₐ;
      R_{2'} is OH, NR_{b}R_{c}, or NRₐ(CH₂)ₚNR_{b}R_{c};
      R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
      each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
      each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
      wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
      with the proviso that step (b) can be omitted if R_{d} at the 6 position of the compound of Formula XI and R₃ are the same.
29. The method of para 28, wherein R_{2'} and R₄ are the same.
30. The method of para 28, wherein R_{2'} and R₄ are different.
31. The method of any one of paras 28-30, wherein step (a) further comprises steps (a₁) and (a₂): 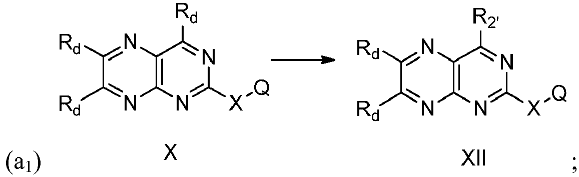 and 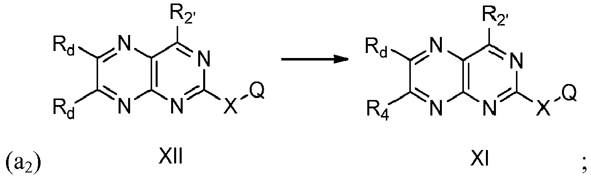 wherein step (a₁) further comprises purifying the compound having the structure of Formula XII.
32. The method of para 31, wherein the purification is a column chromatography purification or HPLC purification.
33. The method of any one of paras 28-30, wherein step (a) further comprises steps (a_{1'}) and (a_{2'}): 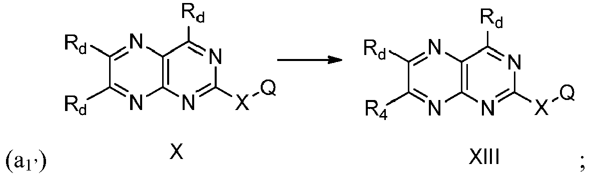 and 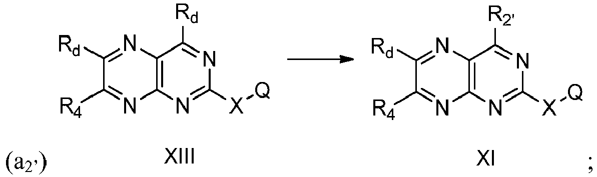 wherein step (a_{1'}) further compresses purifying the compound having the structure of Formula XIII.
34. The method of para 33, wherein the purification is a column chromatography purification or HPLC purification.
35. The method of para 30, wherein step (a) comprises a one-pot synthetic step (a₁ₓ): 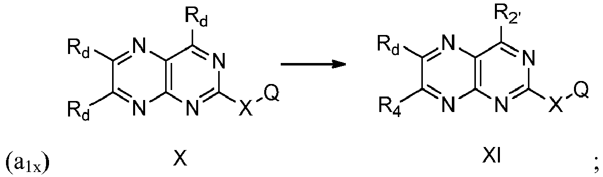 wherein step (a₁ₓ) further comprises purifying the compound having the structure of Formula XI.
36. The method of para 35, wherein the purification is a column chromatography purification or HPLC purification.
37. The method of any one of paras 28-30, wherein the substituent -X-Q in Formulae X and XI is R_{d}, and step (a) comprises converting a compound having the structure of Formula X' to a compound having the structure of Formula XI': 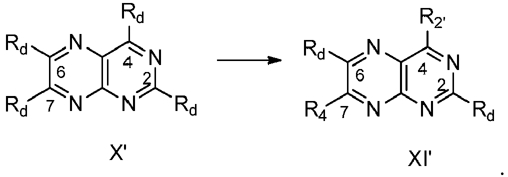
38. The method of para 37, wherein the method further comprises step (a'):
   (a') converting a compound having the structure of Formula XI' to a compound having the structure of Formula XI": 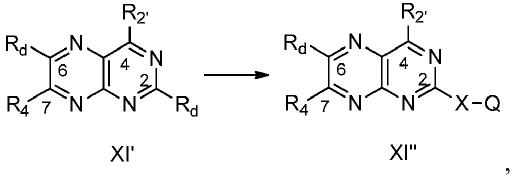 wherein -X-Q is not R_{d}.
39. The method of para 37 or 38, wherein -X-Q is NRₐR_{b}, NRₐ(CH₂)ₚNR_{b}R_{c}, OR₁, or SR₁.
40. The method of any one of paras 37-39, wherein -X-Q is 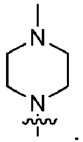
41. The method of any one of paras 37-40, wherein R_{2'} and R₄ are the same.
42. The method of para 41, wherein R_{2'} and R₄ are both 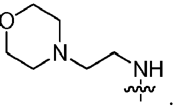
43. The method of any one of paras 37-42, wherein R_{d} and R₃ are both Cl.
44. The method of any one of paras 37-43, wherein the method comprises the following two steps: 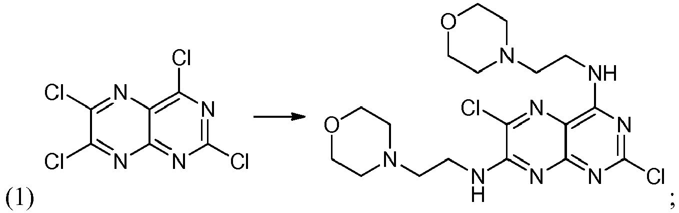 and 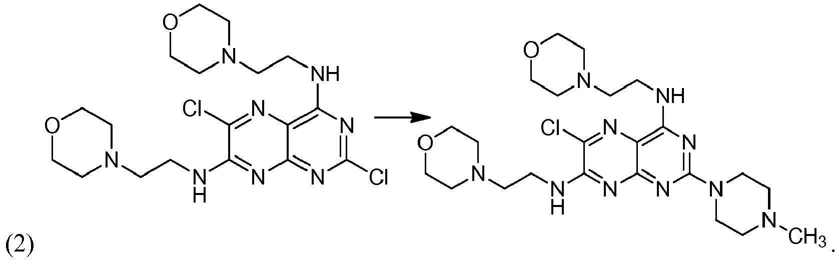
45. The method of para 44, wherein the method further comprises preparing the compound having the structure of 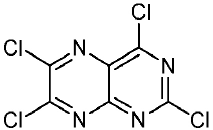 by the following steps: 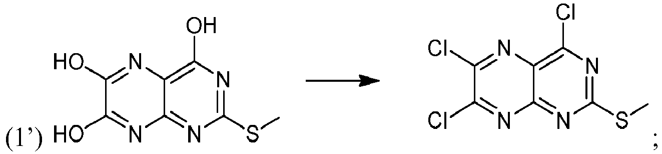 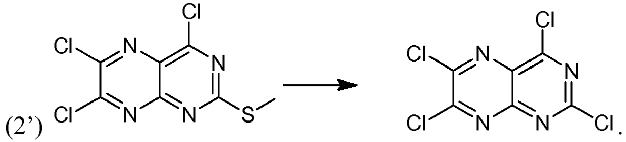
46. The method of para 45, wherein the method further comprises preparing the compound having the structure of 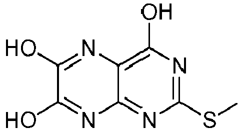 by the following steps: 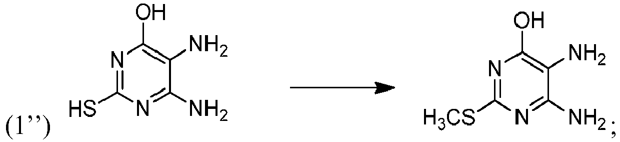 and 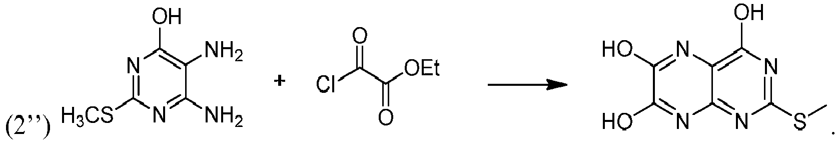
47. A compound selected from the group consisting of: 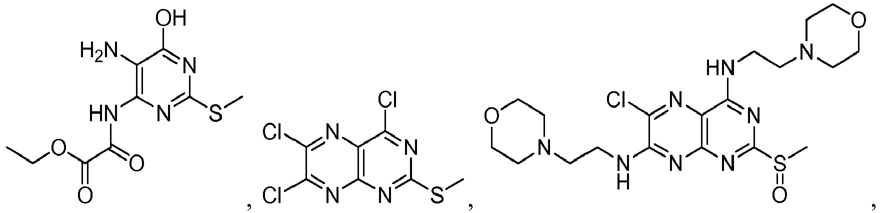 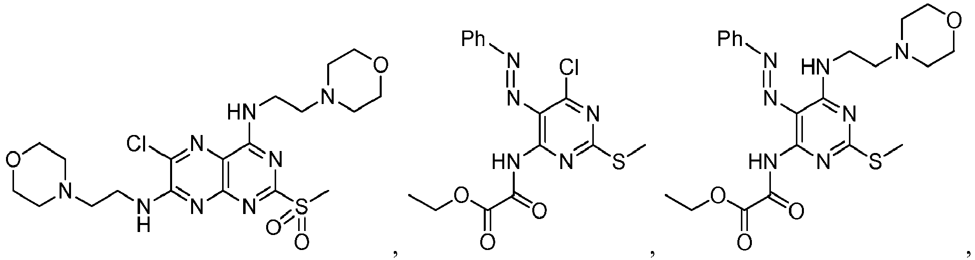 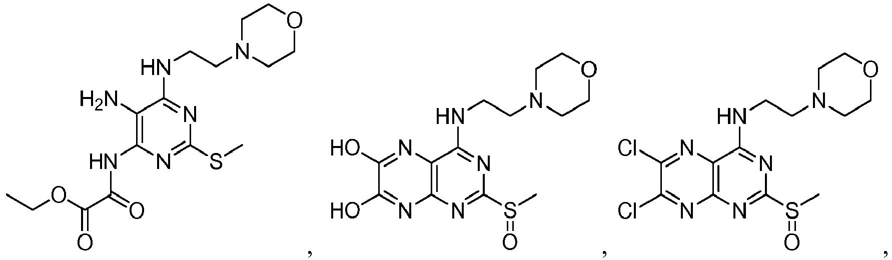 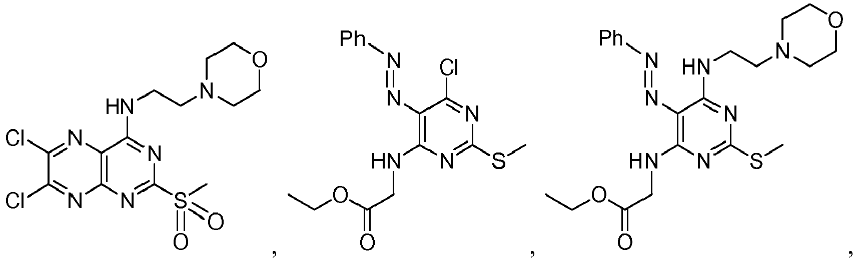 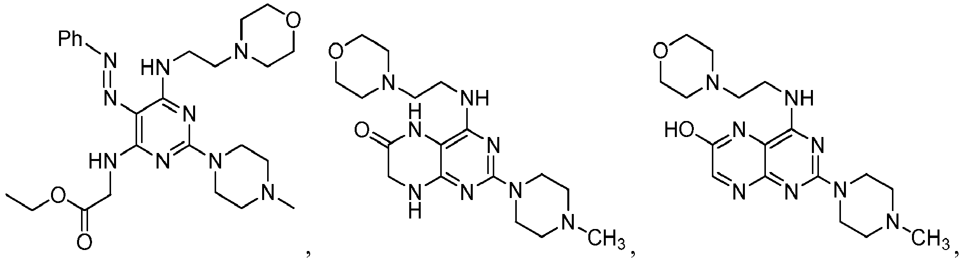 and 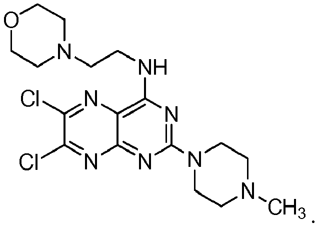
48. A compound of para 8 selected from Table 1.
49. A pharmaceutical composition comprising at least one compound according to any one of paras 1-16 and a pharmaceutically acceptable carrier or diluent.
50. A method of treating an autoimmune disease in a mammalian species in need thereof, comprising administering to the mammalian species a therapeutically effective amount of at least one compound according to any one of paras 1-16.
51. The method of para 50, wherein the mammalian species is human.
52. The method of para 50 or 51, wherein the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.
53. A method of inhibiting TLR-mediated immunostimulation in a mammalian species in need thereof, comprising administering to the mammalian species a therapeutically effective amount of at least one compound according to any one of paras 1-16.
54. The method of para 53, wherein the mammalian species is human.
55. A method of inhibiting TLR-mediated immunostimulatory signaling, comprising contacting a cell expressing a TLR with an effective amount of at least one compound according to any one of paras 1-16.
56. Use of a therapeutically effective amount of at least one compound according to any one of paras 1-16 for the manufacture of a medicament for treating an autoimmune disease in a mammalian species in need thereof.
57. The use of para 56, wherein the mammalian species is human.
58. The use of para 56 or 57, wherein the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.
59. Use of a therapeutically effective amount of at least one compound according to any one of paras 1-16 for the manufacture of a medicament for inhibiting TLR-mediated immunostimulation in a mammalian species in need thereof.
60. The use of para 59, wherein the mammalian species is human.
61. A therapeutically effective amount of at least one compound according to any one of paras 1-16 for the treatment of an autoimmune disease in a mammalian species in need thereof.
62. The compound of para 61, wherein the mammalian species is human.
63. The compound of para 61 or 62, wherein the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.
64. A therapeutically effective amount of at least one compound according to any one of paras 1-16 for the treatment of unwanted TLR-mediated immunostimulation in a mammalian species in need thereof.
65. The compound of para 64, wherein the mammalian species is human.
66. Compound, method, or composition as described in the description.

## Claims

1. A compound of Formula Ia or a pharmaceutically acceptable salt thereof, 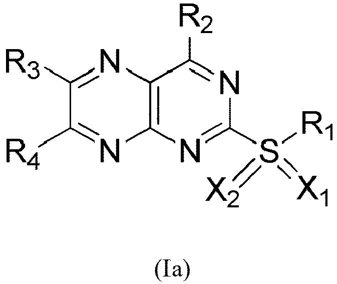 wherein
R₁ is hydrogen, alkyl, alkenyl, cycloalkyl, alkylcycloalkyl, aryl, alkylaryl, heterocycle, or alkylheterocycle;
X₁ and X₂ are each independently absent or O;
R₂ is halogen, ORₐ, SRₐ, OS(=O)₂Rₐ, OC(=O)Rₐ, NR_{b}R_{c}, or NRₐ(CH₂)ₚNR_{b}R_{c}, wherein p is 2-4;
R₃ and R₄ are each hydrogen, halogen, cyano, nitro, CF₃, OCF₃, alkyl, cycloalkyl, alkenyl, optionally substituted aryl, heterocycle, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c};
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl; and
each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
provided that when R₂ is ORₐ, SRₐ, NR_{b}R_{c}, or NRₐ(CH₂)ₚNR_{b}R_{c}, at least one of X₁ and X₂ is O.

2. The compound of claim 1, wherein R₁ is alkyl, optionally substituted aryl, or optionally substituted heteroaryl; or
wherein R₂ is Cl or Br; or
wherein R₂ is NR_{b}R_{c} or NRₐ(CH₂)ₚNR_{b}R_{c}; or
wherein R₄ is NR_{b}R_{c} or NRₐ(CH₂)ₚNR_{b}R_{c}; or
wherein R₂ and R₄ are the same or different; or
wherein R₂ and R₄ are each independently selected from the group consisting of:

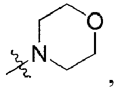

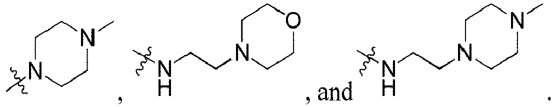

3. A compound of claim 1 selected from Table 1.

4. A pharmaceutical composition comprising at least one compound according to any one of claims 1-2 and a pharmaceutically acceptable carrier or diluent.

5. A therapeutically effective amount of at least one compound according to any one of claims 1-3 for the treatment of an autoimmune disease in a mammalian species in need thereof.

6. The compound of claim 5, wherein the mammalian species is human; or
wherein the autoimmune disease is selected from cutaneous and systemic lupus erythematosus, insulin-dependent diabetes mellitus, rheumatoid arthritis, multiple sclerosis, atherosclerosis, psoriasis, psoriatic arthritis, inflammatory bowel disease, ankylosing spondylitis, autoimmune hemolytic anemia, Behget's syndrome, Goodpasture's syndrome, Graves' disease, Guillain-Barre syndrome, Hashimoto's thyroiditis, idiopathic thrombocytopenia, io myasthenia gravis, pernicious anemia, polyarteritis nodosa, polymyositis/dermatomyositis, primary biliary sclerosis, sarcoidosis, sclerosing cholangitis, Sjogren's syndrome, systemic sclerosis (scleroderma and CREST syndrome), Takayasu's arteritis, temporal arteritis, and Wegener's granulomatosis.

7. A therapeutically effective amount of at least one compound according to any one of claims 1-3 for the treatment of unwanted TLR-mediated immunostimulation in a mammalian species in need thereof.

8. The compound of claim 7, wherein the mammalian species is human.

9. A method for the synthesis of a compound having the structure of Formula II, 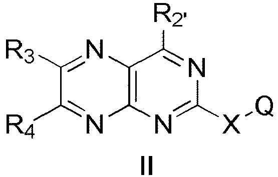 comprising:
(a) converting a compound having the structure of Formula X to a compound having the structure of Formula XI: 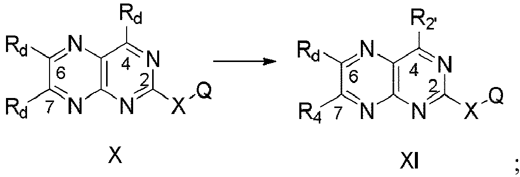 and
(b) converting the compound having the structure of Formula XI to the compound having the structure of Formula II: 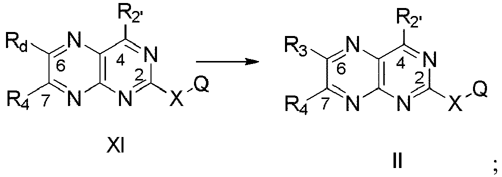 wherein
each occurrence of X is independently absent or is an alkyl, cycloalkyl, aryl, or heterocycle;
each occurrence of Q is independently H, R_{d}, (CH₂)_{q}NRₐR_{b}, NRₐ(CH₂)ₚNR_{b}R_{c}, OR₁, SR₁, 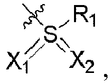 or CRₐR_{b}R_{c}, in which q is 0 or 1 and p is 2-4; and X₁ and X₂ are each independently absent or O;
R₁ is hydrogen, alkyl, alkenyl, cycloalkyl, alkylcycloalkyl, aryl, alkylaryl, heterocycle, alkylheterocycle;
each occurrence of R_{d} is independently halogen, OS(=O)₂Rₐ, or OC(=O)Rₐ;
R_{2'} is OH, NR_{b}R_{c}, or NRₐ(CH₂)ₚNR_{b}R_{c};
R₃ and R₄ are each independently hydrogen, halogen, cyano, nitro, CF₃, OCF₃, alkyl, cycloalkyl, alkenyl, optionally substituted aryl, heterocycle, ORₐ, SRₐ, S(=O)Rₐ, S(=O)₂Rₐ, NR_{b}R_{c}, S(=O)₂NR_{b}R_{c}, C(=O)ORₐ, C(=O)Rₐ, C(=O)NR_{b}R_{c}, OC(=O)Rₐ, OC(=O)NR_{b}R_{c}, NR_{b}C(=O)ORₐ, NR_{b}C(=O)Rₐ, alkaryl, alkylheterocyclic, or NRₐ(CH₂)ₚNR_{b}R_{c}, wherein p is 2-4; and
each occurrence of Rₐ is independently hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
each occurrence of R_{b}, and R_{c} is independently hydrogen, alkyl, cycloalkyl, alkenyl, cycloalkenyl, alkynyl, heterocycle, aryl; or said R_{b} and R_{c} together with the nitrogen atom to which they are bonded optionally form a heterocycle comprising 1-4 heteroatoms; or said Rₐ and R_{b} together with the nitrogen atom to which they are bonded optionally form a heterocycle comprising 1-4 heteroatoms;
wherein the formed heterocycle is optionally substituted by (C₁-C₄)alkyl and one or more carbon atoms in the formed heterocycle are optionally replaced with O, NR₈, or S, wherein R₈ is hydrogen, optionally substituted alkyl, optionally substituted cycloalkyl, optionally substituted alkenyl, optionally substituted cycloalkenyl, optionally substituted alkynyl, optionally substituted heterocycle, or optionally substituted aryl;
with the proviso that step (b) can be omitted if R_{d} at the 6 position of the compound of Formula XI and R₃ are the same.

10. The method of claim 9, wherein step (a) further comprises steps (a₁) and (a₂): 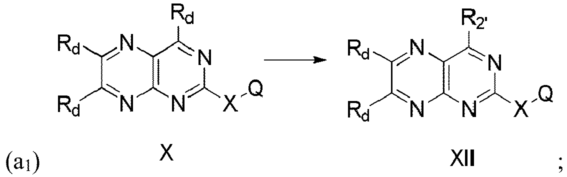 and 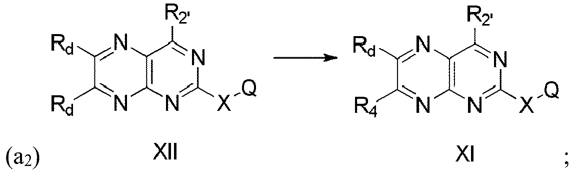 wherein step (a₁) further comprises purifying the compound having the structure of Formula XII; or
wherein step (a) further comprises steps (a_{1'}) and (a_{2'}): 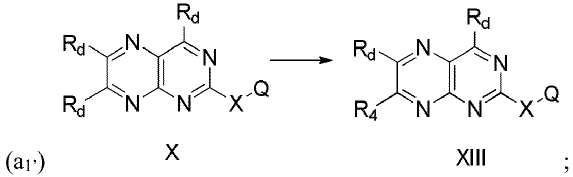 and 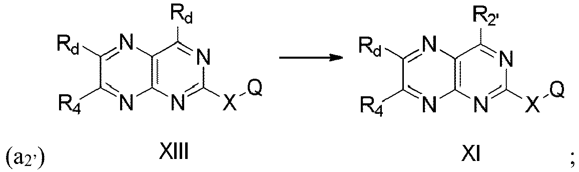 wherein step (a_{1'}) further compresses purifying the compound having the structure of Formula XIII.

11. The method of claim 9 or 10, wherein the substituent -X-Q in Formulae X and XI is R_{d}, and step (a) comprises converting a compound having the structure of Formula X' to a compound having the structure of Formula XI': 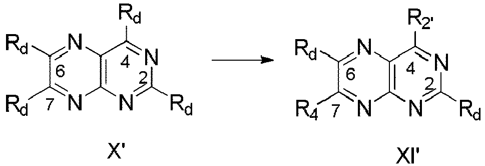 ; or
wherein the substituent -X-Q in Formulae X and XI is R_{d}, and step (a) comprises converting a compound having the structure of Formula X' to a compound having the structure of Formula XI': 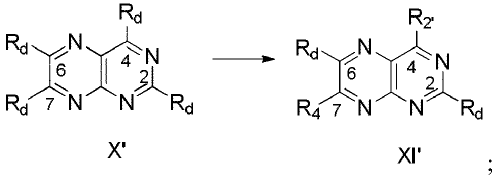 and the method further comprises step (a'):
(a') converting a compound having the structure of Formula XI' to a compound having the structure of Formula XI": 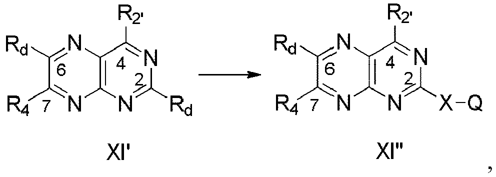 wherein -X-Q is not R_{d}.

12. The method of any one of claims 9-11, wherein R_{2'} and R₄ are the same or different; or-X-Q is NRₐR_{b}, NRₐ(CH₂)ₚNR_{b}R_{c}, OR₁, or SR₁; or wherein R_{2'} and R₄ are both 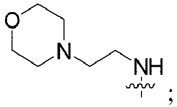 or
wherein R_{d} and R₃ are both Cl.

13. The method of any one of claims 9-12, wherein the method comprises the following two steps: 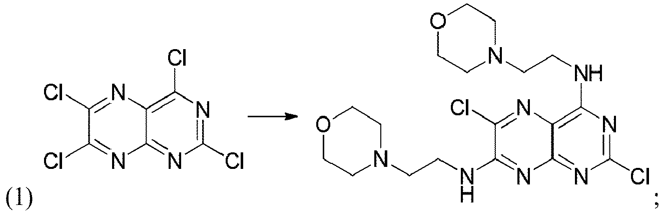 and

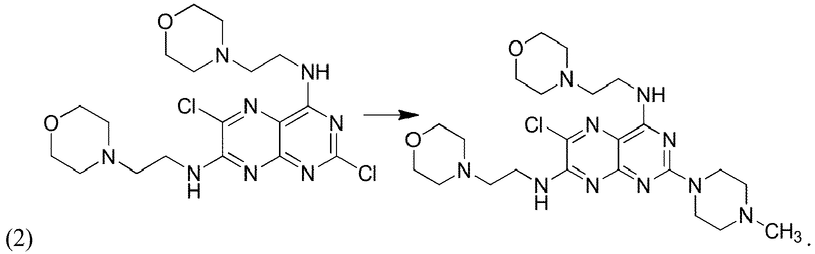

14. The method of claim 13, wherein the method further comprises preparing the compound having the structure of 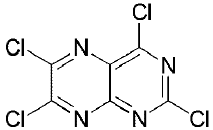 by the following steps:

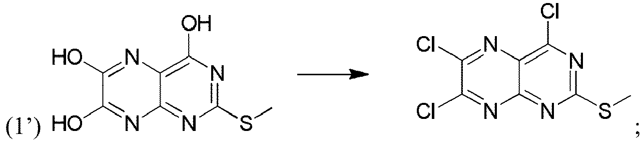

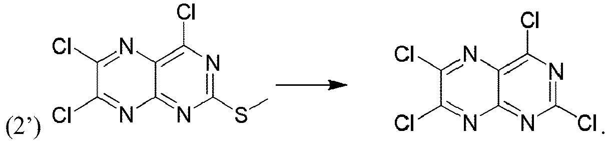

15. The method of claim 14, wherein the method further comprises preparing the compound having the structure of 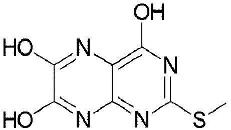 by the following steps: 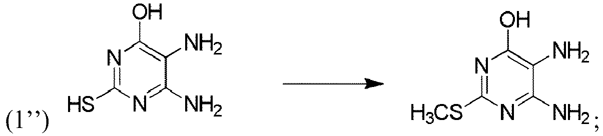 and
